# EUROPEAN PATENT APPLICATION

(11) **EP 4 537 851 A1**
(43) Date of publication of application: **16.04.2025**
(21) Application number: 23818943.5
(22) Date of filing: 24.05.2023
(51) Int. Cl.: A61K 47/62, A61K 47/54, A61K 51/08, C07K 16/18, A61P 35/00, A61P 25/00, A61P 9/10

(54) **TRIFUNCTIONAL COMPOUND AND USE THEREOF**

(30) Priority: 10.06.2022 WO PCT/CN2022/098074
(71) Applicant: Peking University, Beijing 100871 (CN)
(72) Inventor: LIU, Zhibo, Beijing 100871 (CN); CUI, Xiyang, Beijing 100871 (CN)
(74) Representative: Metroconsult Srl
(86) International application number: PCT/CN2023/096111
(87) International publication number: WO 2023/236778

(57) **Abstract**

The present invention relates to a series of trifunctional compounds, comprising at least one covalent warhead capable of forming a reversible or irreversible covalent linkage with a protein or tissue. The present invention further relates to a pharmaceutical composition and kit comprising the trifunctional compounds, and use of the trifunctional compounds in the diagnosis or treatment of diseases.

## Description

### Technical Field

The present disclosure relates to the field of medicines, in particular to a trifunctional compound containing a covalent warhead, a pharmaceutical composition thereof, and the use thereof for diagnosing or treating a disease.

### Background Art

Radiotherapy, surgery, and chemotherapy are three traditional first-line treatments for human beings against cancer. Radioactive drugs (referred to as "radio-drugs") can be regarded as an internal irradiation form of radiotherapy, which has the advantage of the so-called powerful "cross fire" compared with traditional radiotherapy and is one of the few treatment means for patients with advanced cancer and systemic metastasis. In mechanism, radio-drugs induce cancer cell death by disrupting DNA strands through high-energy ionizing radiation produced by the decay of medical isotopes. This requires such radio-drugs to have extremely high tumor selectivity/targeting performance and a certain level of tumor uptake and retention, thereby allowing for targeted delivery of a sufficient dose of radiation only to the tumor with limited damage to surrounding normal tissues.

Radionuclide drug conjugates (RDC) are a main class of pharmaceutical forms of radioactive drugs, which are generally chelator-linker-targeting ligand binary conjugates, wherein the ligand moiety can be a small molecule, a polypeptide, a nucleic acid aptamer, an antibody, etc.; the linker mainly functions as a spacer to prevent the conjugated chelator from substantially reducing the affinity of the targeting molecule and has a certain effect on regulating the pharmacokinetic properties; and the chelator moiety can coordinate with various diagnostic/therapeutic nuclides with the same molecule or by changing the chelator, depending on the need for diagnosis and treatment, and has the advantage of "integrated diagnosis and treatment"; therefore, some molecules with ordinary targeting and safety but a higher tumor uptake are still promising for molecular imaging diagnosis. Diagnostic nuclides can be divided into two categories, including positron nuclides (such as ¹⁸_{F}, ⁶⁸Ga, ⁶⁴Cu, ⁸⁶Y, and ⁸⁹Zr) that can be traced by positron emission tomography (PET-CT) and nuclides with γ-ray radiation (such as ^{99m}Tc, ¹³³Xe, ¹²³I, and ^{91m}Kr) that can be traced by single photon emission tomography (SPECT-CT). Therapeutic nuclides can be divided into β-emitters (such as ⁹⁰Y, ¹²⁴I, ¹⁵¹Tb, and ¹⁷⁷Lu) and α-emitters (such as ²¹¹At, ²¹²Pb, ²¹³Bi, ²²³Ra, and ²²⁵Ac). In addition, the chelator moiety is partially replaced with optical dyes that absorb and emit different wavelengths to become optical probes, which may also be used for optical imaging, for example, at the cellular and *in vivo* levels.

In recent years, diagnostic/therapeutic RDCs based on prostate-specific membrane antigens (PSMAs) and somatostatin receptors (SSTRs) have been approved by FDA/EU, encouraging more RDC drugs to enter clinical research. Since PSMAs and SSTRs are only expressed in prostate cancer and neuroendocrine tumor, respectively, and do not have broad-spectrum tumor targeting, so researchers around the world are pursuing to develop RDC drugs with a larger broad-spectrum. In addition, although some existing RDC drugs can be taken up by and retained in tumors to some extent, they have the drawback of significant side effects due to high background uptake in non-target organs. There is still a need to develop RDC drugs with high uptake only at the target site.

Fibroblast activation protein (FAP-α), a type II membrane-bound glycoprotein and also a member of the serine protease family, is overexpressed in cancer-related fibroblasts (CAFs) and activated fibroblasts in wound healing/inflammation sites. As a broad-spectrum cancer target, it is expressed in 90% or more epithelial tumor microenvironments, including pancreatic, colon, breast, ENT (ear-nose-throat) cancers, etc. In 2019, Kratochwil et al. clinically confirmed that a quinoline-containing ⁶⁸Ga-FAPI-04 small-molecular nuclear drug can detect as many as 28 different cancers (*J. Nucl. Med.* 2019, 60, 801). Due to its low expression in normal tissues, FAP-α can not only act as a biomarker for tumor diagnosis and prognosis, but also be a promising therapeutic target for radiotherapy. Since Lindner et al. first applied FAPI-04 to clinic in 2018 (J. Nucl. Med. 2018, 59, 1415), PET imaging and radiotherapy based on FAP-α inhibitors (FAPIs) have been studied and used for various cancer and non-cancer diseases. In addition to the previously mentioned PSMAs and SSTRs, specific and highly expressed tumor-associated targets similar to FAP targets further include targets that have been found in corresponding RDC reports, such as integrins (Intergrin, Front Oncol, 2022, 12, 837952), chemokine receptor 4 (CXCR4, Chem Med Chem 2011, 6, 1789), etc. These are all targets for the specific trifunctional compound disclosed in the present patent.

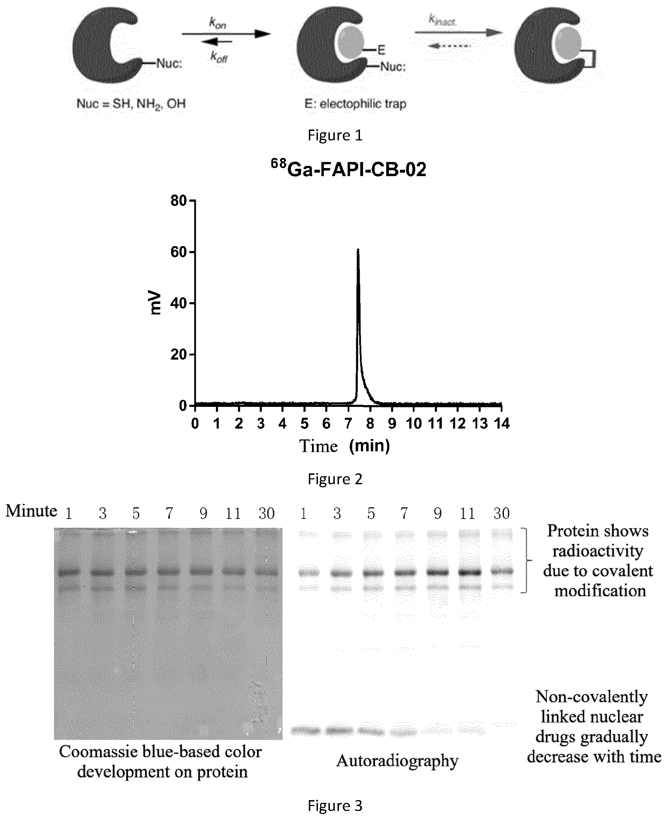

Covalent inhibitors are a class of inhibitors that can irreversibly bind to target protein residues via covalent bonds and thus exert their biological functions. Such inhibitors are mostly small-molecular kinase inhibitors in form (Eur. J. Med. Chem. 2017, 138, 96). Generally, covalent inhibition is a two-step process (Fig. 1): firstly, the inhibitor reversibly binds to the target enzyme, thus bringing the warhead in the small molecule close to the active residue in the enzyme; and in the second step, the warhead of the inhibitor undergoes a bond-forming reaction with the residue to form a covalent bond. Common nucleophilic amino acid residues that can undergo covalent reactions include cysteine, serine, tyrosine, lysine, arginine, glutamic acid, etc. Common covalent warheads are generally electrophiles, which can be divided into two categories according to reversible/irreversible covalent bonds formed by covalent warheads. One category is warheads that generally form irreversible covalent bonds, for example, acrylamide, epoxy, chloroacetyl, and sulfonyl fluoride; and the other category is warheads that generally form reversible covalent bonds, for example, cyano and ketocarbonyl. Covalent warheads that can be used *in vivo* and eventually become drugs are generally "latent electrophiles", i.e., those that can be activated to accelerate covalent bond formation only when they bind to specific proteins, which have good bio-orthogonality, thus minimizing the off-target toxicity related to warhead activity. In 2014, Sharpless et al. developed a new class of "latent electrophile" fluorosulfates (Angew. Chem. Int. Ed. 2014, 53, 9430) based on the click chemistry of hexavalent sulfur (SuFEx). Compared with the most common acrylamide-based covalent warheads, which react only with cysteine residues, they not only have better *in vivo* stability but can also form covalent linkage with a broader range of nearby lysine, histidine, tyrosine, and serine residues after binding to the target protein and are expected to become a new class of covalent drug warheads.

On the basis of the prior art, innovative research is conducted in the present disclosure to obtain a trifunctional compound by introducing a covalent warhead such as a fluorosulfate into a drug. The uptake and retention of the trifunctional compound at a target site such as at a tumor are substantially enhanced, while still ensuring consistently low uptake at non-target organs, thus exhibiting substantially reduced toxicity compared with traditional strategies for enhancing retention in tumors.

### Summary of the Invention

In one aspect, the present disclosure provides a trifunctional compound or a pharmaceutically acceptable salt, stereoisomer or solvate thereof, wherein the trifunctional compound has a structure of general formula (I), (II), (III), (IV), or (V), 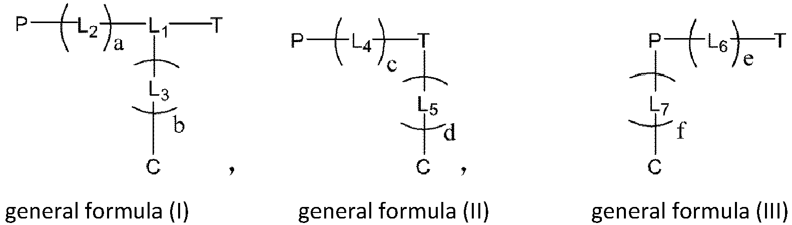 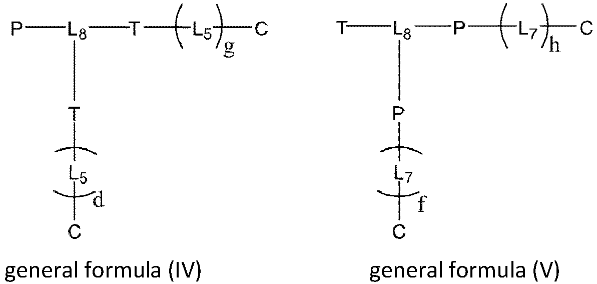 wherein P is a payload, T is a targeting moiety, C is a covalent warhead, L₁ and L₈ are independently linker units, and a to h are independently integers of 0 to 6.

In another aspect, the present disclosure provides a pharmaceutical composition comprising the trifunctional compound or the pharmaceutically acceptable salt, stereoisomer or solvate as described above, and a pharmaceutically acceptable carrier.

In another aspect, the present disclosure provides a kit comprising the trifunctional compound or the pharmaceutically acceptable salt, stereoisomer or solvate as described above, or the pharmaceutical composition as described above In yet another aspect, the present disclosure provides a method for diagnosing or treating a disease, the method comprising administering to a subject a therapeutically effective amount of the trifunctional compound or the pharmaceutically acceptable salt, stereoisomer or solvate thereof as described above.

In yet another aspect, the present disclosure provides the use of the trifunctional compound or the pharmaceutically acceptable salt, stereoisomer or solvate thereof as described above for preparing a medicament for diagnosing or treating a disease.

In some embodiments, in the trifunctional compound, P is a payload comprising at least one radionuclide-containing group, or at least one chelating group capable of chelating a radionuclide, or at least one optical dye group;
T is a targeting moiety comprising at least one targeting group capable of targeting a protein or a cell or specific tissue with an unknown target, wherein the targeting group is a small molecule, a polypeptide, or a nucleic acid aptamer;
C is a covalent warhead capable of forming a reversible or irreversible covalent linkage with the protein or tissue targeted by T; and
L₁, L₂, L₃, L₄, L₅, L₆, L₇, and L₈ are each independently a linker unit.

In some embodiments, in the trifunctional compound, C is selected from the group consisting of the following groups: 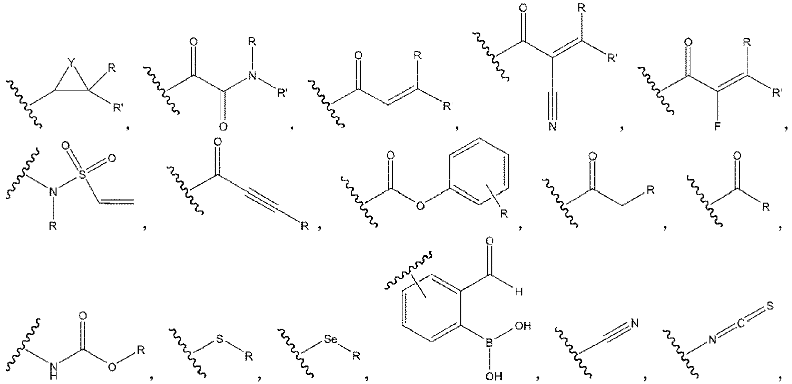 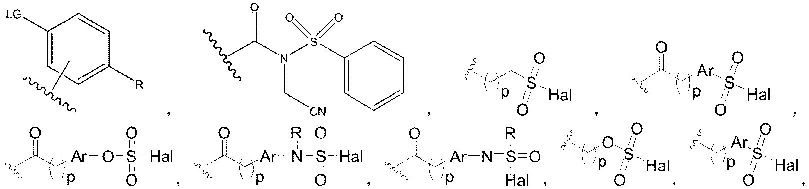 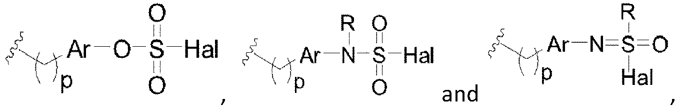 wherein:
Y is selected from the group consisting of O, S, and NR¹;
LG is a leaving group replaceable by a protein residue and is preferably halogen or OTs, with Ts being p-toluenesulfonyl;
R and R' are independently of each other selected from the group consisting of hydrogen, halogen, nitro, cyano, optionally substituted alkyl, optionally substituted cycloalkyl, optionally substituted heterocycloalkyl, optionally substituted aryl, optionally substituted heteroaryl, and optionally substituted amino;
R¹ is selected from the group consisting of hydrogen, optionally substituted alkyl,
optionally substituted cycloalkyl, optionally substituted heterocycloalkyl, optionally substituted aryl, and optionally substituted heteroaryl;
Ar is optionally substituted aryl or optionally substituted heteroaryl;
Hal is halogen, preferably F or Cl; and
p is an integer between 0 and 12.

In preferred embodiments, in the trifunctional compound, C is selected from the group consisting of the following groups: 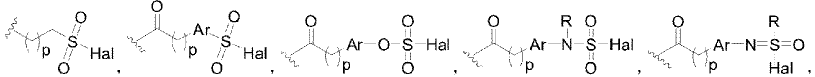 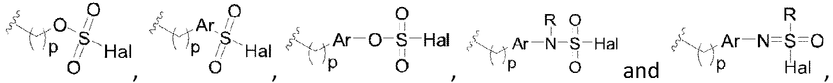 wherein:
Ar is optionally substituted C₆-C₁₀ aryl, preferably optionally substituted phenyl, or optionally substituted C₅-C₁₂ heteroaryl;
Hal is F;
p is an integer between 0 and 6; and
R is selected from the group consisting of hydrogen, optionally substituted alkyl, optionally substituted cycloalkyl, optionally substituted heterocycloalkyl, optionally substituted aryl, and optionally substituted heteroaryl.

In some other preferred embodiments, in the trifunctional compound, C is selected from the group consisting of the following groups: 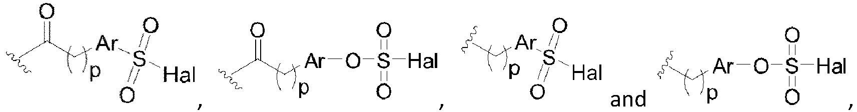 wherein:
Ar is phenyl;
Hal is F; and
p is an integer between 0 and 4.

In some embodiments, in the trifunctional compound, the targeting group targets a target selected from:
fibroblast activation protein-α (FAP-α), prostate-specific membrane antigen (PSMA), poliovirus receptor-associated protein 4 (Nectin-4), programmed death receptor ligand 1 (PD-L1), programmed death receptor 1 (PD-1), human epidermal growth factor receptor 2 (HER2), integrin, gastrin-releasing peptide receptor (GRPR), somatostatin receptor (SSTR) such as SSTR2, folate receptor (FR) such as folate receptor 1 (FOLR1), estrogen receptor (ER), cytotoxic T lymphocyte-associated protein 4 (CTLA-4), chemokine receptor 4 (CXCR4), poly(ADP-ribose) polymerase (PARP), epidermal growth factor receptor (EGFR), fibroblast growth factor receptor (FGFR), disialoganglioside (GD2), vascular endothelial growth factor (VEGF), metastasis-associated lung adenocarcinoma transcript 1 (MALAT-1), insulin growth factor 1 (IGF-1), mesothelin (MSLN), tumor endothelial marker (TEM-1), interleukin-12 (IL-12), leukocyte differentiation antigen CD 13 (CD13), leukocyte differentiation antigen CD 19 (CD19), leukocyte differentiation antigen CD 20 (CD20), leukocyte differentiation antigen CD 22 (CD22), leukocyte differentiation antigen CD 33 (CD33), leukocyte differentiation antigen CD 37 (CD37), leukocyte differentiation antigen CD 38 (CD38), leukocyte differentiation antigen CD 44 (CD44), death receptor 5 (DR5), peroxidase 1 (Prdxl), large neutral amino acid transporter 1 (LAT1), angiotensin converting enzyme 2 (ACE2), vesicular monoamine transporter 2 (VMAT2), dopamine transporter (DAT), and Sigma-1 receptor; or some cell lines whose protein targets are unknown but can be targeted.

The targeting group optionally comprises one or more warheads or structures capable of forming a reversible or irreversible covalent linkage with the targeted protein or tissue.

In preferred embodiments, in the trifunctional compound, the targeting group targets fibroblast activation protein-a, prostate-specific membrane antigen (PSMA), integrin, chemokine receptor 4 (CXCR4), folate receptor (FR) such as folate receptor 1 (FOLR1), poliovirus receptor-associated protein 4 (Nectin-4), or somatostatin receptor (SSTR) such as SSTR2, and is selected from the following structures:
group I: structures targeting fibroblast activation protein-α and having general formula (VI), preferably general formula (VII), 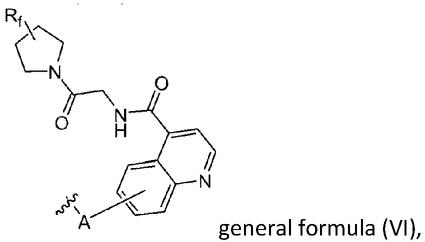
   wherein A is selected from O, S, and NR_{A}, with R_{A} being selected from H and C₁-C₆ alkyl,
   a plurality of R_{f} groups are present on the tetrahydropyrrole ring as shown, in which each R_{f} group is independently selected from H, F, Cl, -CN, and C₁-C₆ alkyl, and optionally two R_{f} groups on adjacent carbons are capable of being connected with each other to form cycloalkyl, preferably C₃-C₇ cycloalkyl; 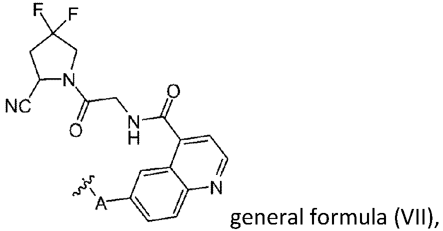
   wherein A is selected from O, S, and NR_{A} in which R_{A} is selected from H, methyl, ethyl, n-propyl, and isopropyl;
group II: peptidomimetic groups having general formula (VIII), 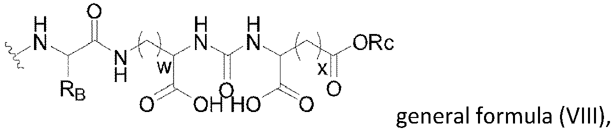
   wherein R_{B} is -CH₂R_{B'} in which R_{B'} is aryl, preferably optionally substituted phenyl or naphthyl,
   R_{c} is H or alkyl,
   w and x are independently 1, 2, 3, or 4, and
   the peptidomimetic group preferably has the following structure 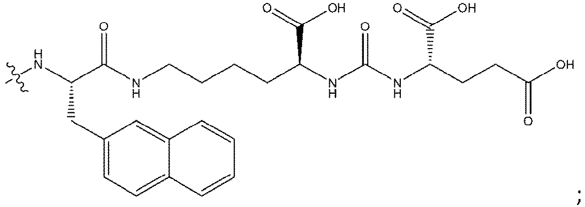
group III: cyclic peptide groups having general formula (X), 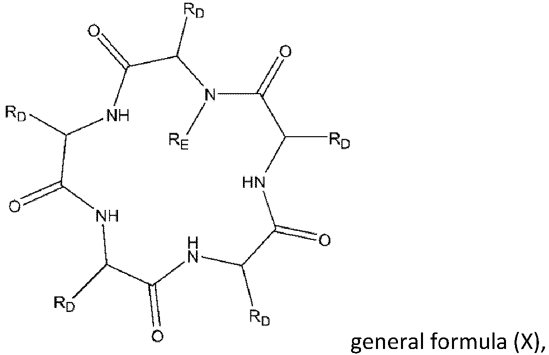
   wherein each R_{D} is independently selected from H, -CH₂R_{D'}, -CH₂COOH, -CH₂CH₂CH₂NH₂,-CH₂CH₂CH₂CH₂NH₂, and 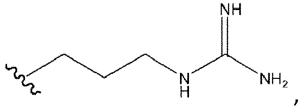 with R_{D'} being aryl, preferably
   optionally substituted phenyl or naphthyl, wherein the cyclic peptide is connected to the rest of the trifunctional compound via R_{D'} and/or -NH₂ at an end of R_{D}, or -CH₂R_{D'} or R_{D'} is capable of forming part of covalent warhead C;
   R_{E} is H or methyl;
   the cyclic peptide group preferably has the following formula 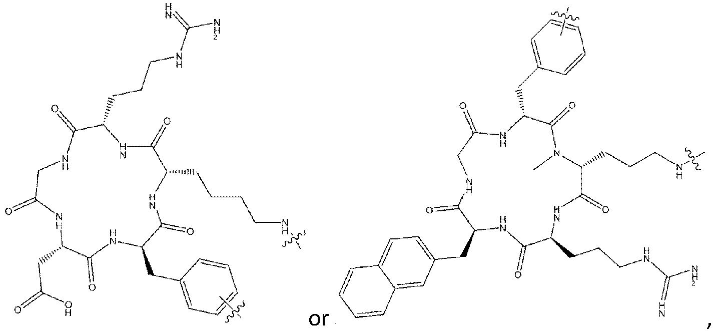
   wherein two wavy lines in each formula represent optional sites of attachment;
group IV: folic groups having general formula (XI), 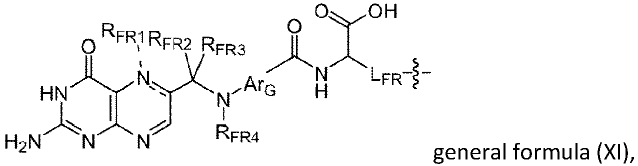
   wherein the dashed line represents the presence or absence of the bond, and only one of the bonds shown by the two dashed lines exists,
   R_{FR1}, when present, is H or C₁-C₄ alkyl,
   R_{FR2}, R_{FR3}, and R_{FR4} are each independently selected from H or C₁-C₄ alkyl,
   Ar_{G} is aryl or heteroaryl, preferably optionally substituted phenyl or pyridyl,
   L_{FR} is -CH₂-R_{FR5}-, -(CH₂)₂-R_{FR5}-, -(CH₂)₃-R_{FR5}-, -(CH₂)₃-R_{FR5}-, or -(CH₂)₄-R_{FR5}-, in which R_{FR5} is selected from carbonyl (-CO-) or NR_{FR6}, and R_{FR6} is H or C₁-C₄ alkyl, and
   the folic group preferably has the following structure 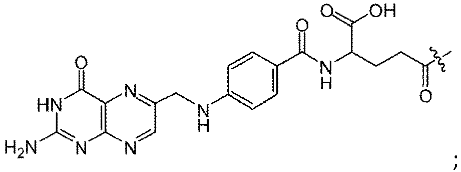
group V: disulfide bond-containing cyclic groups having general formula (XII), 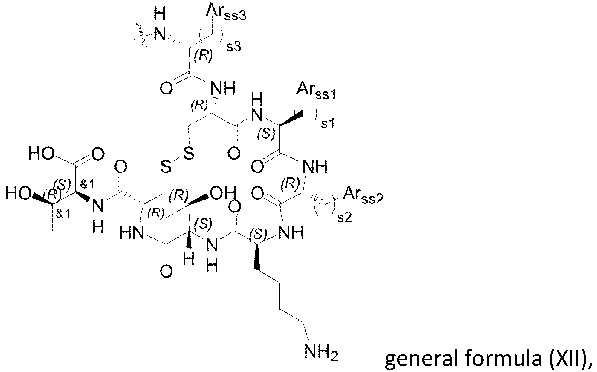
   wherein Arₛₛ₁, Arₛₛ₂, and Arₛₛ₃ are each independently selected from optionally substituted aryl or heteroaryl, preferably optionally substituted C₆-C₁₀ aryl or C₅-C₁₀ heteroaryl, preferably Arₛₛ₁ and Arₛₛ₃ are C₆-C₁₀ aryl optionally substituted with -OH, and Arₛₛ₂ is C₅-C₁₀ heteroaryl,
   s1, s2, and s3 are each independently selected from 1, 2, 3, or 4,
   wherein the disulfide bond-containing cyclic group is connected to the rest of the trifunctional compound via a site shown by the wavy line, or -(CH₂)ₛ₃Arₛₛ₃ or Arₛₛ₃ is capable of forming part of covalent warhead C,
   the disulfide bond-containing cyclic group preferably has the following structure 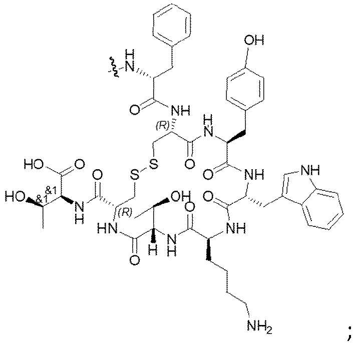 and
group VI: bicyclic peptide groups, preferably bicyclic peptide groups targeting poliovirus receptor-associated protein 4 (Nectin-4),
   more preferably, the bicyclic peptide group has the following structure

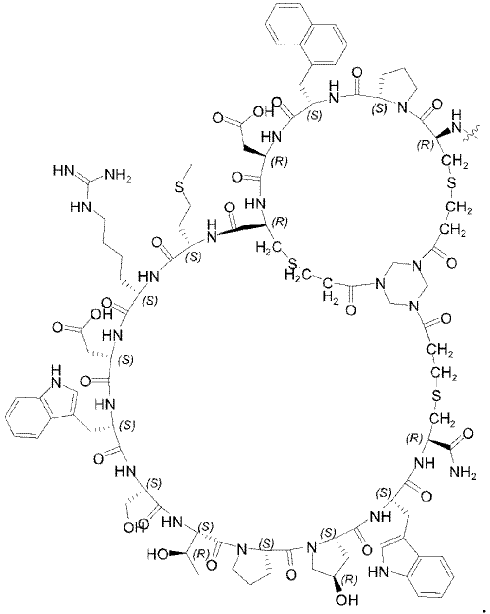

In some embodiments, in the trifunctional compound, the payload comprises at least one group selected from the following groups:
group I: groups with at least one radionuclide group, selected from: 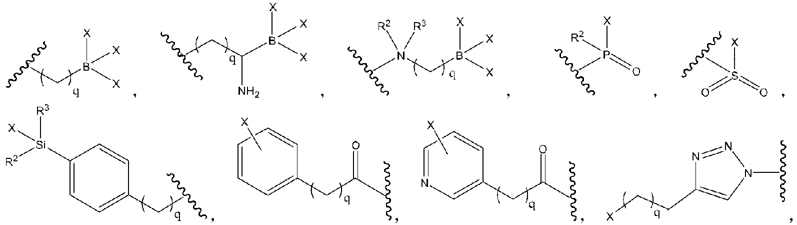 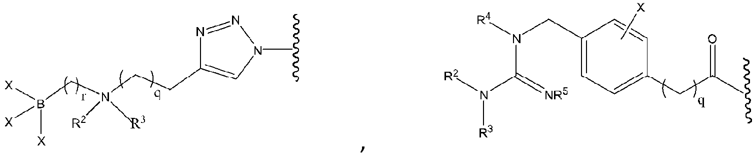 and 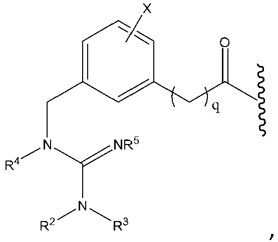
   wherein R², R³, R⁴, and R⁵ are independently of each other selected from the group consisting of hydrogen, optionally substituted alkyl, optionally substituted cycloalkyl, optionally substituted heterocycloalkyl, optionally substituted aryl, and optionally substituted heteroaryl,
   X is selected from ¹⁸F, ¹²³I, ¹²⁴I, ¹²⁵I, ¹³¹I, and ²¹¹At, or non-radioactive isotopes thereof,
   q and r are independently an integer between 0 and 4; and
   N on the triazole ring at the site of attachment as shown here may not exist on the payload, but on the linker;
group II: chelating groups capable of chelating a radionuclide, selected from: 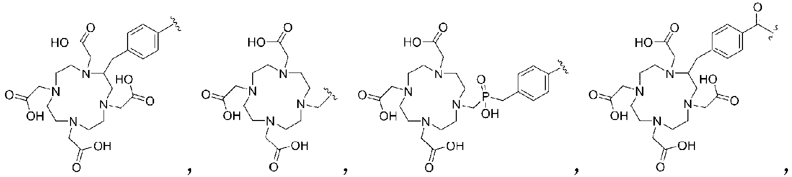 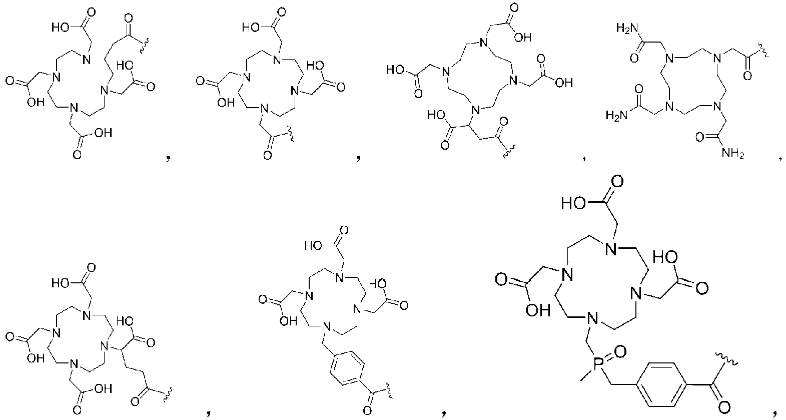 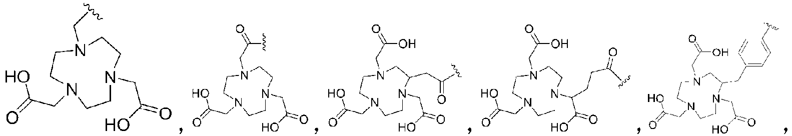 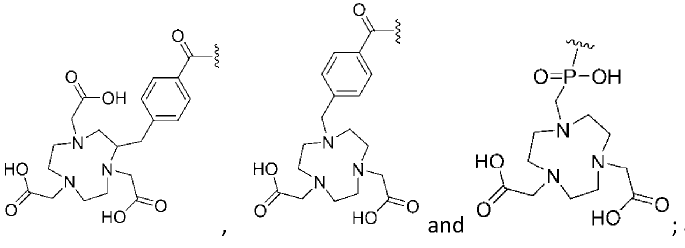 and
group III: optical dye groups selected from:

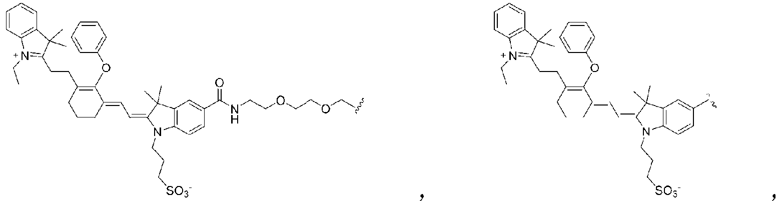

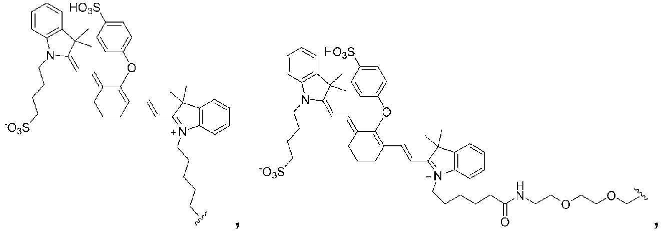

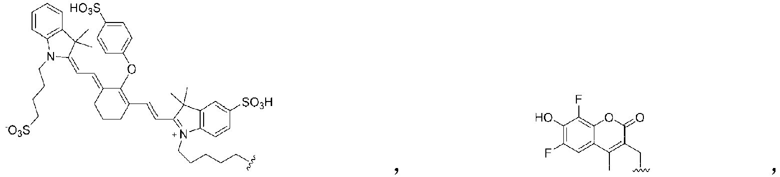

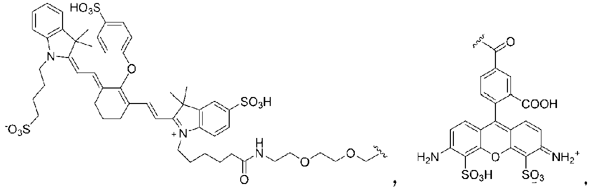

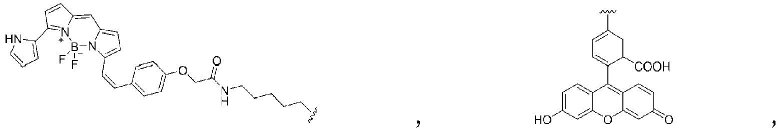

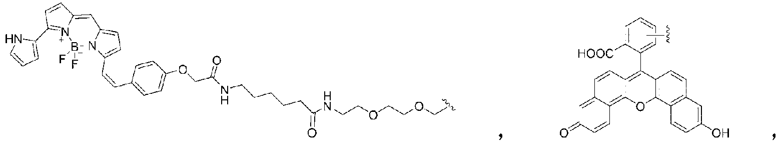

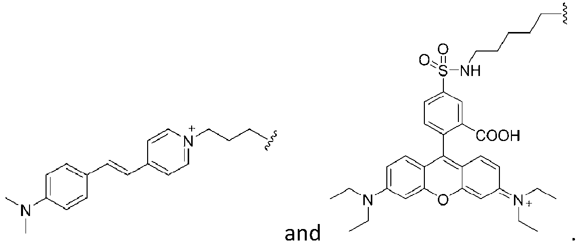

In some embodiments, in the trifunctional compound, L₁ and L₈ are independently selected from the following groups: 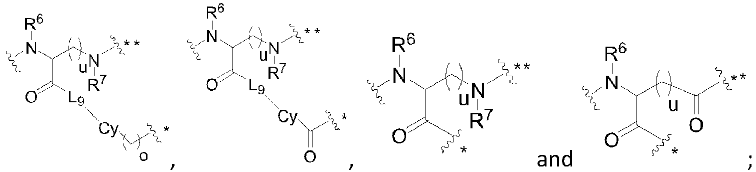 L₂, L₃, L₄, L₅, L₆, and L₇ are each independently selected from the following groups: a single bond, 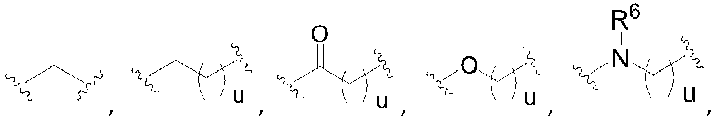 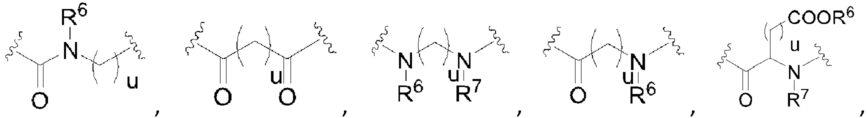 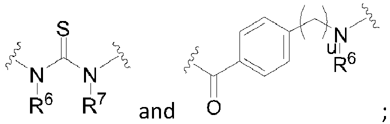 L₉ is a single bond, -CH₂-, -NHCH₂-, or 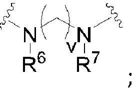 Cy is selected from 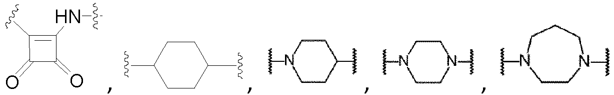 and 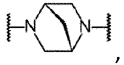
a to h are independently 0, 1, 2, or 3,
u, v, and o are independently 1, 2, 3, 4, or 5,
R⁶ and R⁷ are independently of each other selected from the group consisting of hydrogen,
optionally substituted alkyl, optionally substituted cycloalkyl, optionally substituted heterocycloalkyl, optionally substituted aryl, and optionally substituted heteroaryl, and
* and ** show sites optionally linked to two moieties of the trifunctional compound via L₂, L₃, L₄, L₅, L₆, or L₇,
provided that in linker groups formed from L₁, L₂, L₃, L₄, L₅, L₆, L₇, and L₈, heteroatoms are not directly connected by a covalent bond, and the heteroatoms are selected from N, O, and S.

In preferred embodiments, the trifunctional compound has a structure of general formula (I), wherein at least one L₁ is 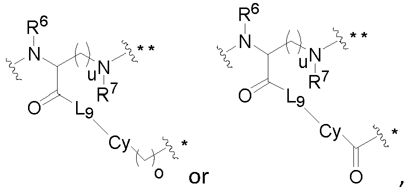 L₁ preferably has the following structure: 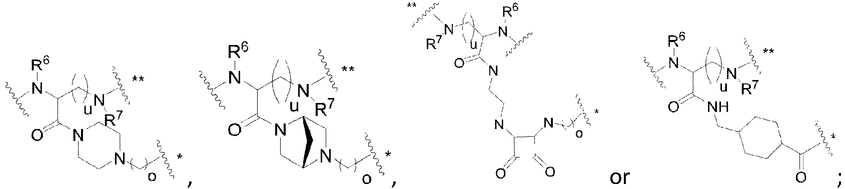 wherein R⁶, R⁷, L₉, Cy, a, b, o, and u are as defined above.

In some embodiments, the trifunctional compound has a structure of general formula (I): 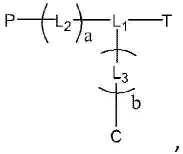 general formula (I)
wherein:
L₁ as shown is a trivalent linker unit,
L₂ and L₃ are each independently a divalent linker unit;
a and b are independently an integer between 0 and 6;
C as shown is a covalent warhead selected from the group consisting of the following groups: 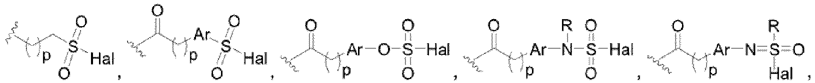 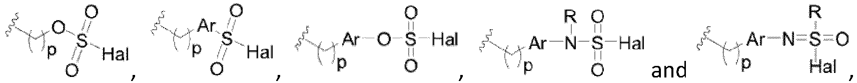 wherein:
   Ar is optionally substituted C₆-C₁₀ aryl, preferably optionally substituted phenyl, or
   optionally substituted C₅-C₁₂ heteroaryl;
   Hal is F or Cl;
   p is an integer between 0 and 6;
   R is selected from the group consisting of hydrogen, optionally substituted alkyl, optionally substituted cycloalkyl, optionally substituted heterocycloalkyl, optionally substituted aryl, and optionally substituted heteroaryl;
   T as shown is a targeting group targeting fibrocyte activation protein-α (FAP-α); and
   P as shown is a chelating group capable of chelating a radionuclide.

In preferred embodiments, the trifunctional compound has a structure of general formula (I),
wherein:
L₁ is a trivalent linker group selected from: 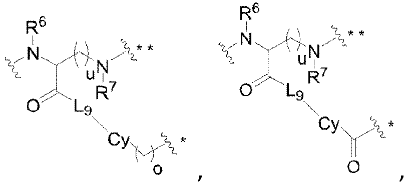 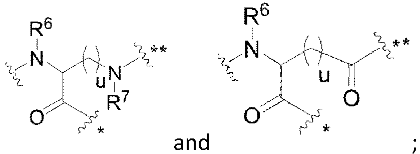
L₂ and L₃ are each independently selected from the following groups:
a single bond, 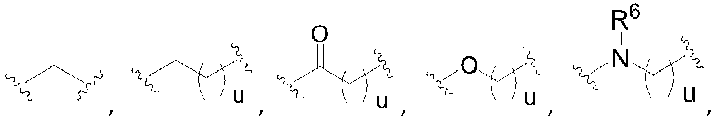 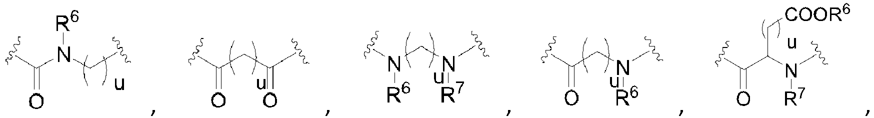 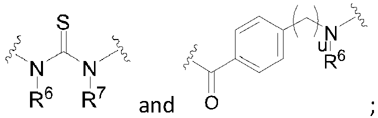
L₉ is a single bond, -CH₂-, -NHCH₂-, or 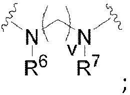
Cy is selected from 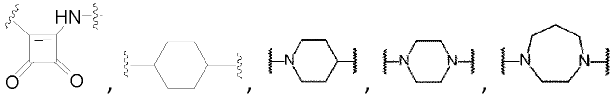 and 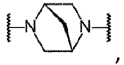
a, b, and p are independently 0, 1, 2, or 3,
u is independently 1, 2, 3, 4, or 5,
R⁶ and R⁷ are independently of each other selected from the group consisting of hydrogen,
optionally substituted alkyl, optionally substituted cycloalkyl, optionally substituted heterocycloalkyl, optionally substituted aryl, and optionally substituted heteroaryl,
* is the site of attachment to T,
** is the site of attachment to C,
provided that in the linkers formed by L₁, L₂, and L₃, heteroatoms are not directly connected by a covalent bond, and the heteroatoms are selected from N, O, and S; C is selected from the group consisting of the following groups: 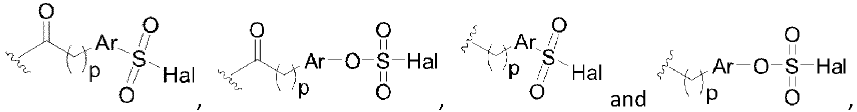 wherein:
   Ar is phenyl;
   Hal is F;
   p is an integer between 0 and 4;
   T as shown is a targeting group targeting fibrocyte activation protein-α (FAP-α); and
   P as shown is a chelating group capable of chelating a radionuclide and is selected from the following groups: 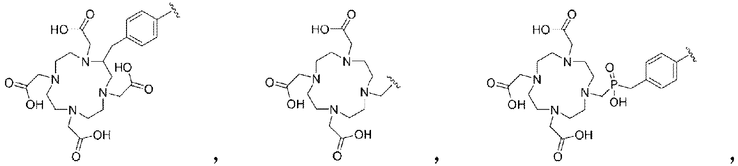 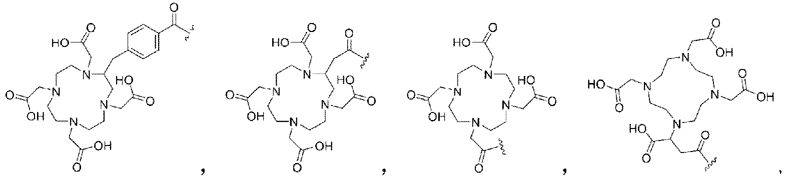 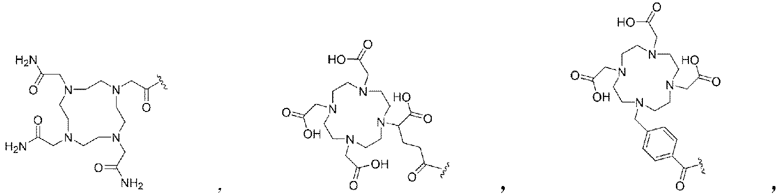 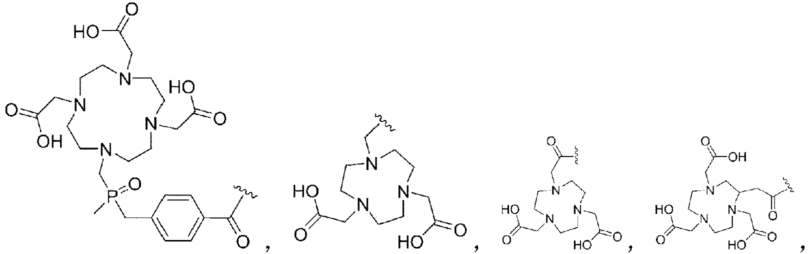 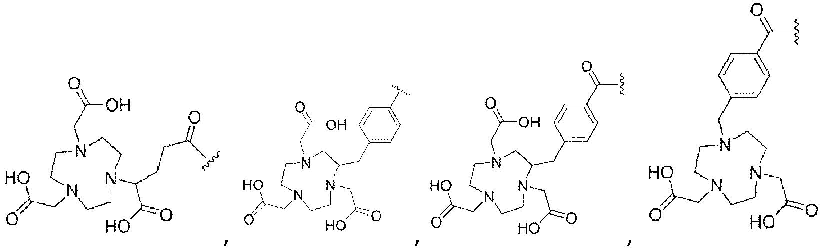 and

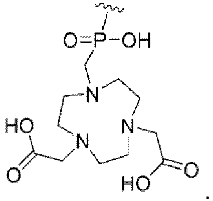

In preferred embodiments, the trifunctional compound has a structure of general formula (I),
wherein:
C and P have the meanings as defined previously,
L₁ has the following structure: 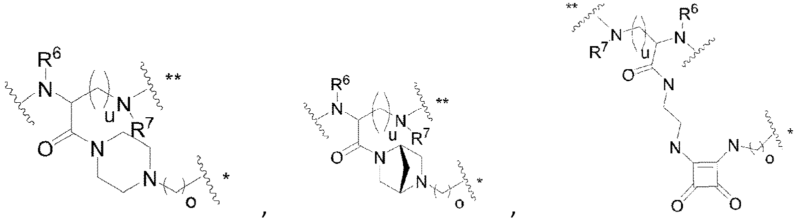 or 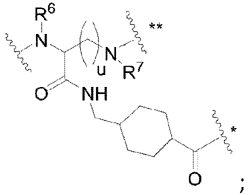
a and b are both 1,
L₂ and L₃ are independently the following groups:
a single bond, 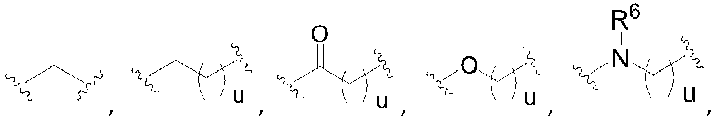 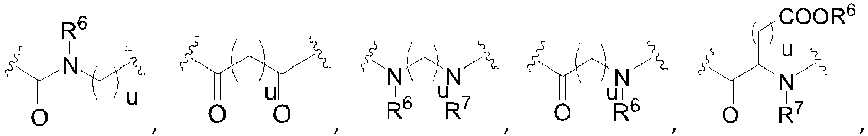 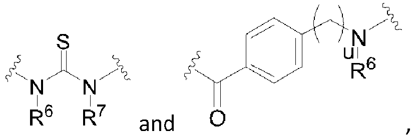
   wherein u and o are independently 1, 2, 3, 4, or 5,
   R⁶ and R⁷ are independently of each other selected from hydrogen and optionally substituted C₁-C₄,
   * is the site of attachment to T,
   ** is the site of attachment to C,
   provided that in the linkers formed by L₁, L₂, and L₃, heteroatoms are not directly connected by a covalent bond, and the heteroatoms are selected from N, O, and S;
   T has general formula (VI), preferably general formula (VII), 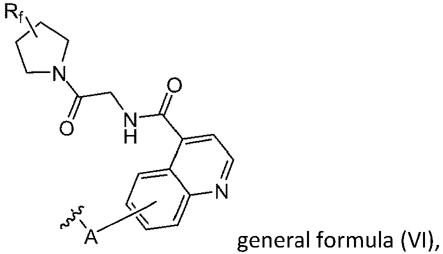
      wherein A is selected from O, S, and NR_{A}, with R_{A} being selected from H and C₁-C₆ alkyl,
      a plurality of R_{f} groups are present on the tetrahydropyrrole ring as shown, in which each R_{f} group is independently selected from H, F, Cl, -CN, and C₁-C₆ alkyl, and optionally two R_{f} groups on adjacent carbons are capable of being connected with each other to form cycloalkyl, preferably C₃-C₇ cycloalkyl; 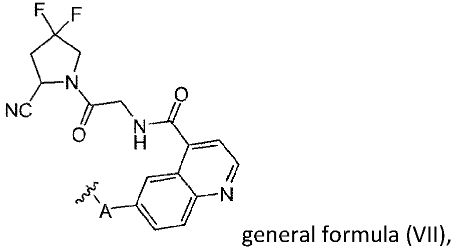
      wherein A is selected from O, S, and NR_{A} in which R_{A} is selected from H, methyl, ethyl, n-propyl, and isopropyl.

In some embodiments, the trifunctional compound has a structure of general formula (I),
wherein:
L₁ is a trivalent linker group selected from: 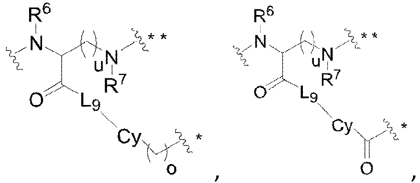 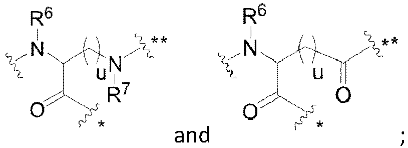
L₂ and L₃ are each independently selected from the following groups:
a single bond, 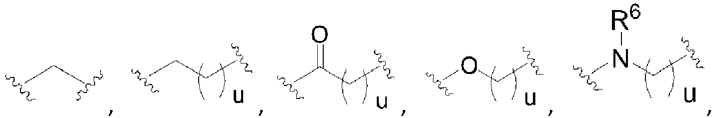 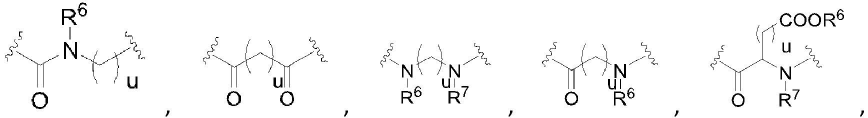 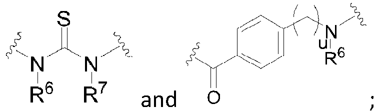
L₉ is a single bond, -CH₂-, -NHCH₂-, or 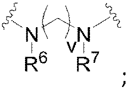
Cy is selected from 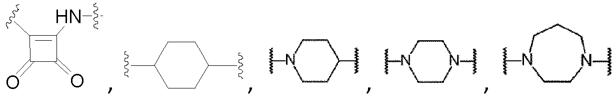 and 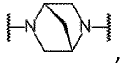
   a and b are independently 0, 1, 2, or 3,
   u is independently 1, 2, 3, 4, or 5,
   R⁶ and R⁷ are independently of each other selected from the group consisting of hydrogen,
   optionally substituted alkyl, optionally substituted cycloalkyl, optionally substituted heterocycloalkyl, optionally substituted aryl, and optionally substituted heteroaryl,
   * is the site of attachment to T,
   ** is the site of attachment to C,
   provided that in the linkers formed by L₁, L₂, and L₃, heteroatoms are not directly connected by a covalent bond, and the heteroatoms are selected from N, O, and S;
   C is selected from the group consisting of the following groups: 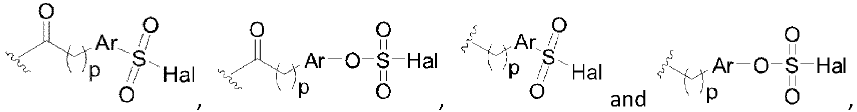 wherein:
      Ar is phenyl;
      Hal is F;
      p is an integer between 0 and 4;
      T as shown is a targeting group targeting prostate-specific membrane antigen (PSMA);
      P as shown is a chelating group capable of chelating a radionuclide and is selected from the following groups: 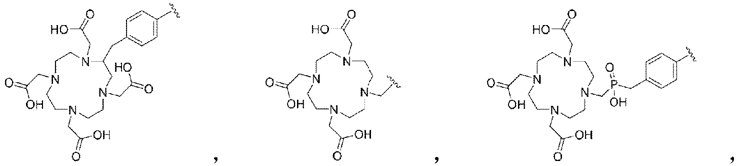 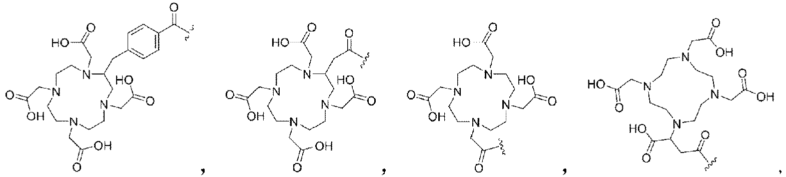 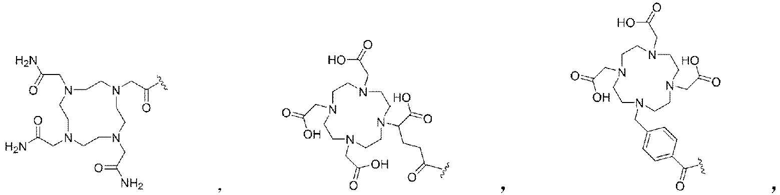 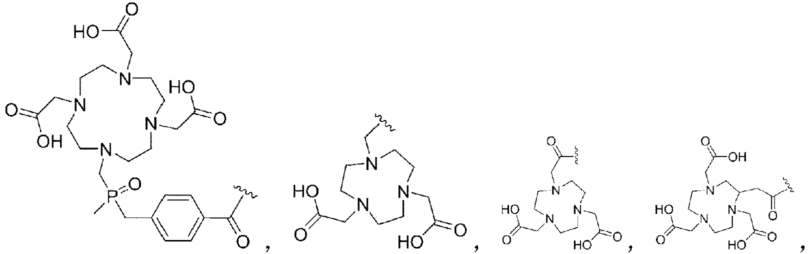 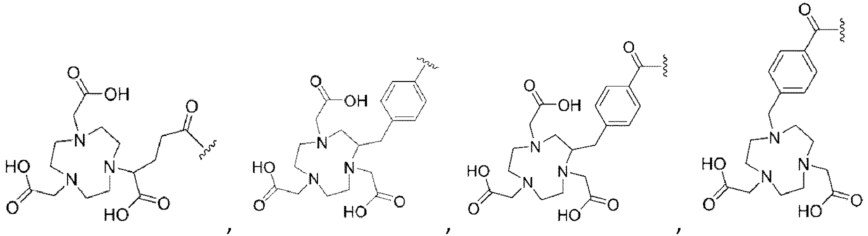 and

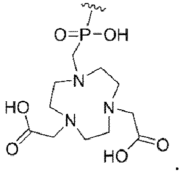

In preferred embodiments, the trifunctional compound has a structure of general formula (I),
wherein:
C and P have the meanings as defined previously,
L₁ has the following structure: 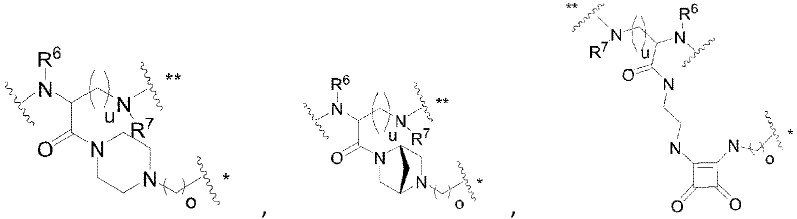 or 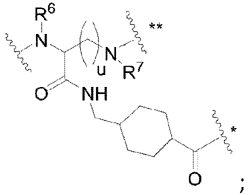
   a and b are both 1,
   L₂ and L₃ are independently the following groups:
   a single bond, 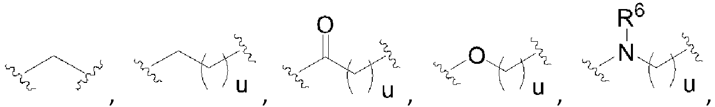 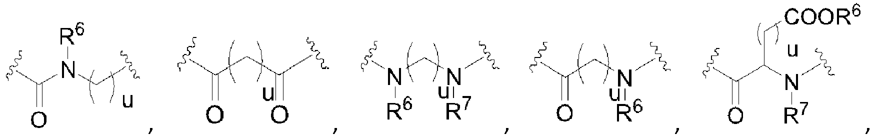 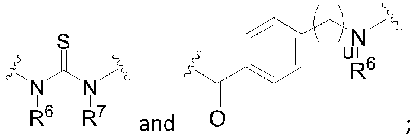
      wherein u and o are independently 1, 2, 3, 4, or 5,
      R⁶ and R⁷ are independently of each other selected from hydrogen and optionally substituted C₁-C₄,
      * is the site of attachment to T,
      ** is the site of attachment to C,
      provided that in the linkers formed by L₁, L₂, and L₃, heteroatoms are not directly connected by a covalent bond, and the heteroatoms are selected from N, O, and S;
      T has general formula (VIII), 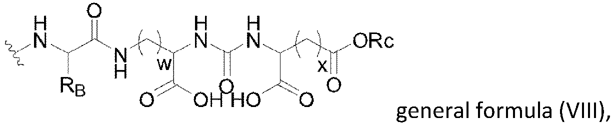
         wherein R_{B} is -CH₂R_{B'} in which R_{B'} is aryl, preferably optionally substituted phenyl or naphthyl,
         R_{c} is H or alkyl,
         w and x are independently 1, 2, 3, or 4, and
         T preferably has the following structure

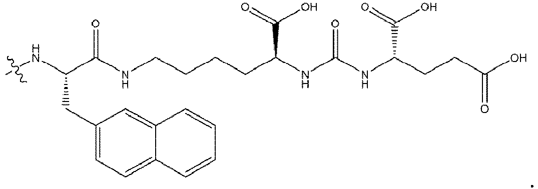

In some embodiments, the trifunctional compound has a structure of general formula (I),
wherein:
L₁ is a trivalent linker group selected from: 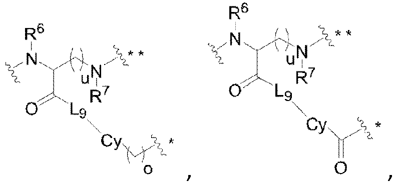 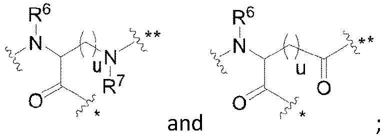
L₂ and L₃ are each independently selected from the following groups:
a single bond, 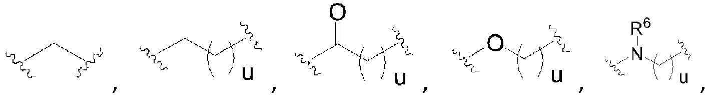 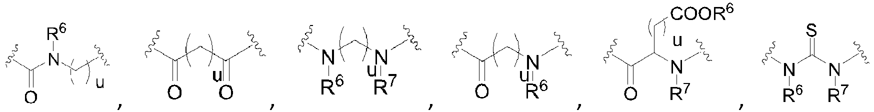 and 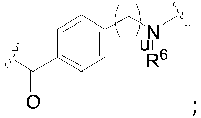
L₉ is a single bond, -CH₂-, -NHCH₂-, or 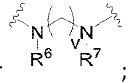
Cy is selected from 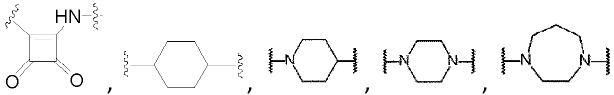 and 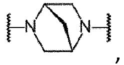
   a and b are independently 0, 1, 2, or 3,
   u is independently 1, 2, 3, 4, or 5,
   R⁶ and R⁷ are independently of each other selected from the group consisting of hydrogen,
   optionally substituted alkyl, optionally substituted cycloalkyl, optionally substituted heterocycloalkyl, optionally substituted aryl, and optionally substituted heteroaryl,
   * is the site of attachment to C,
   ** is the site of attachment to P,
   provided that in the linkers formed by L₁, L₂, and L₃, heteroatoms are not directly connected by a covalent bond, and the heteroatoms are selected from N, O, and S;
   C is selected from the group consisting of the following groups: 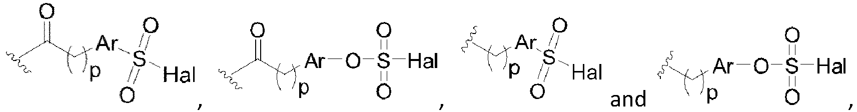 wherein:
      Ar is phenyl;
      Hal is F;
      p is an integer between 0 and 4;
      T as shown is a targeting group targeting folate receptor (FR) such as folate receptor 1 (FOLR1);
      P as shown is a chelating group capable of chelating a radionuclide and is selected from the following groups: 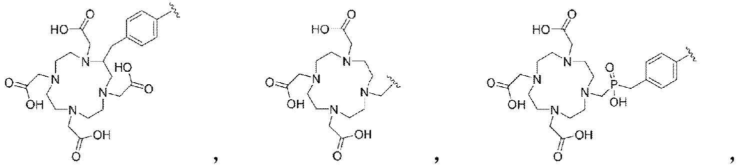 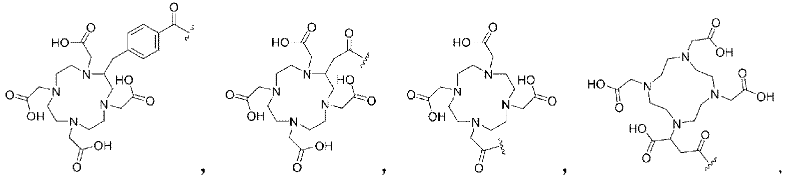 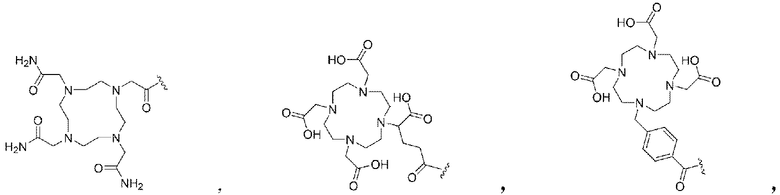 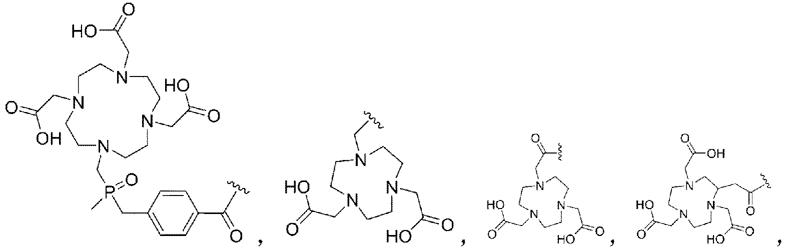 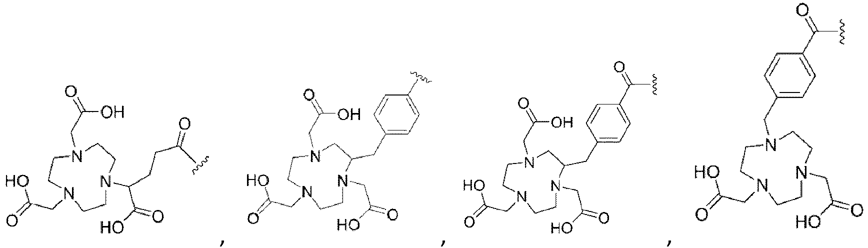 and

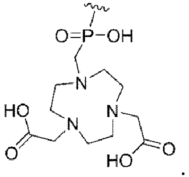

In preferred embodiments, the trifunctional compound has a structure of general formula (I),
wherein:
C and P have the meanings as defined previously,
L₁ has the following structure: 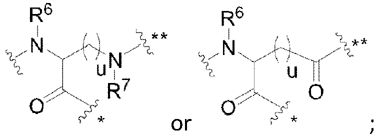
   a and b are both 1,
   L₂ and L₃ are independently the following groups:
   a single bond, 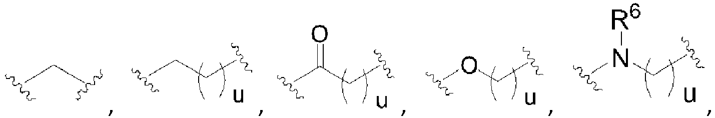 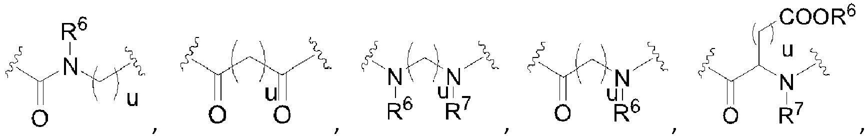 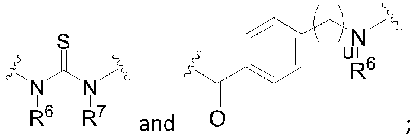
      wherein u is independently 1, 2, 3, 4, or 5,
      R⁶ and R⁷ are independently of each other selected from hydrogen and optionally substituted C₁-C₄,
      * is the site of attachment to C,
      ** is the site of attachment to P,
      provided that in the linkers formed by L₁, L₂, and L₃, heteroatoms are not directly connected by a covalent bond, and the heteroatoms are selected from N, O, and S;
      T has general formula (XI), 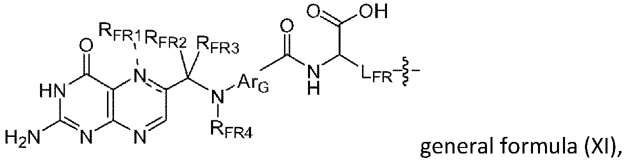
         wherein the dashed line represents the presence or absence of the bond, and only one of the bonds shown by the two dashed lines exists,
         R_{FR1}, when present, is H or C₁-C₄ alkyl,
         R_{FR2}, R_{FR3}, and R_{FR4} are each independently selected from H or C₁-C₄ alkyl,
         Ar_{G} is aryl or heteroaryl, preferably optionally substituted phenyl or pyridyl,
         L_{FR} is -CH₂-R_{FR5}-, -(CH₂)₂-R_{FR5}-, -(CH₂)₃-R_{FR5}-, -(CH₂)₃-R_{FR5}-, or -(CH₂)₄-R_{FR5}-, in which R_{FR5} is selected from carbonyl (-CO-) or NR_{FR6}, and R_{FR6} is H or C₁-C₄ alkyl, and
         T preferably has the following structure

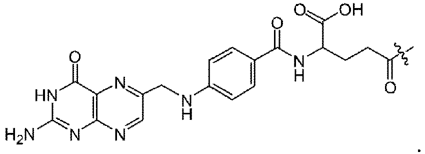

In some embodiments, the trifunctional compound has a structure of general formula (I),
wherein:
L₁ is a trivalent linker group selected from: 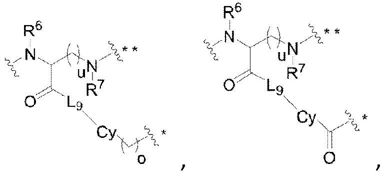 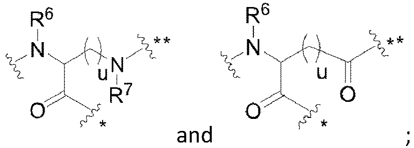
L₂ and L₃ are each independently selected from the following groups:
a single bond, 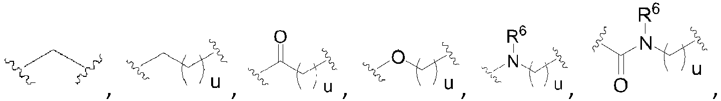 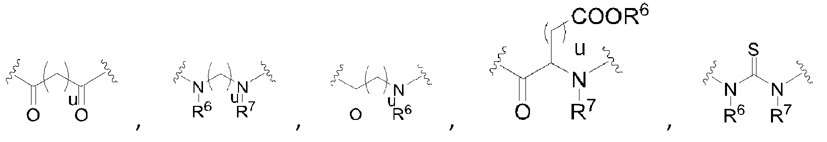 and 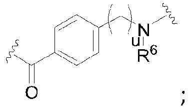
L₉ is a single bond, -CH₂-, -NHCH₂-, or 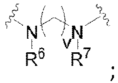
Cy is selected from 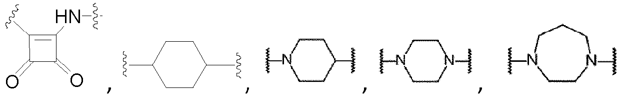 and 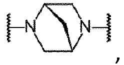
   a and b are independently 0, 1, 2, or 3,
   u is independently 1, 2, 3, 4, or 5,
   R⁶ and R⁷ are independently of each other selected from the group consisting of hydrogen,
   optionally substituted alkyl, optionally substituted cycloalkyl, optionally substituted heterocycloalkyl, optionally substituted aryl, and optionally substituted heteroaryl,
   * is the site of attachment to T,
   ** is the site of attachment to C,
   provided that in the linkers formed by L₁, L₂, and L₃, heteroatoms are not directly connected by a covalent bond, and the heteroatoms are selected from N, O, and S;
   C is selected from the group consisting of the following groups: 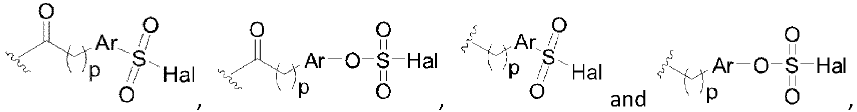 wherein:
      Ar is phenyl;
      Hal is F;
      p is an integer between 0 and 4;
      T as shown is a targeting group targeting poliovirus receptor-associated protein 4 (Nectin-4);
      P as shown is a chelating group capable of chelating a radionuclide and is selected from the following groups: 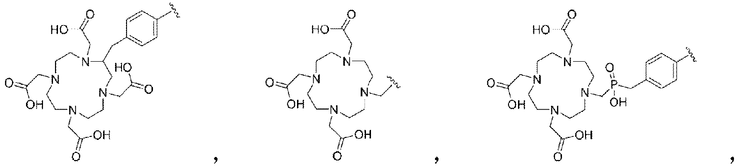 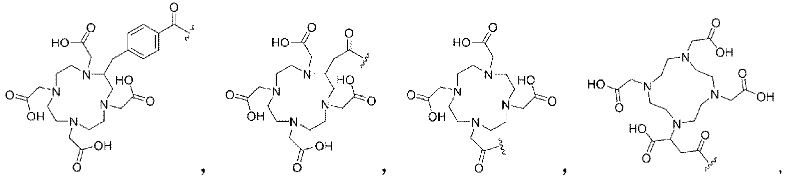 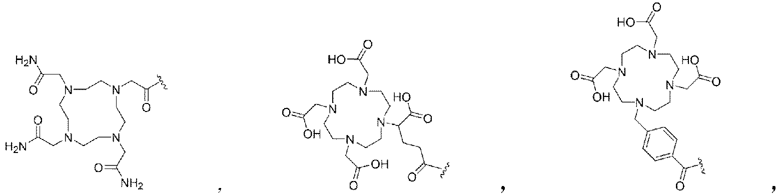 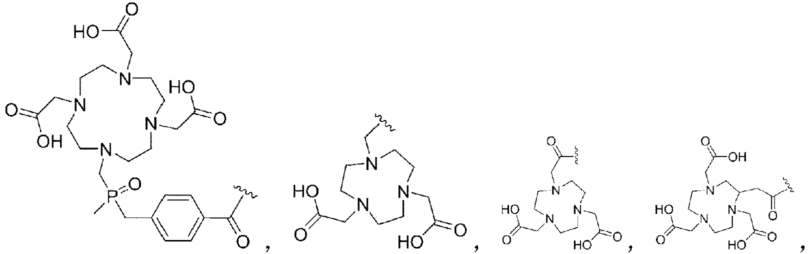 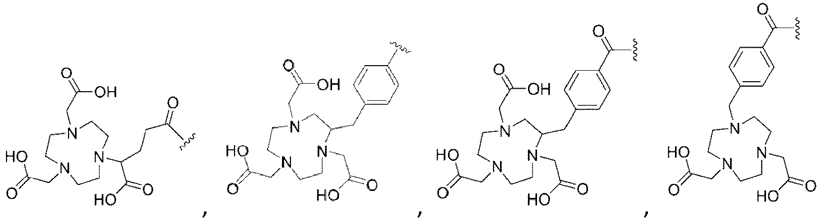 and

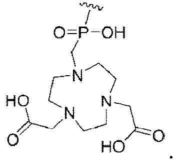

In preferred embodiments, the trifunctional compound has a structure of general formula (I),
wherein:
C and P have the meanings as defined previously,
L₁ has the following structure: 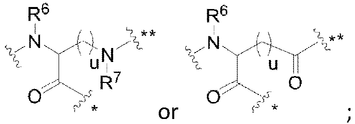
   a and b are both 1,
   L₂ and L₃ are independently the following groups:
   a single bond, 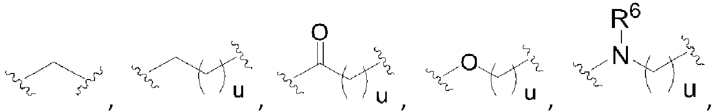 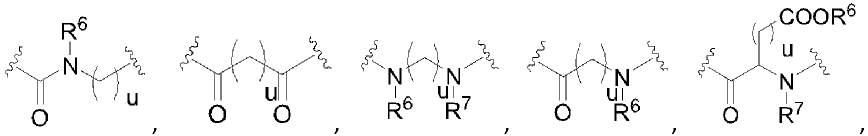 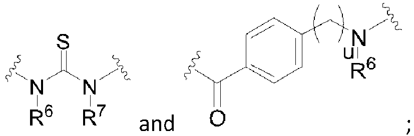
      wherein u is independently 1, 2, 3, 4, or 5,
      R⁶ and R⁷ are independently of each other selected from hydrogen and optionally substituted C₁-C₄,
      * is the site of attachment to T,
      ** is the site of attachment to C,
      provided that in the linkers formed by L₁, L₂, and L₃, heteroatoms are not directly connected by a covalent bond, and the heteroatoms are selected from N, O, and S;
      T is a bicyclic peptide group, preferably a bicyclic peptide group targeting poliovirus receptor-associated protein 4 (Nectin-4),
      more preferably, the bicyclic peptide group has the following structure

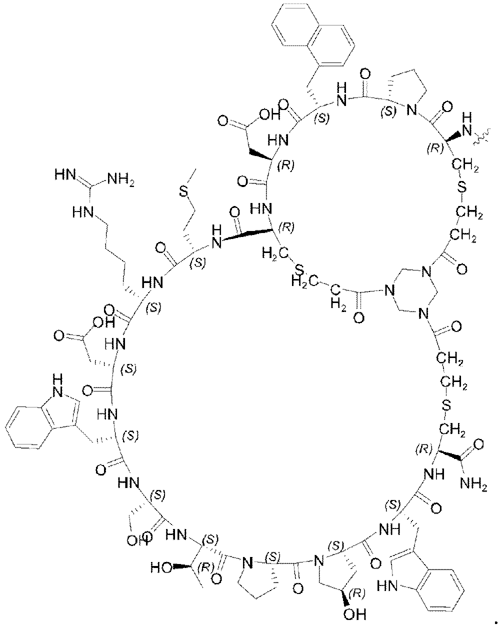

In some embodiments, the trifunctional compound has a structure of general formula (II): 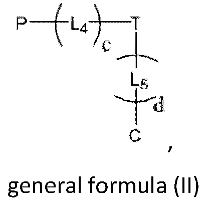 wherein:
L₄ and L₅ as shown are each independently a divalent linker unit;
c and d are independently integers of 0 to 6;
C as shown is a covalent warhead selected from the group consisting of the following groups: 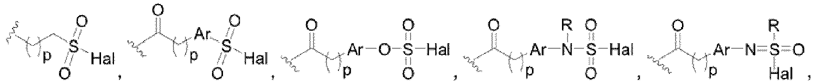 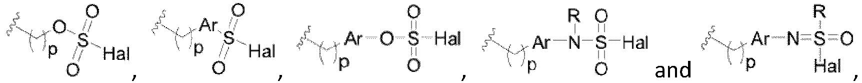 wherein:
   Ar is optionally substituted C₆-C₁₀ aryl, preferably optionally substituted phenyl, or optionally substituted C₅-C₁₂ heteroaryl;
   Hal is F or Cl;
   p is an integer between 0 and 6;
   R is selected from the group consisting of hydrogen, optionally substituted alkyl, optionally substituted cycloalkyl, optionally substituted heterocycloalkyl, optionally substituted aryl, and optionally substituted heteroaryl;
   T as shown is a targeting group targeting chemokine receptor 4 (CXCR4); and
   P as shown is a chelating group capable of chelating a radionuclide.

In preferred embodiments, the trifunctional compound has a structure of general formula (II),
wherein:
L₄ and L₅ are each independently selected from the following groups:
a single bond, 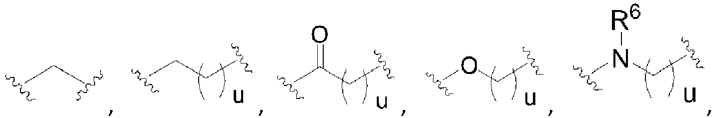 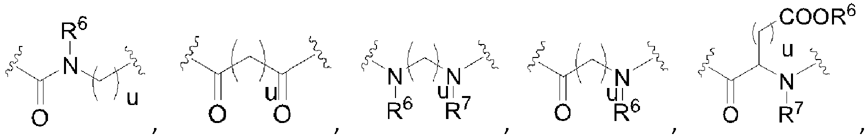 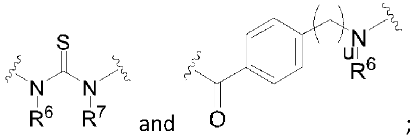
   c and d are independently 0, 1, 2, or 3,
   u is independently 1, 2, 3, 4, or 5,
   R⁶ and R⁷ are independently of each other selected from the group consisting of hydrogen, optionally substituted alkyl, optionally substituted cycloalkyl, optionally substituted heterocycloalkyl, optionally substituted aryl, and optionally substituted heteroaryl,
   provided that in the linker formed by L₄ and L₅, heteroatoms are not directly connected by a covalent bond, and the heteroatoms are selected from N, O, and S;
   C is selected from the group consisting of the following groups: 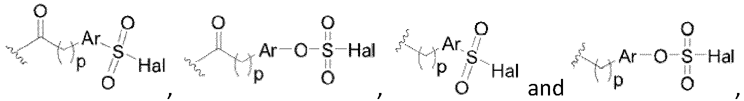 wherein:
      Ar is phenyl;
      Hal is F;
      p is an integer between 0 and 3;
      T as shown is a targeting group targeting chemokine receptor 4 (CXCR4); and
      P as shown is a chelating group capable of chelating a radionuclide and is selected from the following groups: 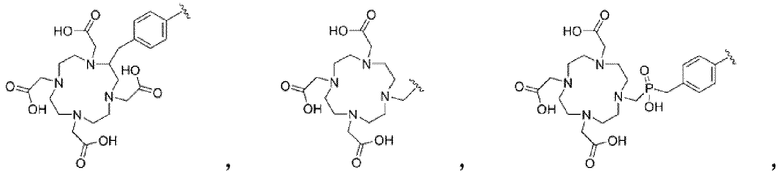 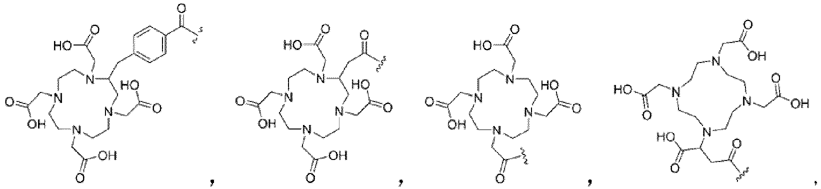 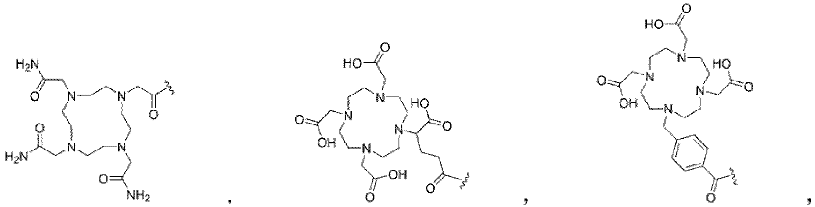 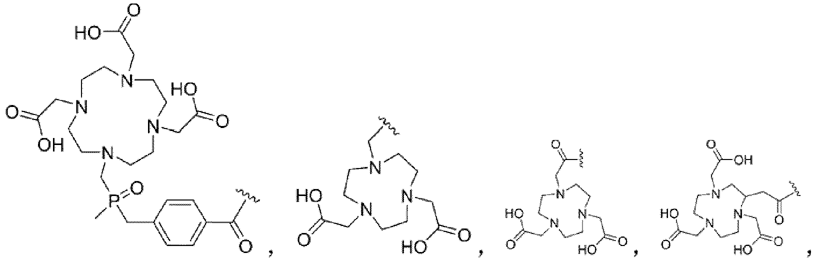 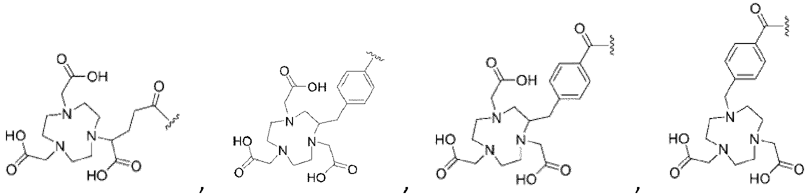 and

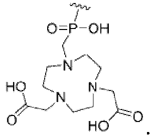

In preferred embodiments, the trifunctional compound has a structure of general formula (II),
wherein:
C and P have the meanings as defined previously,
c and d are both 1,
L₄ and L₅ are independently the following groups:
a single bond, 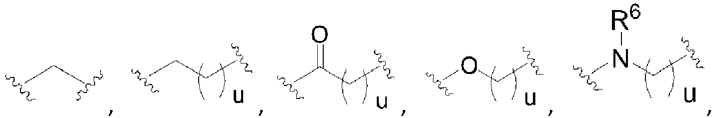 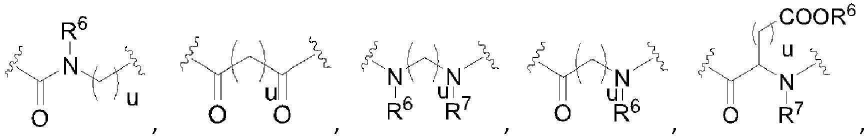 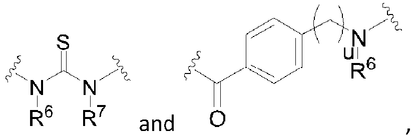
   wherein u and o are independently 1, 2, 3, 4, or 5,
   R⁶ and R⁷ are independently of each other selected from hydrogen and optionally substituted C₁-C₄,
   provided that in the linkers formed by L₁, L₂, and L₃, heteroatoms are not directly connected by a covalent bond, and the heteroatoms are selected from N, O, and S;
   T has general formula (X), 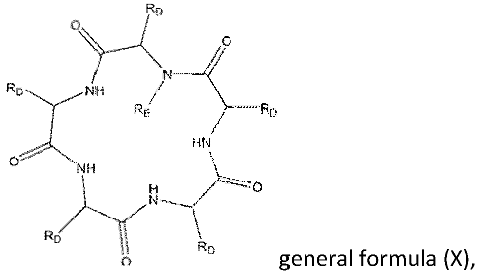
      wherein each R_{D} is independently selected from H, -CH₂R_{D'}, -CH₂COOH, -CH₂CH₂CH₂NH₂,-CH₂CH₂CH₂CH₂NH₂, and 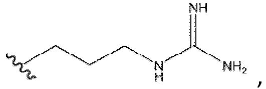 with R_{D'} being aryl, preferably
      optionally substituted phenyl or naphthyl, wherein the cyclic peptide is connected to the rest of the trifunctional compound via R_{D'} and/or -NH₂ at an end of R_{D}, or -CH₂R_{D'} or R_{D'} is capable of forming part of covalent warhead C;
      R_{E} is H or methyl;
      T preferably has the following formula 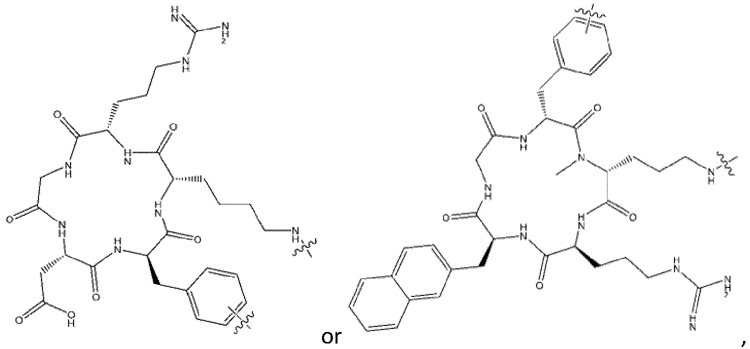
      wherein two wavy lines in each formula represent optional sites of attachment.

In some embodiments, the trifunctional compound has a structure of general formula (II): 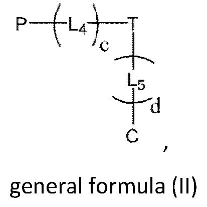 wherein:
L₄ and L₅ as shown are each independently a divalent linker unit;
c and d are independently integers of 0 to 6;
C as shown is a covalent warhead selected from the group consisting of the following groups: 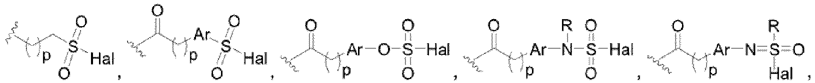 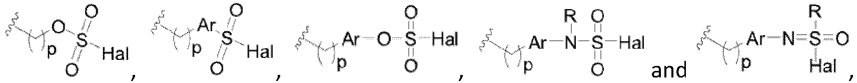 wherein:
   Ar is optionally substituted C₆-C₁₀ aryl, preferably optionally substituted phenyl, or optionally substituted C₅-C₁₂ heteroaryl;
   Hal is F or Cl;
   p is an integer between 0 and 6;
   R is selected from the group consisting of hydrogen, optionally substituted alkyl, optionally substituted cycloalkyl, optionally substituted heterocycloalkyl, optionally substituted aryl, and optionally substituted heteroaryl;
   T is a targeting group targeting somatostatin receptor (SSTR) such as SSTR2; and
   P as shown is a chelating group capable of chelating a radionuclide.

In preferred embodiments, the trifunctional compound has a structure of general formula (II),
wherein:
L₄ and L₅ are each independently selected from the following groups:
a single bond, 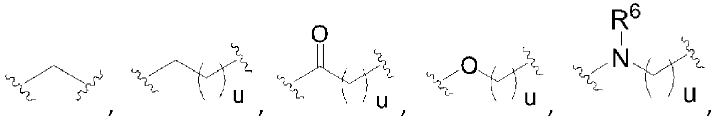 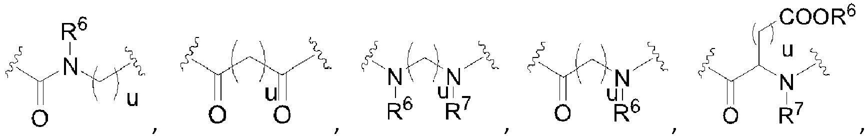 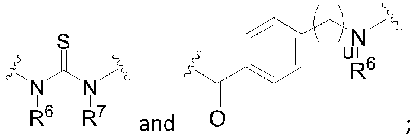
   c and d are independently 0, 1, 2, or 3,
   u is independently 1, 2, 3, 4, or 5,
   R⁶ and R⁷ are independently of each other selected from the group consisting of hydrogen, optionally substituted alkyl, optionally substituted cycloalkyl, optionally substituted heterocycloalkyl, optionally substituted aryl, and optionally substituted heteroaryl,
   provided that in the linker formed by L₄ and L₅, heteroatoms are not directly connected by a covalent bond, and the heteroatoms are selected from N, O, and S;
   C is selected from the group consisting of the following groups: 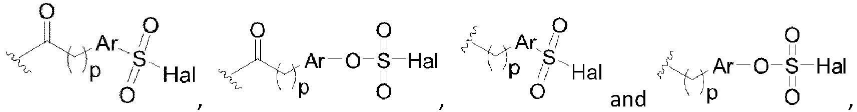 wherein:
      Ar is phenyl;
      Hal is F;
      p is an integer between 0 and 3;
      T is a targeting group targeting somatostatin receptor (SSTR) such as SSTR2; and
      P as shown is a chelating group capable of chelating a radionuclide and is selected from the following groups: 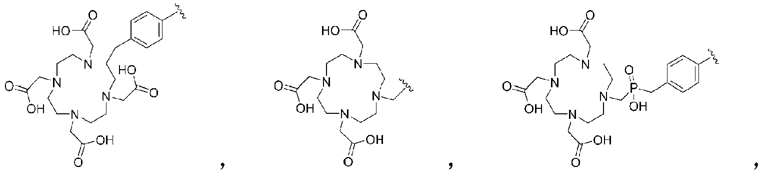 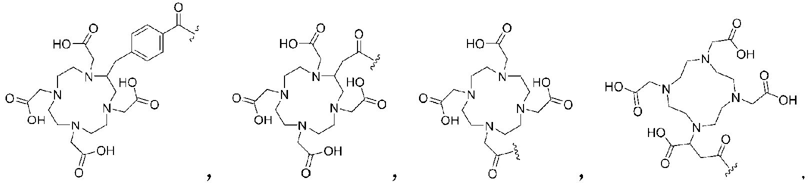 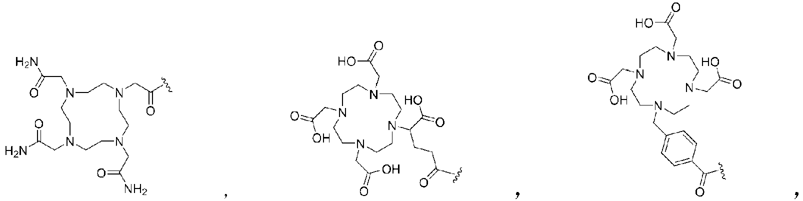 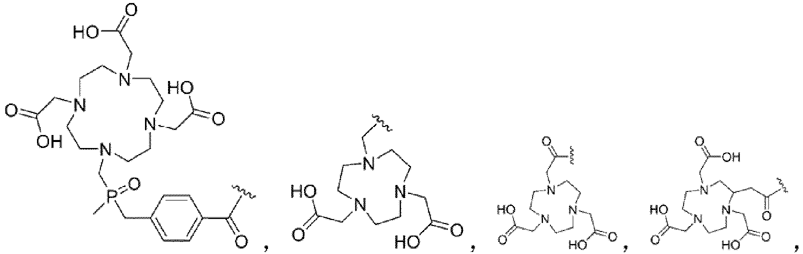 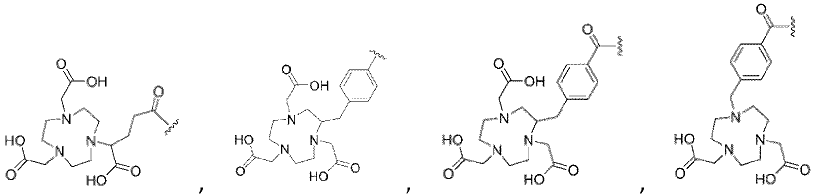 and

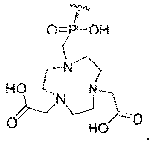

In preferred embodiments, the trifunctional compound has a structure of general formula (II),
wherein:
C and P have the meanings as defined previously,
c and d are both 1,
L₄ and L₅ are independently the following groups:
a single bond, 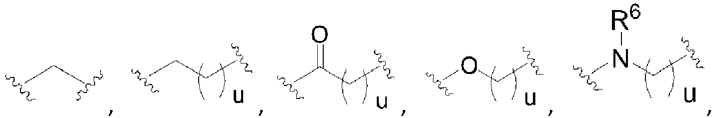 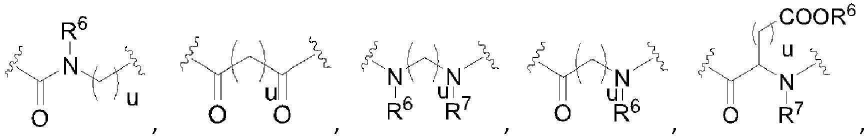 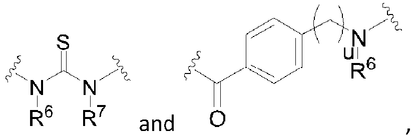
   wherein u and o are independently 1, 2, 3, 4, or 5,
   R⁶ and R⁷ are independently of each other selected from hydrogen and optionally substituted C₁-C₄,
   provided that in the linkers formed by L₁, L₂, and L₃, heteroatoms are not directly connected by a covalent bond, and the heteroatoms are selected from O, N, and S; T has general formula (XII), 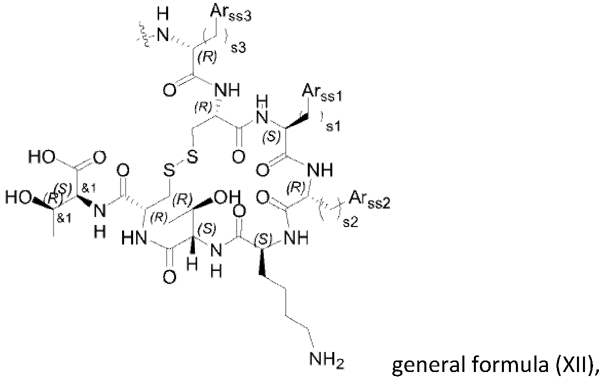
      wherein Arₛₛ₁, Arₛₛ₂, and Arₛₛ₃ are each independently selected from optionally substituted aryl or heteroaryl, preferably optionally substituted C₆-C₁₀ aryl or C₅-C₁₀ heteroaryl,
      preferably Arₛₛ₁ and Arₛₛ₃ are C₆-C₁₀ aryl optionally substituted with -OH, and Arₛₛ₂ is C₅-C₁₀ heteroaryl,
      s1, s2, and s3 are each independently selected from 1, 2, 3, or 4,
      wherein T is connected to the rest of the trifunctional compound via a site shown by the wavy line, or -(CH₂)ₛ₃Arₛₛ₃ or Arₛₛ₃ is capable of forming part of covalent warhead C,
      T preferably has the following structure

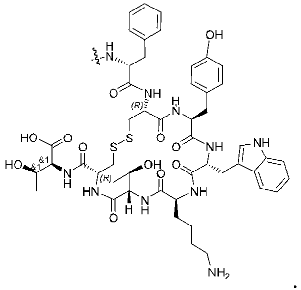

In most preferred embodiments, the trifunctional compound has one of the following structures:

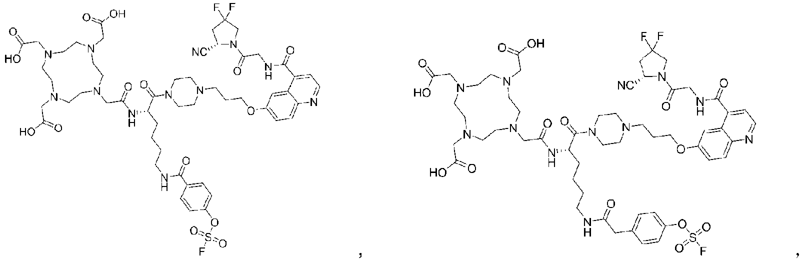

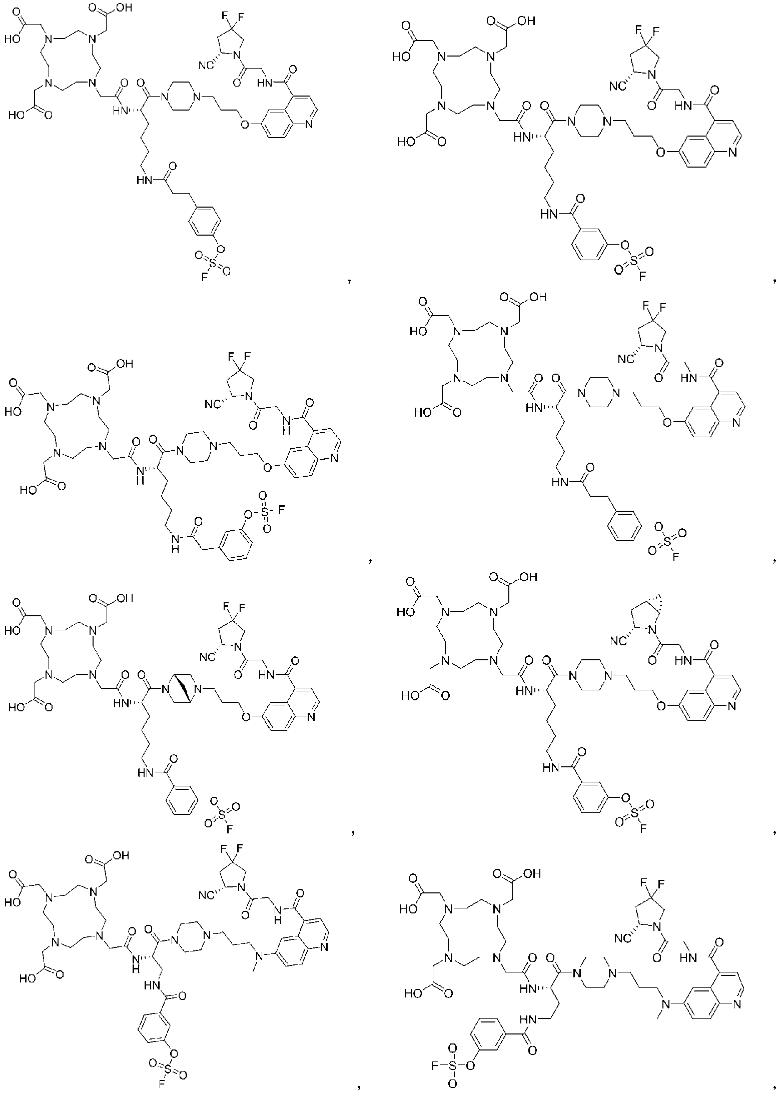

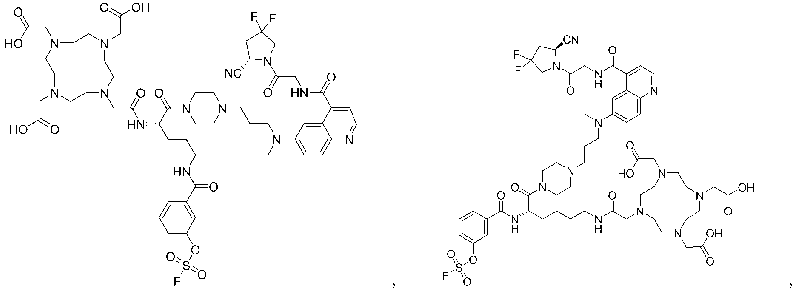

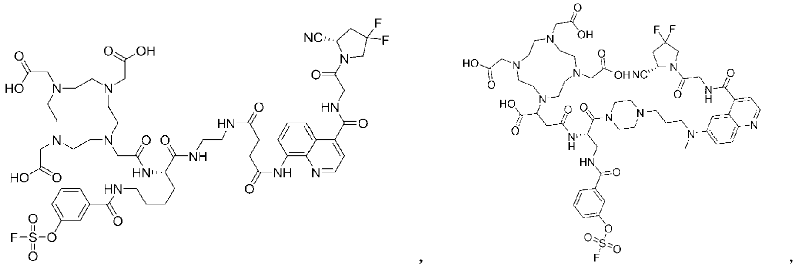

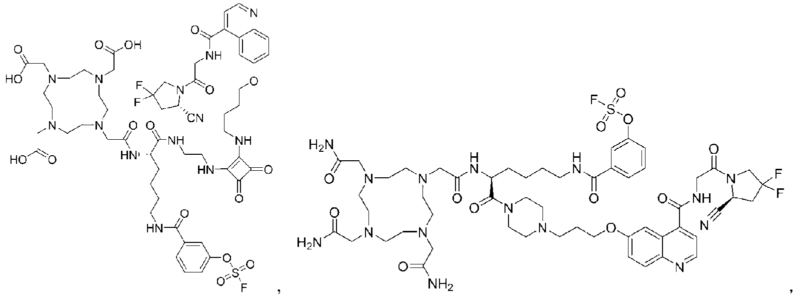

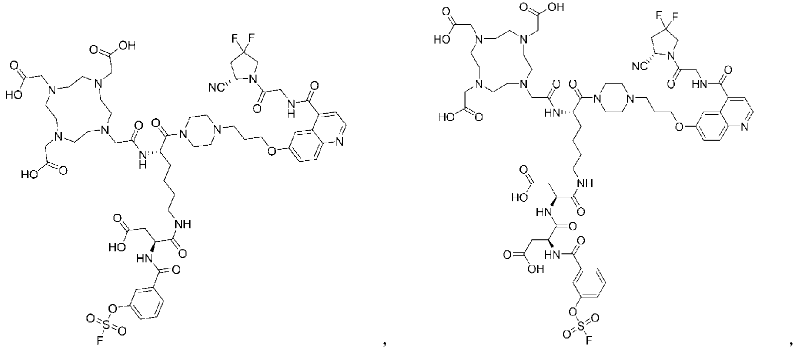

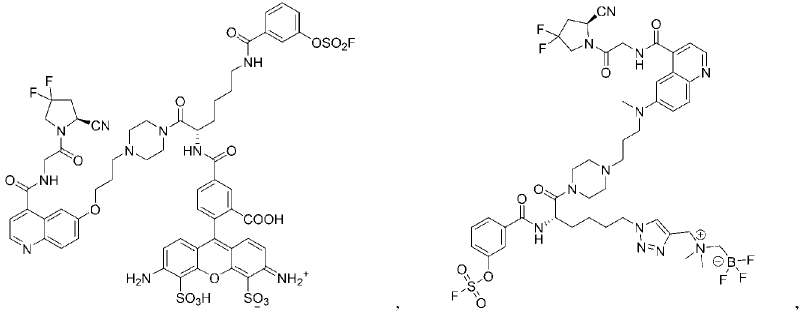

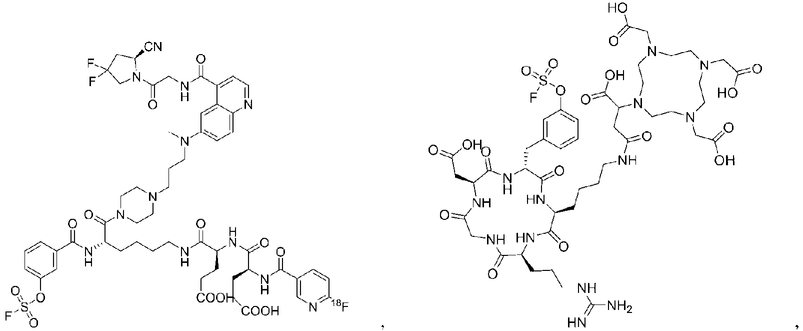

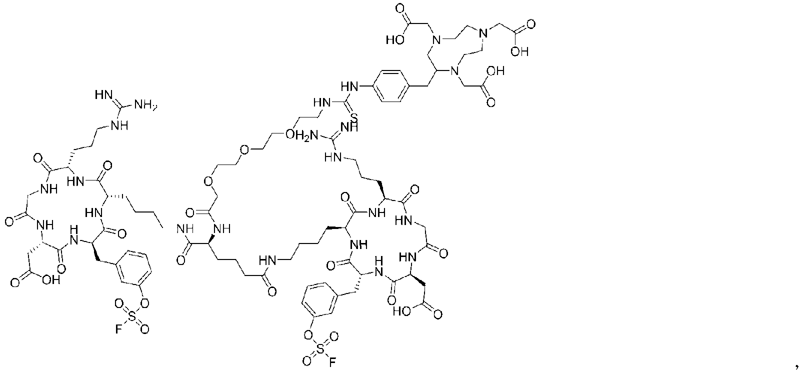

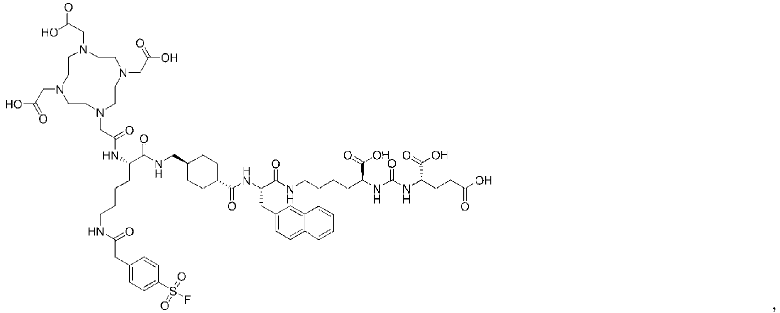

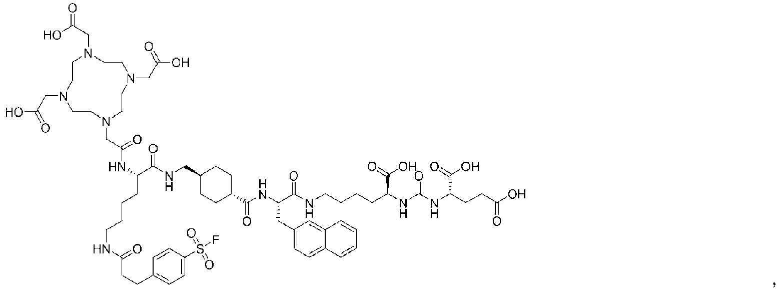

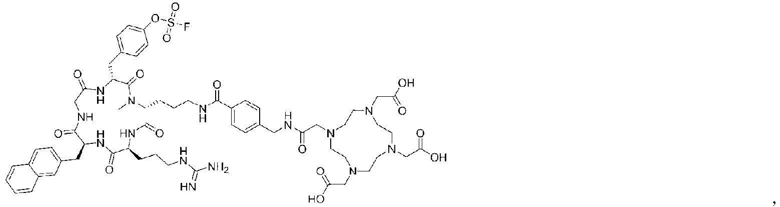

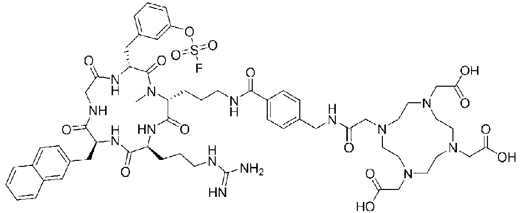

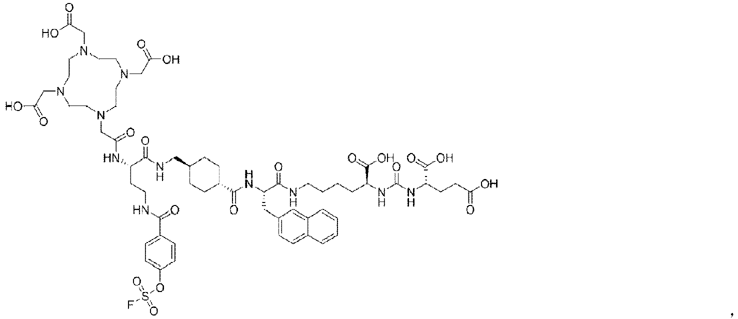

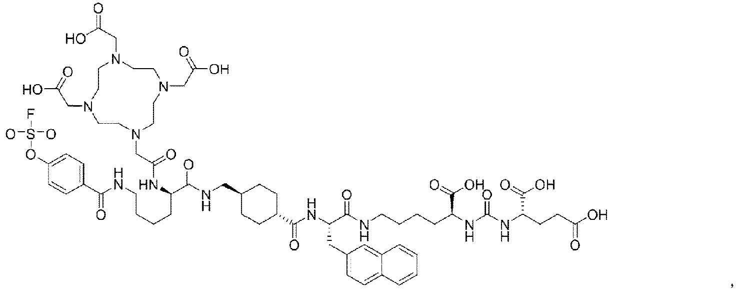

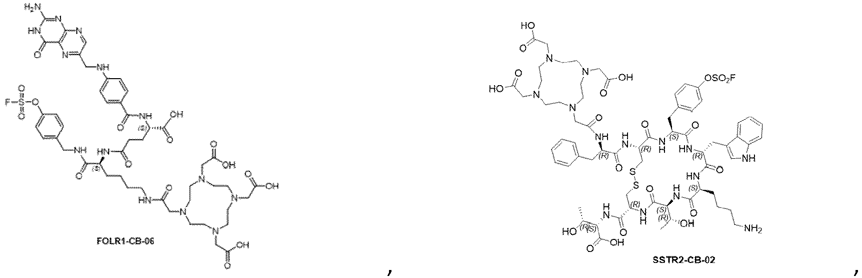

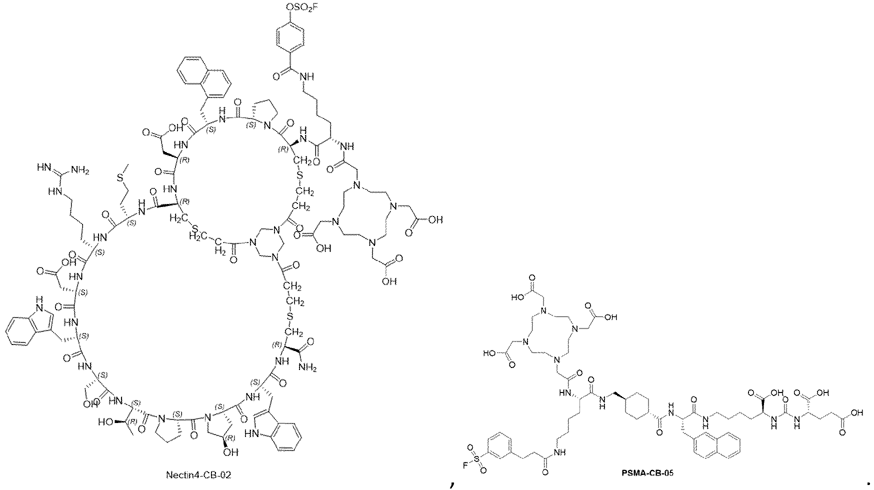

In some other preferred embodiments, the payload is a chelating group capable of chelating a radionuclide and the payload chelates a radionuclide.

In some embodiments, the radionuclide carried or chelated by the trifunctional compound is a positron nuclide, a β emitter, an α emitter, an isotope that emits Auger electrons, an isotope that emits X-rays, an isotope that emits fluorescence, or a stable metal/nonmetal element coordinated with a radionuclide, preferably ¹¹C, ¹³N, ¹⁵O, ¹⁸F and coordination metals thereof, ⁴⁷Sc, ⁵¹Cr, ⁶⁷Ga, ⁶⁸Ga,⁸⁶Y, ⁹⁰Y, ⁶⁴Cu, ⁶⁷Cu, ⁷²As, ⁷²Se, ⁸⁹Zr, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁴²Pr, ¹⁵¹Eu, ¹⁵³Eu, ¹⁶⁹Eu, ¹⁵⁹Gd, ¹⁶¹Tb, ¹⁷⁷Lu, ¹⁹⁸Au, ¹⁹⁹Ag, ²⁰¹Tl, ²¹¹At, ²⁰³Pb, ²¹²Pb, ²¹²Bi, ²¹³Bi, ²²⁵Ac,^{99m}Tc, ¹¹¹In, ¹⁴⁹Pm, ¹⁵³Sm, ¹⁶⁵Dy, ¹⁶⁹Er, ¹⁸⁶Re, ¹⁸⁸Re, ¹⁹⁷Hg, ²²⁷Th, ⁶⁷Ga, ⁶⁸Ga, ⁸⁶Y, ⁹⁰Y, ⁵⁵Co, ¹³⁹La, ¹⁴⁰La, ¹⁴⁹Tb, ¹⁵²Tb, ¹⁵⁵Tb, ¹⁶⁶Ho, ¹⁷⁵Yb, ²²⁶Th, ²²³Ra, and ²³⁰U.

In some embodiments, the present disclosure further relates to a pharmaceutical composition comprising the trifunctional compound or the pharmaceutically acceptable salt, stereoisomer or solvate, and a pharmaceutically acceptable carrier.

In some embodiments, the present disclosure further relates to a kit comprising or consisting of the trifunctional compound or the pharmaceutically acceptable salt, stereoisomer or solvate thereof as described above or the pharmaceutical composition as described above, and instructions for diagnosing a disease.

In some embodiments, the present disclosure further relates to a method for diagnosing or treating a disease, the method comprising administering to a subject a therapeutically effective amount of the trifunctional compound or the pharmaceutically acceptable salt, stereoisomer or solvate thereof as described previously, wherein the disease is preferably a central nervous system disease; a metabolic disease, preferably a cardiovascular metabolic disease; or cancer.

In preferred embodiments, the cancer is selected from prostate cancer, breast cancer, pancreatic cancer, liver cancer, lung cancer, gastric cancer, renal cancer, ovarian cancer, bladder cancer, esophageal cancer, head and neck cancer, thymic cancer, cervical cancer, endometrial cancer, neuroendocrine tumor, thyroid cancer, intestinal cancer, glioma, and bone metastasis cancer.

### Brief Description of the Drawings

Fig. 1 shows the general mechanism of action of a covalent inhibitor.
Fig. 2 shows a radioactive purity spectrum of ⁶⁸Ga-FAPI-CB-02.
Fig. 3 shows the efficiency of the covalent linkage of ¹⁷⁷Lu-FAPI-CB-01 to FAP target protein, obtained after protein co-incubation - gel electrophoresis - autoradiography-Coomassie blue staining. From left to right, the incubation times for the lanes are 1, 3, 5, 7, 9, 11, and 30 min, respectively.
Fig. 4 shows the efficiency of the covalent linkage of ¹⁷⁷Lu-PSMA-CB-05 to recombinant human PSMA protein.
Fig. 5 shows the efficiency of the covalent linkage of ¹⁷⁷Lu-FLOR-CB-06 to recombinant human FOLR1 protein.
Fig. 6 shows molecular affinity and binding kinetics graphs measured by surface plasmon resonance experiments.
Fig. 7 shows the kinetic parameters of FAPI-04 control, hydro-FAPI-CB-02 control (easily obtained by hydrolysis of FAPI-CB-02, see Fig. 12a for the structure), FAPI-CB-01, and FAPI-CB-02 as measured in a single-cycle mode.
Fig. 8 shows a graph of some sites of modification on recombinant human FAP protein with ^{nat}Lu-FAPI-CB-01.
Fig. 9 shows an IC₅₀ graph of FAPI-CB-01 on HT-1080-FAP cells.
Fig. 10 shows PET/CT contrast imaging of ⁶⁸Ga-FAPI-CB-00 and ⁶⁸Ga-FAPI-04 in an HT-1080-FAP mouse model.
Fig. 11 shows PET/CT contrast imaging of ⁶⁸Ga-FAPl-CB-01 and ⁶⁸Ga-FAPI-04 in an HT-1080-FAP mouse model.
Fig. 12 shows PET/CT contrast imaging of ⁶⁸Ga-FAPI-CB-02 and ⁶⁸Ga-FAPI-04 in an HT-1080-FAP tumor-bearing mouse model.
Fig. 13 shows PET/CT contrast imaging of ⁸⁶Y-FAPI-CB-02 and ⁸⁶Y-FAPI-04 in an HT-1080-FAP mouse model at 9 hours.
Fig. 14 shows the advantages of the compound of the present disclosure over FAPI-04 in an HT-1080-FAP tumor mouse model and specifically shows that the integral area under the tumor uptake-time curve (AUC) of the ⁸⁶Y-labeled FAPI-CB of the present disclosure is significantly improved.
Fig. 15 shows an uptake graph of ⁶⁸Ga-FAPI-CB-02 in various organs.
Fig. 16 shows the advantages of the compound of the present disclosure over FAPI-04 in an HT-1080-FAP tumor mouse model and specifically shows that the tumor uptake value of the ⁶⁸Ga-labeled FAPI-CB of the present disclosure is significantly improved.
Fig. 17 shows the binding selectivity of precursor molecules to homologous proteins of a target.
Fig. 18 shows a ¹⁷⁷Lu radionuclide therapy experiment in a tumor mouse model.
Fig. 19 shows a ²²⁵Ac radionuclide therapy experiment in a tumor mouse model.
Fig. 20 shows the advantages of the compound of the present disclosure over PSMA-617 in a tumor mouse model with high PSMA expression and specifically shows that the tumor uptake value of the ⁶⁸Ga-labeled PSMA-CB of the present disclosure is significantly improved.
Fig. 21 shows the advantages of the compound of the present disclosure over DOTATATE in a tumor mouse model with high SSTR2 expression and specifically shows that the tumor uptake value of the ⁶⁸Ga-labeled SSTR2-CB of the present disclosure is significantly improved.
Fig. 22 shows the advantages of the compound of the present disclosure over FOLR1-control in a tumor mouse model with high FOLR1 expression and specifically shows that the tumor uptake value of the ⁶⁸Ga-labeled FOLR1-CB of the present disclosure is significantly improved.
Fig. 23 shows the advantages of the compound of the present disclosure over pentixafor in a tumor mouse model with high CXCR4 expression and specifically shows that the tumor uptake value of the ⁶⁸Ga-labeled CXCR4-CB of the present disclosure is significantly improved.
Fig. 24 shows the advantages of the compound of the present disclosure over NCT4-control in a tumor mouse model with high Nectin-4 expression and specifically shows that the tumor uptake value of the ⁶⁸Ga-labeled Nectin4-CB of the present disclosure is significantly improved.

### Detailed Description of Embodiments

In the present disclosure, a trifunctional compound is obtained by introducing a fluorosulfate as a covalent warhead into a drug. The uptake and retention of the trifunctional compound at a target site such as at a tumor are substantially enhanced, while the uptake is low at non-target organs, thus exhibiting substantially reduced toxicity. The trifunctional compound of the present disclosure also has excellent pharmacokinetic properties.

Before further describing the present disclosure, some terms used in the description, examples, and appended claims are collected in the following sections. The definitions listed herein should be read and understood by those skilled in the art according to the rest of the present disclosure. Unless otherwise defined, all technical and scientific terms used herein have the same meanings as commonly understood by those of ordinary skill in the art to which the present disclosure belongs.

### Definitions

Unless otherwise specified, where any type of range is disclosed or claimed, it is intended to separately disclose or claim all possible numerical values that the range can reasonably cover, including any subranges included therein. For example, the number of groups from 1 to 6 indicates the integers within this range, wherein 1-6 should be understood to include 1, 2, 3, 4, 5, and 6, as well as subranges of 1-5, 1-4, and 1-3.

The description of the present disclosure should be interpreted to be consistent with the laws and principles of chemical bonding. In some cases, it is possible to remove hydrogen atoms in order to accommodate substituents at given positions.

Similar words used in the present disclosure, such as "including", "containing", or "comprising", mean that the elements appearing before the words cover the elements listed after the words and equivalents thereof, but do not exclude the elements that are not listed. The terms "containing" or "including (comprising)" used herein may be open, semi-closed, and closed. In other words, the term also includes "consisting essentially of" or "consisting of".

The term "pharmaceutically acceptable" in the present application means that the compound or composition is chemically and/or toxicologically compatible with other components constituting the preparation and/or with humans or mammals in which it is used to prevent or treat a disease or condition.

In the present application, the term "subject" or "patient" includes humans and mammals.

In the context of the present application, unless specifically stated to the contrary, the term "treatment" may also include prevention.

The term "alkyl" refers to a saturated linear or branched carbon chain. Preferably, the chain comprises 1 to 10 carbon atoms, namely 1, 2, 3, 4, 5, 6, 7, 8, 9, or 10 carbon atoms, preferably 1 to 6 carbon atoms, most preferably 1 to 3 carbon atoms. The alkyl is, for example, methyl, ethyl, n-propyl, isopropyl, butyl, isobutyl, tert-butyl, pentyl, hexyl, pentyl, or octyl. The alkyl is optionally substituted.

The term "heteroalkyl" refers to a saturated linear or branched carbon chain. Preferably, the chain comprises 1 to 9 carbon atoms, namely 1, 2, 3, 4, 5, 6, 7, 8, or 9 carbon atoms, preferably 1 to 6 carbon atoms, most preferably 1 to 3 carbon atoms, and is, for example, methyl, ethyl, propyl, isopropyl, butyl, isobutyl, sec-butyl, tert-butyl, pentyl, hexyl, pentyl, or octyl, which is interrupted by the same or different heteroatoms once or more, for example, once, twice, three times, four times, or five times. Preferably, the heteroatom is selected from O, S, and N, and is, for example, -O-CH₃, -S-CH₃, -CH₂-O-CH₃, -CH₂-O-C₂H₅, -CH₂-S-CH₃, -CH₂-S-C₂H₅, -C₂H₄-O-CH₃, -C₂H₄-O-C₂H₅, -C₂H₄-S-CH₃, or - C₂H₄-S-C₂H₅. The heteroalkyl group is substituted.

Unless otherwise specified, the terms "cycloalkyl" and "heterocycloalkyl" themselves or in combination with other terms respectively mean cyclic forms of "alkyl" and "heteroalkyl", in which preferably, 3, 4, 5, 6, 7, 8, 9, or 10 atoms are formed into rings, such as cyclopropyl, cyclobutyl, cyclopentyl, cyclohexyl, cycloheptyl, and cyclooctyl. The terms "cycloalkyl" and "heterocycloalkyl" are also meant to include bicyclic, tricyclic and polycyclic forms thereof. The term "heterocycloalkyl" preferably refers to a five-membered saturated ring in which at least one ring member is an N, O, or S atom, and it optionally comprises an additional O or an additional N; a six-membered saturated ring in which at least one ring member is an N, O, or S atom, and it optionally comprises an additional O, or an additional N, or two additional N atoms; or a nine- or ten-membered saturated bicyclic ring in which at least one ring member is an N, O, or S atom, and it optionally comprises one, two, or three additional N atoms. The "cycloalkyl" and "heterocycloalkyl" are optionally substituted. Examples of cycloalkyl include cyclopropyl, cyclobutyl, cyclopentyl, cyclohexyl, 1-cyclohexenyl, 3-cyclohexenyl, cycloheptyl, spiro[3,3]heptyl, spiro[3,4]octyl, spiro[4,3]octyl, spiro[3,5]nonyl, spiro[5,3]nonyl, spiro[3,6]decyl, spiro[6,3]decyl, spiro[4,5]decyl, spiro[5,4]decyl, bicyclo[2.2.1]heptyl, bicyclo[2.2.2]octyl, adamantyl, etc. Examples of heterocycloalkyl include 1-(1,2,5,6-tetrahydropyridyl), 1-piperidinyl, 2-piperidinyl, 3-piperidinyl, 4-morpholinyl, 3-morpholinyl, 1,4-diazabicyclo[2.2.2]oct-2-yl, tetrahydrofuran-2-yl, tetrahydrofuran-3-yl, tetrahydrothiophen-2-yl, tetrahydrothiophen-3-yl, 1-piperazinyl, 2-piperazinyl, etc.

The term "aryl" preferably refers to an aromatic monocyclic ring containing 6 carbon atoms, an aromatic bicyclic ring system containing 10 carbon atoms, or an aromatic tricyclic ring system containing 14 carbon atoms. Examples are phenyl, naphthyl, or anthracenyl. The aryl is optionally substituted.

The term "heteroaryl" preferably refers to a five- or six-membered aromatic monocyclic ring in which at least one carbon atom is replaced by 1, 2, 3, or 4 (for five-membered rings), or 1, 2, 3, 4, or 5 (for six-membered rings) identical or different heteroatoms, which are preferably selected from O, N, and S; an aromatic bicyclic system in which 1, 2, 3, 4, 5, or 6 carbon atoms of 8, 9, 10, 11, or 12 carbon atoms are replaced by the same or different heteroatoms, which are preferably selected from O, N, and S; or an aromatic tricyclic system in which 1, 2, 3, 4, 5, or 6 carbon atoms of 13, 14, 15, or 16 carbon atoms are replaced by the same or different heteroatoms, which are preferably selected from O, N, and S. Examples are oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3-thiadiazolyl, 1,2,5-thiadiazolyl, pyridyl, pyrimidinyl, pyrazinyl, 1,2,3-triazinyl, 1,2,4-triazinyl, 1,3,5-triazinyl, 1-benzofuryl, 2-benzofuryl, indolyl, isoindolyl, benzothiophenyl, 2-benzothiophenyl, 1H-indazolyl, benzimidazolyl, benzoxazolyl, indolizinyl, 2,1-benzoxazolyl, benzothiazolyl, 1,2-benzisothiazolyl, 2,1-benzisothiazolyl, benzotriazolyl, quinolinyl, isoquinolinyl, quinoxalinyl, quinazolinyl, quinolinyl, 1,2,3-benzotriazinyl, or 1,2,4-benzotriazinyl.

As used herein, the term "linker" refers to any chemically suitable linker. Preferably, the linker does not break or only slowly breaks under physiological conditions.

The expression "optionally substituted" means that one, two, three, or more hydrogen atoms in a group can be substituted independently of each other by various substituents. The substituent can be selected from C₁₋₆ alkyl, heteroalkyl, cycloalkyl, heterocycloalkyl, aryl, heteroaryl, halogen, cyano, amino, nitro, -OH, and -COOH.

As used herein, "radionuclide" is a radioactive isotope of an element that emits α particles, β particles, and/or y rays. The radionuclide includes, but is not limited to, the following species: ¹⁸F, ⁵¹Cr, ⁶⁷Ga, ⁶⁸Ga, ⁸⁹Zr, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶Ho,⁸⁶Y, ⁸⁸Y, ⁹⁰Y, ¹⁴⁹Pm, ¹⁶¹Tb, ¹⁶⁵Dy, ¹⁶⁹Er, ¹⁷⁷Lu,⁴⁷Sc, ¹⁴²Pr, ¹⁵⁹Gd, ²¹²Bi, ²¹³Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁹⁷Hg, ²¹¹At, ¹⁵¹_{Eu}, ¹⁵³Eu, ¹⁶⁹Eu, ²⁰¹Tl, ²⁰³Pb, ²¹²Pb, ⁶⁴Cu, ⁶⁷Cu, ¹⁸⁸Re, ¹⁸⁶Re, ¹⁹⁸Au, ²²⁵Ac, ²²⁷Th, and ¹⁹⁹Ag.

The term "radioactive drug" used in the context of the present disclosure refers to a biologically active compound modified with a radioisotope or radionuclide.

The terms "chelator" and "chelate" are used interchangeably in the context of the present disclosure and refer to a molecule that has two or more unshared electron pairs available for metal ions, usually an organic molecule, usually a Lewis base. Metal ions usually coordinate with a chelator via two or more electron pairs. The terms "bidentate chelator", "tridentate chelator", and "tetradentate chelator" refer to chelators with two, three and four electron pairs, respectively, which are easily provided to metal ions coordinated by the chelator at the same time. Usually, an electron pair of a chelator forms a coordination bond with a single metal ion. However, in some examples, a chelator can form a coordination bond with one or more metal ions, and various modes of bonding are possible. The term "chelating group" refers to a group formed by removing one or more hydrogen atoms from a "chelator" or a "chelate".

The term "optical dye" refers to a compound that emits visible light or infrared light after being excited by electromagnetic radiation with a short and appropriate wavelength. It should be understood by those skilled in the art that each optical dye has a predetermined excitation wavelength.

The term "nuclear drug molecule" or "nuclear drug" refers to a molecule or compound carrying or chelated with a radionuclide.

The term of a structure "targeting fibroblast activation protein-a" refers to a molecular fragment derived from a fibroblast activation protein inhibitor, e.g., a molecular fragment formed by a compound disclosed in WO 2019154886 A1.

The term of a structure "targeting PSMA" refers to a fragment derived from a PSMA-targeting molecule, e.g., molecular fragments formed by compounds disclosed in WO 2015055318 A1 and Journal of Nuclear Medicine, 2015, 56, 914.

The term "pharmaceutically acceptable salt" refers to a relatively nontoxic addition salt of a compound of the present disclosure. For example, see S. M. Berge et al., "Pharmaceutical Salts", J. Pharm. Sci. 1977, 66, 1-19.

Suitable pharmaceutically acceptable salts of the compounds of the present disclosure may be, for example, acid addition salts of the compounds of the present disclosure which are sufficiently basic and carry nitrogen atoms in chains or rings, e.g., acid addition salts formed with the following inorganic acids: for example, hydrochloric acid, hydrobromic acid, hydroiodic acid, sulfuric acid, phosphoric acid, or nitric acid, or acid addition salts formed with the following organic acids: for example, formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)benzoic acid, camphoretic acid, cinnamic acid, cyclopentanepropionic acid, 3-hydroxy-2-naphthoic acid, nicotinic acid, pamoic acid, pectinic acid, persulfuric acid, 3-phenylpropionic acid, picric acid, pivalic acid, 2-hydroxyethylsulfonic acid, itaconic acid, sulfamic acid, trifluoromethanesulfonic acid, dodecylsulfuric acid, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid, or thiocyanic acid.

In addition, another suitable pharmaceutically acceptable salt of the compound of the present disclosure with sufficient acidity is an alkali metal salt such as a sodium salt or a potassium salt, an alkaline earth metal salt such as a calcium salt or a magnesium salt, an ammonium salt, or a salt formed with an organic base providing a physiologically acceptable cation, e.g., salts formed with: N-methylglucosamine, dimethylglucosamine, ethylglucosamine, lysine, dicyclohexylamine, 1,6-hexamethylenediamine, ethanolamine, glucosamine, sarcosine, silanol, trihydroxymethylaminomethane, aminopropanediol, and 1-amino-2,3,4-butanetriol. In addition, basic nitrogen-containing groups may be quaternized with the following reagents: lower alkyl halides, e.g., chlorides, bromides, and iodides of methyl, ethyl, propyl, and butyl; dialkyl sulfate, e.g., dimethyl sulfate, diethyl sulfate, dibutyl sulfate, and dipentyl sulfate; long-chain halides, e.g., chlorides, bromides, and iodides of decyl, lauryl, myristyl, and stearyl; and aralkyl halides, e.g., benzyl and phenylethyl bromides.

Those skilled in the art will also recognize that an acid addition salt of the claimed compound can be prepared by reacting the compound with an appropriate inorganic or organic acid by any of a number of known methods. Alternatively, alkali metal salts and alkaline earth metal salts of acidic compounds in the present disclosure are prepared by reacting them with appropriate bases by various known methods.

The present disclosure includes all possible salts of the disclosed compounds, which may be single salts or any mixtures of the salts at any ratio.

The term "solvate" refers to a substance formed by combining, physically binding, and/or solvating the compound of the present disclosure with solvent molecules, e.g., di-solvates, mono-solvates, or semi-solvates, wherein the ratio of solvent molecules to the compound of the present disclosure is about 2:1, about 1:1, or about 1:2. This physical binding involves ionization and covalent bonding (including hydrogen bonding) to varying extents. In some cases (for example, when one or more solvent molecules are incorporated into the crystal lattice of a crystalline solid), solvates can be separated. Therefore, a solvate comprises a solution phase and a separable solvate. The compound of the present disclosure can be in a solvated form with a pharmaceutically acceptable solvent (e.g., water, methanol, and ethanol), and the present application is intended to cover solvated and non-solvated forms of the compound of the present disclosure. A solvate is a hydrate.

The compound of the present disclosure may comprise one or more asymmetric centers, depending on the desired positions and properties of various substituents. Asymmetric carbon atoms can exist in (R) or (S) configuration, racemic mixtures can be obtained in the case of having one asymmetric center, and diastereomer mixtures can be obtained in the case of having multiple asymmetric centers. In some cases, there may also be asymmetry due to hindered rotation around a specific bond, for example, the central bond connecting two substituted aromatic rings of a specific compound.

Preferred compounds are those which can produce more desirable biological activities. Isolated, purified or partially purified isomers and stereoisomers of the compounds of the present disclosure, or racemic or diastereomeric mixtures are all included in the scope of the present disclosure. Purification and separation of such substances can be achieved by standard techniques known in the art.

The term "pharmaceutical composition" used in the present application refers to a substance and/or a combination of substances used for identifying, preventing, or treating a tissue state or disease. The pharmaceutical composition is formulated to be suitable for administration to a patient to diagnose, prevent and/or treat a disease. In addition, a pharmaceutical composition refers to a combination of an active agent and an inert or active carrier, which makes the composition suitable for therapeutic use.

"Pharmaceutically acceptable" means those approved by the regulatory agencies of the federal or state governments or listed in the US Pharmacopoeia or other recognized pharmacopoeias for animals, especially for humans.

As used herein, the term "carrier" refers to a diluent, adjuvant, excipient, or carrier administered with a therapeutic agent. This drug carrier may be a sterile liquid, e.g., saline solutions in water and oils, including those of petroleum, animal, plant, or synthetic origin, such as peanut oil, soybean oil, mineral oil, or sesame oil. When the pharmaceutical composition is administered intravenously, a salt solution is a preferred carrier. A salt solution, glucose aqueous solution and glycerol solution may also be used as liquid carriers, especially for injectable solutions. Suitable pharmaceutical excipients include starch, glucose, lactose, sucrose, gelatin, malt, rice, flour, chalk, silica gel, sodium stearate, glyceryl monostearate, talcum powder, sodium chloride, skim milk powder, glycerol, propylene, ethylene glycol, water, ethanol, etc. If necessary, the composition may further comprise a small amount of a wetting agent, an emulsifier, or a pH buffer. Examples of suitable drug carriers are described in "Remington's Pharmaceutical Sciences" by E. W. Martin.

The term "halogen" refers to fluorine, chlorine, bromine, iodine, and astatine.

The term "optional" means that this situation may or may not occur.

The term "not directly connected by a covalent bond" means that there is at least one carbon atom between the two, wherein the carbon atom may exist in the form of C, CH, CH₂, or C=O.

### Examples

### Reagents and models used

The starting materials of the examples are commercially available and/or can be prepared by various methods familiar to those skilled in the art of organic synthesis. Those skilled in the art of organic synthesis will appropriately select reaction conditions (including the solvent, reaction atmosphere, reaction temperature, experimental duration, and post-treatment) in the following synthesis methods. Those skilled in the art of organic synthesis will understand that the functional groups present in various parts of the molecule should be compatible with the proposed reagents and reactions.

All reagents and compounds for synthesis can be purchased in China (excluding Hong Kong, Macao, and Taiwan) through general commercial channels, and the suppliers include WuXi AppTec (China), Shanghai Bide Pharmatech Ltd., Sigma-Aldrich (US), Sichuan Shangfu Technology Co., Ltd., Energy Chemical, and Cytiva (US).

Nuclides: ⁶⁸GaCl₃ was derived from a ⁶⁸Ge-⁶⁸Ga generator (iThemba LABS, South Africa) leached with 0.6 M hydrochloric acid. ⁸⁶YCl₃ was produced by using a 14.6-MeV cyclotron from China Institute of Atomic Energy. ¹⁷⁷LuCl₃ was in 0.1 M hydrochloric acid and purchased from ITG (Germany).

Recombinant protein: Recombinant human protein FAP-His was purchased from Novoprotein (China).

Cell model: A human fibrosarcoma cell line (HT-1080-FAP) with high FAP expression was constructed by transfection of an FAP plasmid into a human fibrosarcoma cell line (HT-1080) by WuXi Biologics (China).

Mouse model: Nu/Nu mice (SPF grade) were purchased from Beijing Charles River Laboratories (China) and injected subcutaneously with HT-1080-FAP cells to construct a tumor model with high FAP expression.

### Instruments used

Preparation and identification of compounds; High performance liquid chromatography (Waters), high performance liquid chromatography radioactive detector (Eckert & Ziegler Group), ultra-high performance liquid chromatograph-mass spectrometer (Waters), high-resolution mass spectrometer (Orbitrap Fusion Lumos or Bruker Solarix XR), nuclear magnetic resonance instrument (Bruker 400/500/600 MHz), Amersham Typhoon imaging system (Amersham Typhoon RGB), and Cytiva surface plasmon resonance instrument (Biacore 8K).

Cell experiment: Fluorescence confocal microscope (Nikon A1R-si).

Animal experiment: Small animal PET/CT (Mediso nanoScan ^{®} PET122S).

### Example 1 Synthesis of compounds

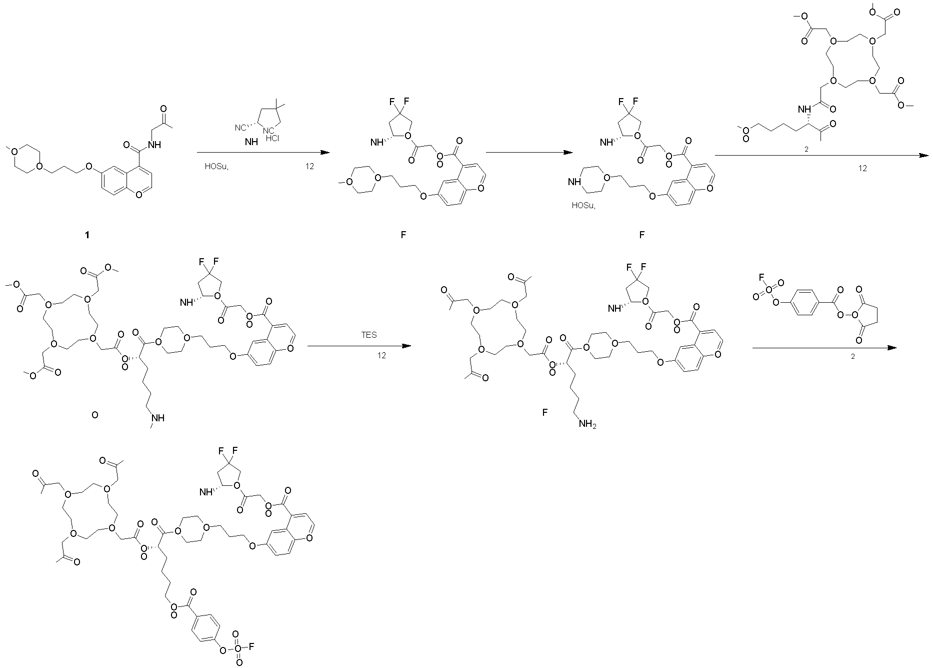

The compounds FAPI-CB-01 and FAPI-CB-02 of the present disclosure can be synthesized by the above synthesis routes; in addition, some FAP-targeting compounds in this patent application can be synthesized by similar synthesis routes.

### Example 1a. Synthesis of FAPI-CB-01

The initial raw materials, Compound 1 and Int 1, were synthesized from commercially available raw materials by WuXi AppTec (China) by reference to published routes. Compound 1 (2.40 g, 5.08 mmol) was dissolved in dichloromethane (30.0 mL), N-hydroxysuccinimide (935 mg, 8.13 mmol) and a condensing agent 1-ethyl-(3-dimethylaminopropyl)carbodiimide hydrochloride (EDCI, 1.66 g, 8.63 mmol) were added thereto, and the reaction mixture was stirred at 25°C for 1 hour. Raw material 1A (Shanghai Bide, 750 mg, 4.45 mmol) was added to the stirred solution, followed by addition of N,N-diisopropylethylamine (2.35 g, 18.2 mmol, 3.17 mL), and the reaction mixture was stirred at 25°C for 11 hours. The solvent was removed from the reaction mixture under reduced pressure, and the crude product was purified by reversed-phase high performance liquid chromatography (water/acetonitrile) to obtain Compound 2 as a red solid (1.56 g, yield 58.9%, purity 90.0%), which was confirmed by liquid mass spectrometry (LCMS) and ¹H NMR: LCMS ([M+H]⁺ = 587.3, Rt = 0.73 min); ¹H NMR 400 MHz, DMSO-d6, *δ* ppm:9.10-9.07 (m, 1H), 8.80 (d, J = 4.4 Hz, 1H), 7.97 (d, J = 9.6 Hz, 1H), 7.87-7.86 (m, 1H), 7.50 (d, J = 4.0 Hz, 1H), 7.46-7.44 (m, 1H), 5.15-5.12 (m, 1H), 4.29-4.10 (m, 6H), 2.96-2.91 (m, 3h), 2.87 (s, 1H), 2.42-2.37 (m, 4H), 1.99-1.95 (m, 2H), 1.39 (s, 9H).

Compound 2 (350 mg, 596 µmol) was dissolved in acetonitrile (2.00 mL), p-toluenesulfonic acid (328 mg, 1.91 mmol) was added, and the reaction was stirred at 30°C for 48 hours. The solvent was removed from the reaction mixture under reduced pressure to obtain Compound 3 as a red solid (380 mg, yield 96.7%, purity 90.0%, a p-toluenesulfonate), which was confirmed by LCMS: LCMS ([M+H]⁺ = 487.2, Rt = 0.50 min).

Int 1 (purchased from WuXi AppTec, 400 mg, 499 µmol) and Compound 3 (390 mg, 592 µmol, a p-toluenesulfonate) were dissolved in dimethylformamide (2.00 mL), and condensing agents 2-(7-azabenzotriazole)-N,N,N',N'-tetramethyluronium hexafluorophosphate (HATU, 265 mg, 699 µmol) and N,N-diisopropylethylamine (258 mg, 2.00 mmol, 347 µL) were added and stirred at 25°C for 12 hours. After the reaction mixture was filtered, the filtrate was taken and purified by reversed-phase HPLC (water/acetonitrile) and freeze-dried to obtain Compound 4 as a light yellow solid (270 mg, yield 42.5%), which was confirmed by LCMS: LCMS ([M+H]⁺ = 1269.8, Rt = 0.80 min).

Compound 4 (270 mg, 212 µmol) was dissolved in dichloromethane (2.00 mL), and trifluoroacetic acid (6.6 g, 58.3 mmol, 4.32 mL) and triethylsilane (247 mg, 2.13 mmol, 339 µL) were added. The solvent was removed from the reaction under reduced pressure, and the residue as a crude product was purified by reversed-phase HPLC (water/acetonitrile) to obtain Compound 5 as a light yellow solid (122.3 mg, yield 50.8%, purity 95.38%, a trifluoroacetate), which was confirmed by LCMS, HPLC, ¹H HNMR: LCMS ([M+H]⁺= 1001.3, [(M+H+H)/2]⁺ = 501.9, Rt = 1.087 min); HPLC: Separation column Gemini C18 5 µm 110A 150 * 4.6 mm, mobile phase A: deionized water containing 0.1% trifluoroacetic acid, mobile phase B: acetonitrile containing 0.075% trifluoroacetic acid, flow rate 1 mL/min, Rt = 10.889 min, purity 95.38%; ¹H NMR 400 MHz, DMSO-d6, δ ppm:9.13 (br s, 1H), 8.80 (d, *J* = 4.0 Hz, 1H), 8.28 (s, 1H), 8.21 (s, 1H), 7.98 (d, *J* = 9.2 Hz, 1H), 7.87 (d, J = 2.0 Hz, 1H), 7.51 (d, *J* = 4.4 Hz, 1H), 7.45 (dd, *J* = 9.2, 2.0 Hz, 1H), 5.14 (d, *J =* 7.6 Hz, 1H), 4.69 (s, 1H), 4.43-4.08 (m, 6H), 4.05-2.53 (m, 32H), 2.49-2.27 (m, 6H), 2.05-1.90 (s, 2H), 1.78-1.42 (m, 4H), 1.40-1.20 (m, 2H). Compound Int 2 was obtained as described in the literature *(*J. Am. Chem. Soc. 2021, 143, 3753). Compound 5 (10.0 mg, 10 µmol) was dissolved in dimethyl sulfoxide (0.2 mL), then N,N-diisopropylethylamine (6.5 mg, 50 µmol, 8.8 µL) was added thereto, followed by addition of Int 2 (2.7 mg, 20 µmol), and a reaction was allowed to proceed at 25°C for 2 hours. After the reaction mixture was filtered, the filtrate was taken and purified by reversed-phase HPLC (water/acetonitrile) and freeze-dried to obtain the final product FAPI-CB-01 as a light yellow solid (270 mg, yield 85.5%), which was confirmed by UPLC-MS (ultra-high performance liquid chromatography-mass spectrometry): separation column Acquity UPLC BEH C18 1.7 µm, 2.1 × 50 mm; in 0-0.2 min, 10% acetonitrile (containing 0.1% trifluoroacetic acid); in 0.2-3.0 min, acetonitrile (containing 0.1% trifluoroacetic acid) with a linear gradient of 10-95%; in 3-4.5 min, acetonitrile (containing 0.1% trifluoroacetic acid) with a linear gradient of 95-10%; and in 4.5-5.0 min, 10% acetonitrile (containing 0.1% trifluoroacetic acid), flow rate 0.5 mL/min, UPLC-MS ([M+H]⁺ = 1203.8, Rt = 2.7 min, purity 98.5%).

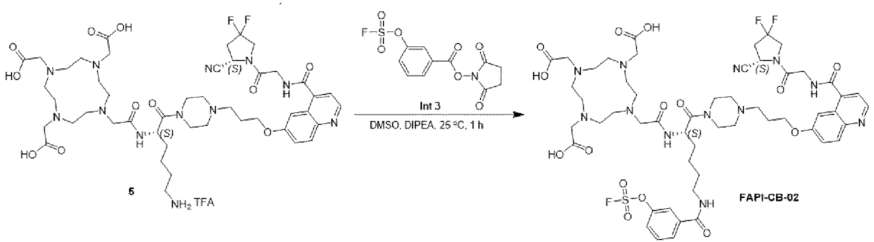

### Example 1b. Synthesis of FAPI-CB-02

Compound Int 3 was obtained according to the literature *(*J. Am. Chem. Soc. 2021, 143, 3753). Compound 5 (10.0 mg, 10 µmol) was dissolved in dimethyl sulfoxide (0.2 mL), N,N-diisopropylethylamine (6.5 mg, 50 µmol, 8.8 µL) was added, followed by Int 3 (2.7 mg, 20 µmol), and the mixture was reacted at 25°C for 2 hours. After the reaction mixture was filtered, the filtrate was taken and purified by reversed-phase HPLC (water/acetonitrile) and freeze-dried to obtain the final product FAPI-CB-02 as a light yellow solid (278 mg, yield 88.0%), which was confirmed by UPLC-MS (ultra-high performance liquid chromatography-mass spectrometry, Waters): separation column Acquity UPLC BEH C18 1.7 µm, 2.1 × 50 mm; in 0-0.2 min, 10% acetonitrile (containing 0.1% trifluoroacetic acid); in 0.2-3.0 min, acetonitrile (containing 0.1% trifluoroacetic acid) with a linear gradient of 10-95%; in 3-4.5 min, acetonitrile (containing 0.1% trifluoroacetic acid) with a linear gradient of 95-10%; and in 4.5-5.0 min, 10% acetonitrile (containing 0.1% trifluoroacetic acid), flow rate 0.5 mL/min, UPLC-MS ([M+H]⁺ = 1203.8, Rt = 2.7 min, purity 98.6%).

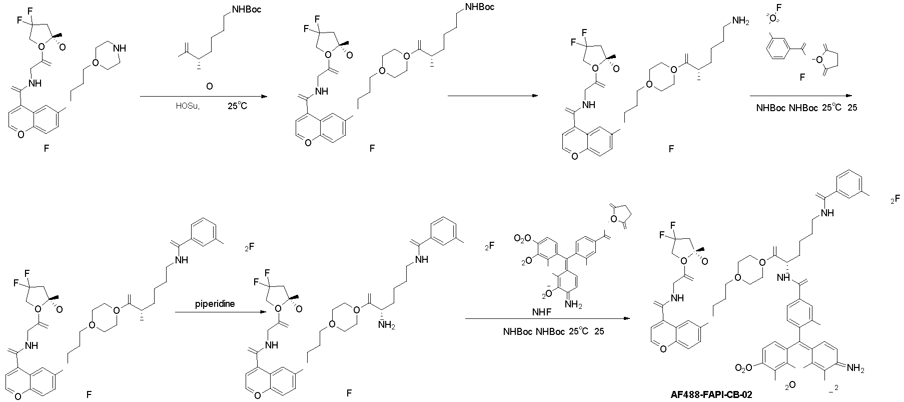

### Example 1c. Synthesis of AF488-FAPI-CB-02

The compound AF488-FAPI-CB-02 of the present disclosure can be synthesized by the above synthesis routes.

Int 4 (Shanghai Bide, 277 mg, 592 µmol) and Compound 3 (390 mg, 592 µmol, p-toluenesulfonate) were dissolved in N,N-dimethylformamide (2.00 mL), and the condensing agent 2-(7-azabenzotriazole)-N,N,N',N'-tetramethyluronium hexafluorophosphate (HATU, 265 mg, 699 µmol) and N,N-diisopropylethylamine (258 mg, 2.00 mmol, 347 µL) were added and stirred at 25°C for 12 hours and stirred at 25°C for 12 hours. After the reaction mixture was filtered, the filtrate was taken and purified by reversed-phase HPLC (water/acetonitrile) and freeze-dried to obtain Compound 6 as a light yellow solid (221 mg, yield 40%), which was confirmed by UPLC-MS (ultra-high performance liquid chromatography-mass spectrometry, Waters): separation column Acquity UPLC BEH C18 1.7 µm, 2.1 × 50 mm; in 0-0.2 min, 10% acetonitrile (containing 0.1% trifluoroacetic acid); in 0.2-3.0 min, acetonitrile (containing 0.1% trifluoroacetic acid) with a linear gradient of 10-95%; in 3-4.5 min, acetonitrile (containing 0.1% trifluoroacetic acid) with a linear gradient of 95-10%; and in 4.5-5.0 min, 10% acetonitrile (containing 0.1% trifluoroacetic acid), flow rate 0.5 mL/min, UPLC-MS ([M+H]⁺ = 937.4, Rt = 2.8 min, purity 99.2%).

Compound 6 (20 mg, 21 mmol) was dissolved in dichloromethane (200 µL) and trifluoroacetic acid (611 mg, 5.4 mmol, 400 µL) was added. The solvent was removed from the reaction under reduced pressure, and the residue as a crude product was purified by reversed-phase HPLC (water/acetonitrile) to obtain Compound 7 as a light yellow solid (12 mg, yield 67%, trifluoroacetate), which was confirmed by UPLC-MS (ultra-high performance liquid chromatography-mass spectrometry, Waters): separation column Acquity UPLC BEH C18 1.7 µm, 2.1 × 50 mm; in 0-0.2 min, 10% acetonitrile (containing 0.1% trifluoroacetic acid); in 0.2-3.0 min, acetonitrile (containing 0.1% trifluoroacetic acid) with a linear gradient of 10-95%; in 3-4.5 min, acetonitrile (containing 0.1% trifluoroacetic acid) with a linear gradient of 95-10%; and in 4.5-5.0 min, 10% acetonitrile (containing 0.1% trifluoroacetic acid), flow rate 0.5 mL/min, UPLC-MS ([M+H]⁺ = 837.4, Rt = 2.4 min, purity 98.6%).

Compound 7 (12 mg, 14.3 µmol) was dissolved in dimethyl sulfoxide (200 µL), N,N-diisopropylethylamine (9.3 mg, 71.5 µmol, 12.6 µL) was added, followed by Int 3 (6.8 mg, 21.5 µmol), and the mixture was reacted at 25°C for 1 hour. After the reaction mixture was filtered, the filtrate was taken and purified by reversed-phase HPLC (water/acetonitrile) and freeze-dried to obtain light yellow Compound 8 (6.2 mg, 6.0 µmol, yield 42%), which was confirmed by UPLC-MS (ultra-high performance liquid chromatography-mass spectrometry, Waters): separation column Acquity UPLC BEH C18 1.7 µm, 2.1 × 50 mm; in 0-0.2 min, 10% acetonitrile (containing 0.1% trifluoroacetic acid); in 0.2-3.0 min, acetonitrile (containing 0.1% trifluoroacetic acid) with a linear gradient of 10-95%; in 3-4.5 min, acetonitrile (containing 0.1% trifluoroacetic acid) with a linear gradient of 95-10%; and in 4.5-5.0 min, 10% acetonitrile (containing 0.1% trifluoroacetic acid), flow rate 0.5 mL/min, UPLC-MS ([M+H]⁺ = 1039.4, Rt = 2.7 min, purity 98.8%).

Compound 8 (6.2 mg, 6.0 µmol) was dissolved in N,N-dimethylformamide (200 µL), piperidine (34 mg, 400 µmol, 40 µL) was added, and the mixture reacted at 25°C for 1 hour. The reaction product was purified by reversed-phase HPLC (water/acetonitrile) and freeze-dried to obtain Compound 9 as a light yellow solid (3 mg, 3.7 µmol, yield 62%), which was confirmed by UPLC-MS (ultra-high performance liquid chromatography-mass spectrometry, Waters): separation column Acquity UPLC BEH C18 1.7 µm, 2.1 × 50 mm; in 0-0.2 min, 10% acetonitrile (containing 0.1% trifluoroacetic acid); in 0.2-3.0 min, acetonitrile (containing 0.1% trifluoroacetic acid) with a linear gradient of 10-95%; in 3-4.5 min, acetonitrile (containing 0.1% trifluoroacetic acid) with a linear gradient of 95-10%; and in 4.5-5.0 min, 10% acetonitrile (containing 0.1% trifluoroacetic acid), flow rate 0.5 mL/min, UPLC-MS ([M+H]⁺ = 817.3, Rt = 2.5 min, purity 98.6%).

Compound 9 (3 mg, 3.7 µmol) was dissolved in dimethyl sulfoxide (200 µL), N,N-diisopropylethylamine (2.4 mg, 18.5 µmol, 3.3 µL) was added, followed by Int 5 (2.8 mg, 4.4 µmol), and the mixture was reacted at 25°C for 1 hour. The reaction product was purified by reversed-phase HPLC (water/acetonitrile) and freeze-dried to obtain the final product AF488-FAPI-CB-02 as an orange-yellow solid (2.0 mg, yield 40%), which was confirmed by UPLC-MS (ultra-high performance liquid chromatography-mass spectrometry, Waters): separation column Acquity UPLC BEH C18 1.7 µm, 2.1 × 50 mm; in 0-0.2 min, 10% acetonitrile (containing 0.1% trifluoroacetic acid); in 0.2-3.0 min, acetonitrile (containing 0.1% trifluoroacetic acid) with a linear gradient of 10-95%; in 3-4.5 min, acetonitrile (containing 0.1% trifluoroacetic acid) with a linear gradient of 95-10%; and in 4.5-5.0 min, 10% acetonitrile (containing 0.1% trifluoroacetic acid), flow rate 0.5 mL/min, UP-LCMS ([M+H]⁺ = 1333.3, Rt = 3.2 min, purity 99.1%).

### Example 1d. Synthesis of FAPI-CB-00

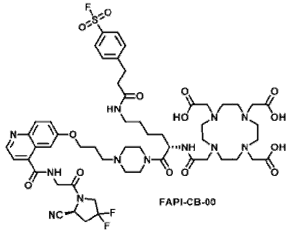

Compound FAPI-CB-00 was easily obtained by reference to the method in Example 1a, which was confirmed by UPLC-MS (ultra-high performance liquid chromatography-mass spectrometry, Waters): separation column Acquity UPLC BEH C18 1.7 µm, 2.1 × 50 mm; in 0-0.2 min, 10% acetonitrile (containing 0.1% trifluoroacetic acid); in 0.2-3.0 min, acetonitrile (containing 0.1% trifluoroacetic acid) with a linear gradient of 10-95%; in 3-4.5 min, acetonitrile (containing 0.1% trifluoroacetic acid) with a linear gradient of 95-10%; and in 4.5-5.0 min, 10% acetonitrile (containing 0.1% trifluoroacetic acid), flow rate 0.5 mL/min, UPLC-MS ([M+H]⁺ = 1214.5, Rt = 2.67 min, purity 98.7%).

### Example 1e. Synthesis of FOLR1-CB-06

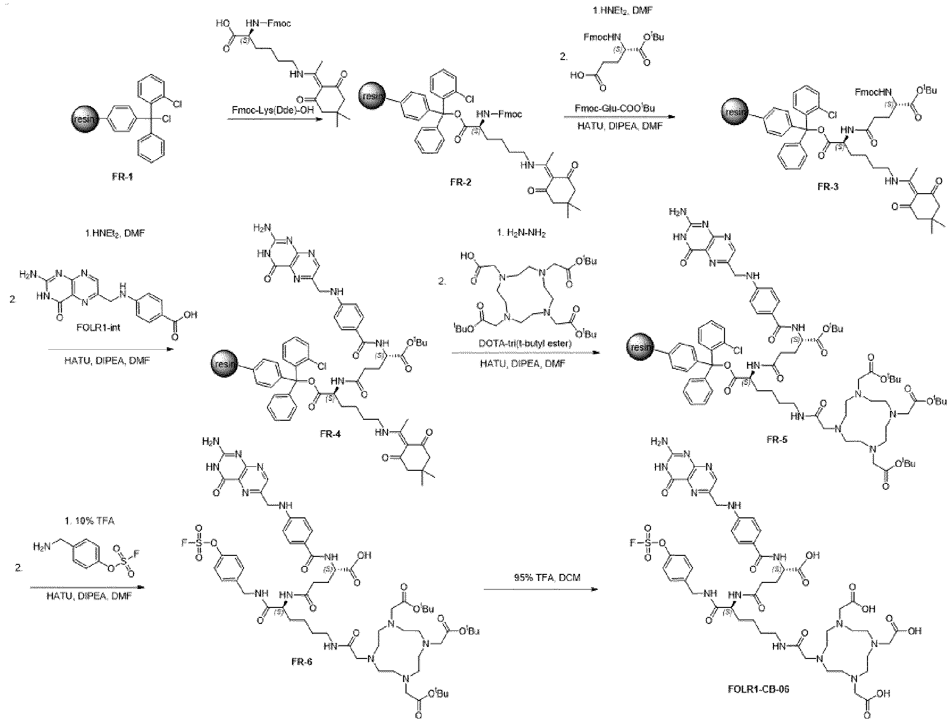

The synthesis route of Compound FOLR1-CB-06 is as shown in the above diagram. The intermediate was obtained by reference to the method in Synthesis and biological evaluation of ⁶⁸Ga-labeled Pteroyl-Lys conjugates for folate receptor-targeted tumor imaging, published by Xuran Zhang et al. The synthesis steps were as follows: 1) 1.0 g of 2-Cl-Trt resin (0.50 mmol, 1.00 eq) (loading: 0.500 mmol/g) was taken, 3 mL of toluene and 2 mL of acetyl chloride were added, and the mixture was activated at 60°C for 3 hours without stirring. The resin was transferred to a 25 mL solid-phase synthesis tube, washed 5 times with 10 mL toluene and 5 times with 10 mL of ultra-dry dichloromethane, and swollen for 1 hour by adding 20 mL of ultra-dry dichloromethane. After swelling, the swollen product was washed 5 times with 10 mL of ultra-dry dichloromethane. Subsequently, Fmoc-Lys(Dde)-OH (0.50 mmol, 266 mg, 1.00 eq) and DIEA (2.00 mmol, 0.35 mL, 4.00 eq) were added, dissolved in 8 mL of dichloromethane, and reacted on a shaker at room temperature for 12 hours. The unreacted sites were blocked by adding 1 mL of methanol. Blocking was carried out on a shaker at room temperature for 1 hour, and the resulting product was filtered and then washed 5 times with 10 mL of DMF.

2) 15 mL of a 20% piperidine/DMF solution was added, and the protecting group Fmoc was removed by a reaction on a shaker for 1 hour at room temperature. After filtration, the reaction product was washed 5 times with 10 mL of DMF. Fmoc-Glu-OtBu (1.50 mmol, 637 mg, 3.00 eq), HBTU (1.42 mmol, 540 mg, 2.85 eq), and DIEA (3.00 mmol, 0.50 mL, 6.00 eq) were added, dissolved in 8 mL of DMF, and reacted on a shaker at room temperature for 3 hours. After filtration, the reaction product was washed 5 times with 10 mL of DMF.

3) 15 mL of a 20% piperidine/DMF solution was added, and the protecting group Fmoc was removed by reaction on a shaker for 1 hour at room temperature. After filtration, the reaction product was washed 5 times with 10 mL of DMF. Pteroic acid (0.65 mmol, 200 mg, 1.30 eq), HATU (0.70 mmol, 266 mg, 1.40 eq), and DIEA (2.00 mmol, 0.37 mL, 4.00 eq) were added, dissolved in 8 mL of DMSO, and reacted on a shaker at room temperature for 3 hours. After filtration, the reaction product was washed 5 times with 10 mL of DMF.

4) 15 mL of a 3% hydrazine/DMF solution was added, and the protecting group Dye was removed by a reaction on a shaker for 1 hour at room temperature. After filtration, the reaction product was washed 5 times with 10 mL of DMF. DOTA-tri (t-butyl ester) (0.65 mmol, 372 mg, 1.30 eq), HATU (0.7 mmol, 266 mg, 1.40 eq), and DIEA (2.00 mmol, 0.37 mL, 4.00 eq) were added, dissolved in 8 mL of DMF, and reacted on a shaker at room temperature for 3 hours. After filtration, the reaction product was washed 5 times with 10 mL of DMF.

5) 15 mL of a 5% TFA/DCM solution was added, and a reaction was carried out on a shaker for 20 min at room temperature. After filtration, the filtrate was collected and subjected to rotary evaporation to obtain a yellow solid, which was separated and purified by HPLC. 4-(Aminomethyl)phenol-OSF (1.30 eq), HATU (1.40 eq), and DIEA (4.00 eq) were added and dissolved in 1 mL of DMSO, and the reaction was stirred at room temperature for 3 hours. The reaction product was separated and purified by HPLC to obtain FR-5.

6) 3 mL of a 95% TFA/DCM solution was added, and the mixture was reacted under stirring at room temperature for 2 hours. The reaction product was subjected to rotary evaporation to remove TFA and DCM. The resulting product was dissolved with DMSO and then separated and purified by HPLC to obtain FOLR1-CB-06. The product was confirmed by UPLC-MS: [M+H]⁺ = 1142.4.

### Example 1f. Synthesis of SSTR2-CB-02

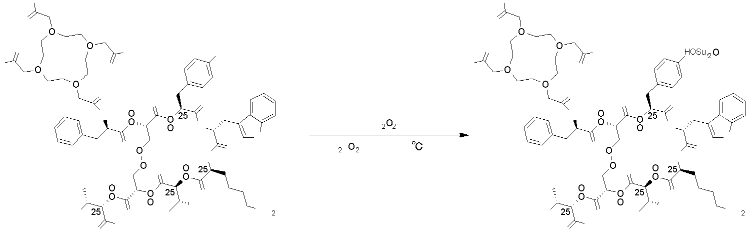

The synthesis route of Compound SSTR2-CB-02 is as shown in the above diagram. Commercially available DOTATATE (14 mg, 10 µmol) was dissolved in two phases of H₂O (0.5 mL) and DCM (0.5 mL). After triethylamine (14 µmol, 10 equiv.) was added, the atmosphere was replaced by SO₂F₂, and the reaction was stirred for about 6 hours. After DCM was removed by low-pressure rotary evaporation, the mixture was purified by reversed-phase preparative high-pressure liquid chromatography. The product was confirmed by UPLC-MS: [M+H]⁺ = 1516.55.

### Example 1g. Synthesis of Nectin4-CB-02

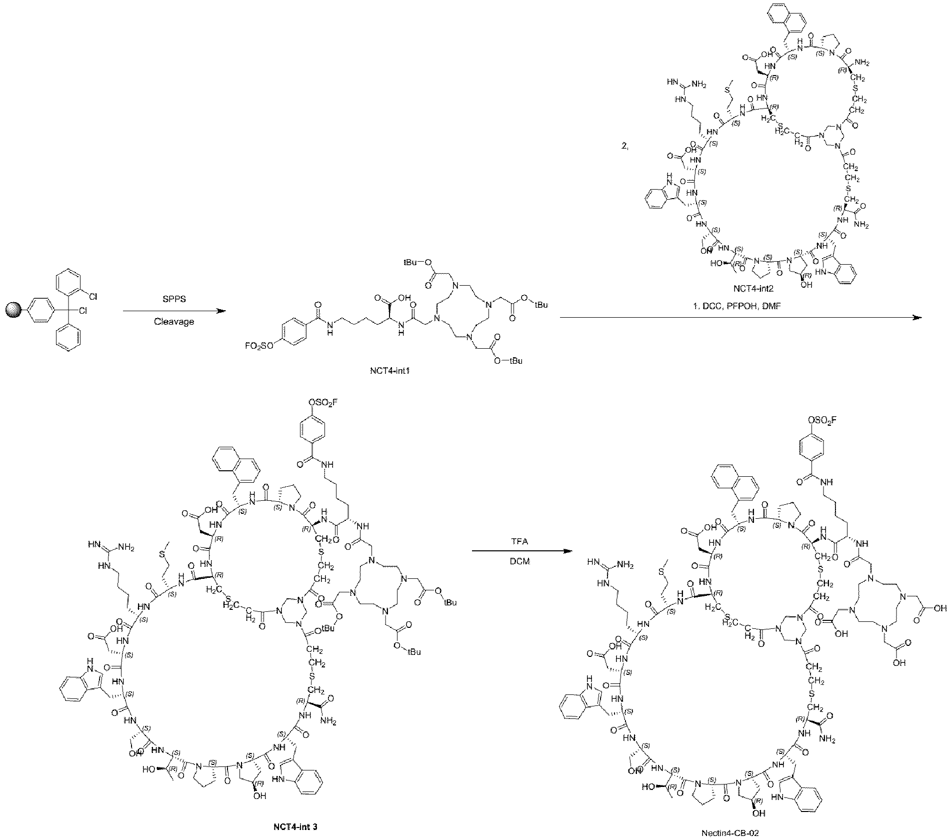

The synthesis route of Compound Nectine4-CB-02 is as shown in the above diagram. NCT4-int1 was obtained by reference to First-in-human study of the radioligand ⁶⁸Ga-N188 targeting nectin-4 for PET/CT imaging of advanced urothelial carcinoma, published by Xiaojiang Duan et al., and the solid-phase synthesis method in the above examples. Subsequently, NCT4-int2 was easily obtained by solid-phase synthesis by reference to Discovery of BT8009: A Nectin-4 Targeting Bicycle Toxin Conjugate for the Treatment of Cancer, published by Gemma E. Mudd et al. DCC (1.2 equiv.) and pentafluorophenol (1.2 equiv.) were added to a solution of NCT4-int1 (9 mg) in DMF to prepare an activated lipid. Subsequently, NCT4-int2 (22 mg) was added and the mixture was stirred and purified by reversed-phase preparative high-pressure liquid chromatography to obtain NCT4-int3. Tert-butyl was removed in 95% TFA dichloromethane to obtain Nectin-CB-02. The product was confirmed by UPLC-MS: [M+H]⁺= 2887.10.

### Example 1h. Synthesis of CXCR4-CB-03

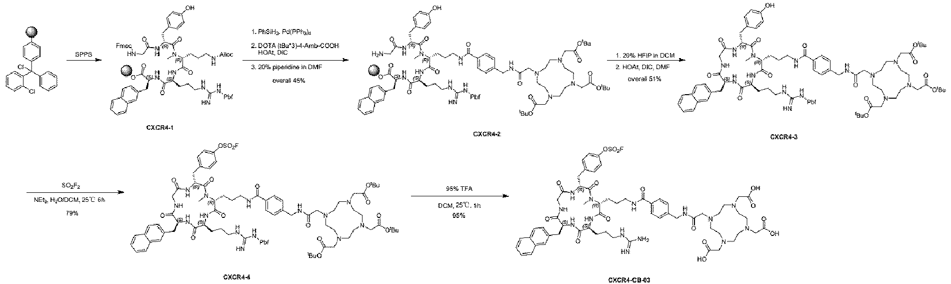

The synthesis route of Compound CXCR4-CB-03 is as shown in the above diagram. CXCR4-3 was obtained by reference to PET Imaging of CXCR4 Receptors in Cancer by a New Optimized Ligand, published by Oliver Demmer et al., and the solid-state synthesis method in the above examples. Subsequently, CXCR4-4 was obtained by a sulfonyl fluorination reaction by reference to Example 1f, and tert-butyl was removed by dichloromethane with 95% TFA. The mixture was purified by reversed-phase preparative high-pressure liquid chromatography to obtain CXCR4-CB-03. The product was confirmed by UPLC-MS: [M+H]⁺ = 1302.55.

### Example 1i. Synthesis of PSMA-CB-05

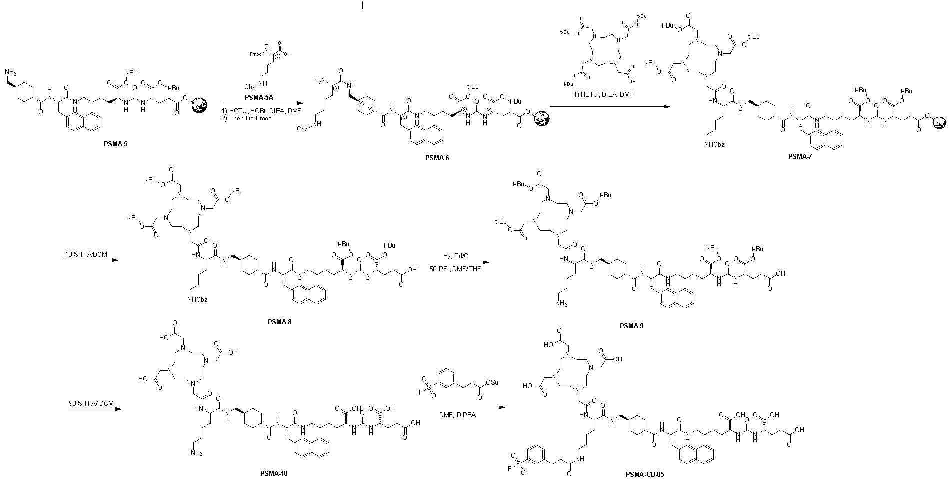

The synthesis route of Compound PSMA-CB-05 is as shown in the above diagram. PSMA-5 was easily obtained by reference to the method in Preclinical Evaluation of a Tailor-Made DOTA-Conjugated PSMA Inhibitor with Optimized Linker Moiety for Imaging and Endoradiotherapy of Prostate Cancer, published by Martina Benesova et al. Subsequently, referring to the condensation reactions in Examples 1d and 1e, PSMA-CB-05 was easily obtained from commercially available raw materials, and the product was purified by reversed-phase preparative high-pressure liquid chromatography. The product was confirmed by UPLC-MS: [M+H]⁺ = 1383.6.

### Example 2 Preparation of nuclear drug molecules by labeling precursor compound with nuclide

Radioactive labeling with ⁶⁸Ga, ⁸⁶Y, ¹⁷⁷Lu, and ²²⁵Ac was carried out by incubating the nuclides with 10-50 nmol of a precursor compound (i.e., the compound described in Example 1) for 10 min at pH = 4-4.5 at 90-100°C. The nuclide-labeled compounds were purified by solid-phase extraction with C₁₈ column (Waters), and the radiochemical purity thereof and the stability in brine and human serum were determined by high performance liquid chromatography equipped with a radioactive detector (radio-HPLC). The conditions for the radioactive high performance liquid chromatography were as follows: in 0-2 min, 10% acetonitrile (containing 0.1% trifluoroacetic acid); in 2-10 min, acetonitrile (containing 0.1% trifluoroacetic acid) with a linear gradient of 10-60%; in 10-12 min, acetonitrile (containing 0.1% trifluoroacetic acid) with a linear gradient of 60-90%; and in 12-15 min, acetonitrile (containing 0.1% trifluoroacetic acid) with a linear gradient of 90-10%, flow rate 1 mL/min, and using C₁₈ column (4.6 × 150 mm, 5 µm, XBridge, Waters) for chromatogram collection.

The radioactive purity spectrum of ⁶⁸Ga-FAPI-CB-02 is as shown in Fig. 2. The purity is 99.2%.

### Example 3 Molecular experiment - co-incubation with target protein - gel electrophoresis - autoradiography - Coomassie brilliant blue staining experiment

A total volume of 20 µL of ¹⁷⁷Lu-labeled nuclear drug molecules (such as ¹⁷⁷Lu-FAPI-CB-01, ¹⁷⁷Lu-PSMA-CB-05, or ¹⁷⁷Lu-FOLR-CB-06) with a concentration of 200 nM was incubated with 4 µM target protein for 1 hour at pH 7.4 (serum pH) or pH 6.5 (tumor microenvironment pH) at 37°C. Subsequently, the sample was subjected to treatments such as denaturation (e.g., denaturation treatment with a relatively strong acid or base), followed by the removal of reversible interactions, and SDS-PAGE was then carried out on a polyacrylamide gel to separate proteins from nuclear drug molecules. Then, the gel was taken for phosphor screen autoradiography (Amersham Typhoon imaging) to obtain radioactive bands of the nuclear drug. Finally, the gel was stained with Coomassie brilliant blue. The target protein was blue. The results of radioactive bands and Coomassie brilliant blue staining were compared. If the nuclide-labeled compound was irreversibly covalently bound to the protein, radioactive bands could be seen at the developed color of the protein.

Fig. 3 shows the efficiency of the covalent linkage of ¹⁷⁷Lu-FAPI-CB-01 to recombinant human FAP protein. In the figure, the light blue diagram on the left is a gel diagram of Coomassie Blue staining. The dark blue bands indicate protein positions (since non-covalent binding was destroyed by strong acid and alkali treatments, the protein was partially denatured, so there are three bands, with the middle band corresponding to the molecular weight position of the remaining undenatured protein). The gray-white diagram on the right is an autoradiogram. It can be seen that the above three positions corresponding to proteins all have radioactivity and gradually darken with time, whereas the bottom band represents a small molecular band of a radioactive drug and gradually lightens with time, indicating efficient covalent linkage between ¹⁷⁷Lu-FAPI-CB-01 and FAP protein.

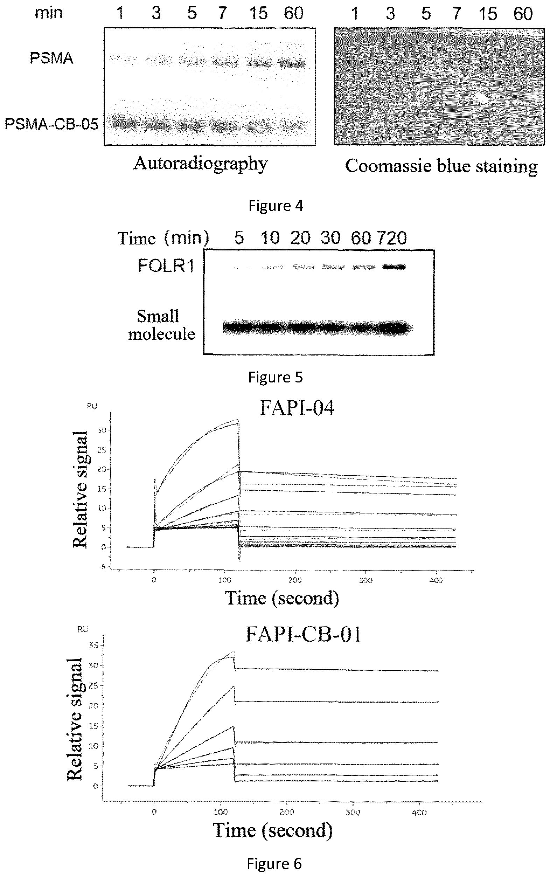

Similarly, Fig. 4 shows the efficiency of the covalent linkage of ¹⁷⁷Lu-PSMA-CB-05 to recombinant human PSMA protein.

Similarly, Fig. 5 shows the efficiency of the covalent linkage of ¹⁷⁷Lu-FLOR-CB-06 to recombinant human FOLR1 protein.

### Example 4 Molecular experiment - determination of molecular affinity and binding kinetics graph by surface plasmon resonance experiment

According to US Pharmacopeia USP43 IMMUNOLOGICAL TEST METHODS-SURFACE PLASMON RESONANCE<1105> and Chinese Pharmacopoeia 2020, Volume IV, Surface plasmon resonance method, Label-free Immunochemistry (IV), 3429 Immunochemistry, General Rule, a surface plasmon resonance experiment was carried out.

### Example 4a Measurement in multi-cycle mode

A surface plasma chromatograph from Cytiva was used for measurement. Before protein injection, 400 mM condensing agent 1-ethyl-(3-dimethylaminopropyl)carbodiimide hydrochloride and 100 mM N-hydroxysuccinimide were mixed, and a CM5 sensor chip was activated with this mixture for 420 seconds at a flow rate of 10 µL/min. 50 µg/mL protein was then injected into an Fc2 sample channel at a flow rate of 10 µL/min to achieve a protein immobilization level of about 8000-15000 RU. In the control group, no protein was injected. Subsequently, the unreacted chip was blocked with 1M ethanolamine hydrochloride-NaOH at a flow rate of 10 µL/min for 420 seconds.

Subsequently, FAPI-04 (control) or FAPI-CB-01 was diluted into multiple gradient concentrations with a buffer. Different gradient solutions were injected into channels Fc1-Fc2 at a flow rate of 30 µL/min, followed by a 60-second binding phase and a 90-second dissociation phase, and data were collected. The collected data were analyzed by a software to obtain the results shown in Fig. 6 and Table 1.

**Table 1 Analysis of surface plasma chromatography results. In the table, kₐ represents binding constant, k_{d} represents dissociation constant, K_{D} represents affinity, Rₘₐₓ represents maximum theoretical analyte binding capacity, and Chi² represents the degree of fitting. During kinetics fitting: Chi² < 1/10 Rₘₐₓ.**

| Test target protein | Test molecule | Test concentratio n gradient | kₐ **(1/Ms)** | k_{d} **(1/s)** | **K_{D} (M)** | **Rₘₐₓ (RU)** | **Chi² (RU²)** | Binding model |
|---|---|---|---|---|---|---|---|---|
| Human FAP Protein | FAPI-04 | 0.098-25 nM | 8.45E+05 | 3.11E-04 | **3.68E-10** | 21.7 | 0.54 | 1 : 1 binding |
| Human FAP Protein | FAPI-CB-01 | 0.391-12. 5nM | 1.04E+07 | 4.79E-05 | **4.62E-12** | 29.3 | 0.07 | 1 : 1 binding |

The results show that using this multi-cycle procedure, FAPI-CB-01 has a significantly increased binding rate, a decreased dissociation rate, and an affinity that was improved by approximately two orders of magnitude, as compared with the FAPI-04 molecule.

### Example 4b Measurement in single-cycle mode

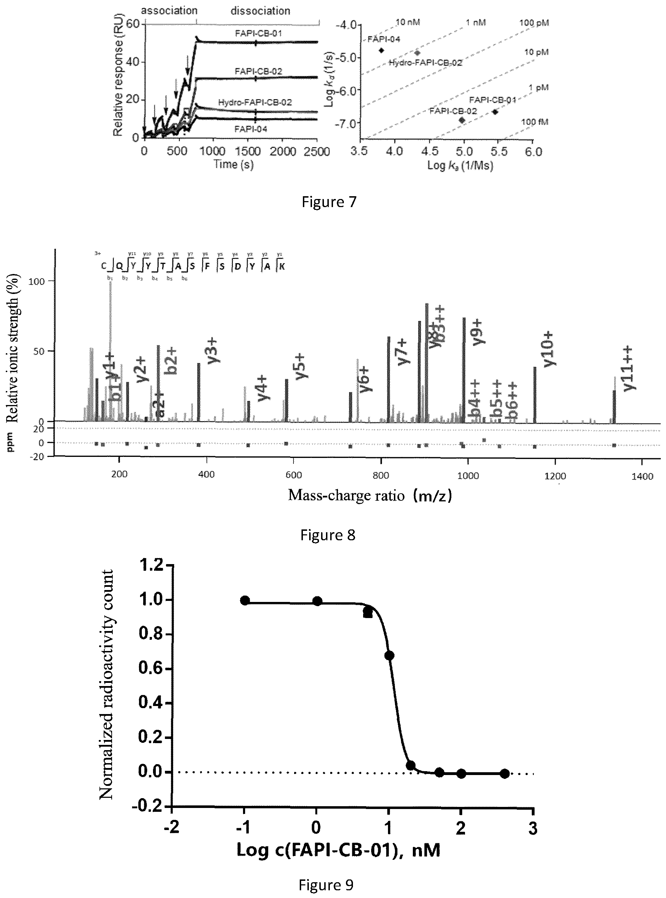

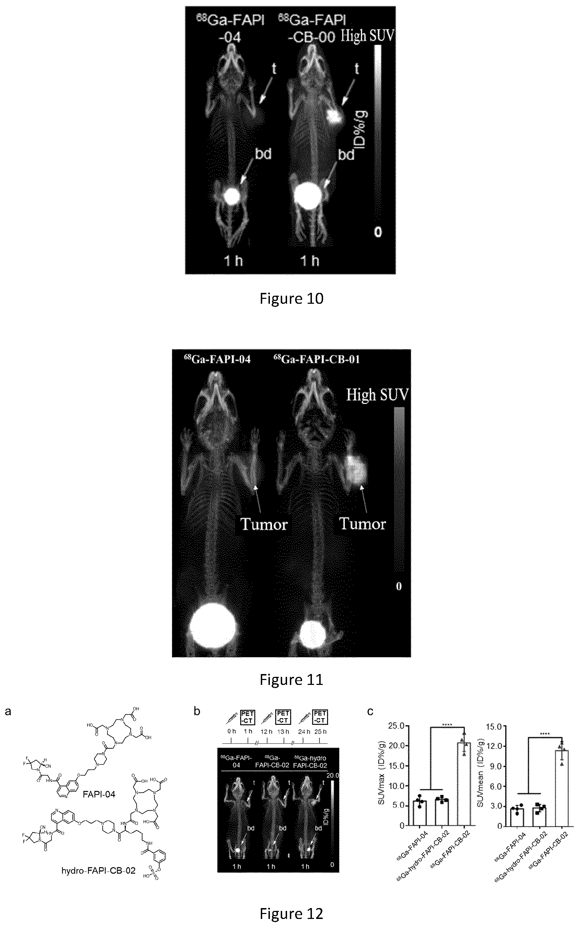

The kinetic parameters of an FAPI-04 control, a hydro-FAPI-CB-02 control (easily obtained by the hydrolysis of FAPI-CB-02, see Fig. 12a for the structure), FAPI-CB-01, and FAPI-CB-02 were measured in a single-cycle mode by reference to the method described in Compound Selectivity and Target Residence Time of Kinase Inhibitors Studied with Surface Plasmon *Resonance.* Journal of Molecular Biology 429, 574-586 (2017), published by Willemsen-Seegers, N. et al. The kinetic constant of the compound was determined by single-cycle kinetics. Depending on the affinity, the compound was injected five times continuously, and the concentrations of the compound were 0.625, 1.25, 2.5, 5, and 10 nM, respectively. In the experiment, for each concentration, the binding time is 100 seconds and the dissociation time is 1800 seconds. In order to avoid the problem of mass transport limitations, a flow rate of 30 µL/min was used. Before compound injection, a blank run was carried out under the same conditions. The SPR sensorgram was analyzed using Biacore evaluation software, wherein a double reference method was used. First, the reference channel was subtracted from the channel containing the immobilized protein. Subsequently, the reference curve obtained by buffer injection was subtracted. The obtained curve was fitted with a 1:1 binding model. Compounds that bind according to an induced fit model were fitted using a two-state reaction model. The kinetic constants (kₐ, k_{d}, and K_{D}) were obtained by a geometric mean method. The collected data were analyzed by a software to obtain the results shown in Fig. 7.

### Example 5 Modification sites

A total volume of 20 µL of a natural ^{nat}Lu-labeled precursor compound with a concentration of 40 µM was incubated with 4 µM recombinant human FAP protein for 1 hour at pH 7.4 (serum pH) at 37°C. Subsequently, the protein was separated/enzymolyzed into polypeptides to prepare a sample by a standard tandem mass spectrometry sample preparation process, and the sample was analyzed by Lumos three-in-one mass spectrometer or other mass spectrometers with HCD-FTMS fragmentation mode. In secondary mass spectrometry, mother ions collide with an inert gas, causing the peptide bonds in the peptide chain to break and form a series of daughter ions. This series of daughter ions are namely N-terminal fragment ions (series B) and C-terminal fragment ions (series Y). Depending on the molecular weight, the information about the sites of modification with the corresponding nuclear drug on the specific peptide segments could be determined.

The results are as shown in Fig. 8. As shown by the analysis by secondary mass spectrometry, ^{nat}Lu-FAPI-CB-01 has covalent linkage with tyrosine at position 450 of FAP. In the peptide segments shown in Fig. 8, Y marked red (third position) represents tyrosine at position 450.

### Example 6 Determination of IC₅₀ in cell experiment

HT-1080-FAP cells were inoculated into a 24-well plate 24 hours in advance, with the density being about 30% (calculated and estimated), 10⁵ cells (countable) per well. 0.5 mL of a complete medium was added. The medium was changed to a serum-free medium. 12 groups of FAPI-CB-01 solutions with a concentration gradient of 0-5000 nM were set up, with internal replicates n = 4 for each group. PC3-PIP cells were added to each group, and 0.75 nM ⁶⁸Ga-FAPI-04 was added to each well, followed by incubation at 37°C for 1 hour. After washing twice with cold PBS and lysis, the radioactivity count was measured.

The IC₅₀ value of FAPI-CB-01 is 11.64±0.32 nM, as shown in Fig. 9.

Using a similar method, the corresponding targeting PSMA-CB-05, FOLR1-CB-06, SSTR2-CB-02, Nectin4-CB-02, and CXCR4-CB-03 were determined for affinity in LNCaP cells, KB cells, AR42J cells, SW-780 cells, or Daudi cells, respectively. All of these compounds maintained good affinity.

### Example 7 Experiment in tumor mouse model with high FAP expression - PET/CT imaging and biological distribution

All PET/CT scans were carried out on Mediso nanoScan^{®} PET 122S small animal PET/CT.

After a certain number of HT-1080-FAP tumor mice were taken and injected via the tail vein with 7.4-18.5 MBq of ⁶⁸Ga/⁸⁶Y-FAPI-04 (control) and ⁶⁸Ga/⁸⁶Y-FAPI-CB series molecules at specified time points (Note: the injection of the control and investigation molecules was separated apart by at least five half-lives of the nuclide to ensure that the radioactivity of the previous probe had almost completely decayed and was essentially completely metabolized), imaging was carried out at specified time points. In some examples, the mice were anesthetized and then sacrificed by neck breaking at a certain time point. Organs were dissected and taken for radioactivity measurement.

### Examples 7a: PET/CT imaging of ⁶⁸Ga-FAPI-CB-00 in HT-1080-FAP tumor-bearing mouse model

A ⁶⁸Ge-⁶⁸Ga generator was eluted with 5 mL of 0.6 M high-purity hydrochloric acid to obtain a Ga-68 hydrochloric acid solution. 1 mL of the Ga-68 solution after elution was taken, and 100 µL of 3 M sodium hydroxide and 130 µL of 3M sodium acetate were added to adjust the acidity to a final pH of 4.0. 12 µg of FAPI-CB-00 compound was added, and the reaction mixture was heated to 70°C and maintained for 20 min. The reaction solution was passed through a C₁₈ cartridge to remove free ions, and the C₁₈ column was then eluted with an ethanol solution. Labeled ⁶⁸Ga-FAPI-CB-00 was obtained. The labeled ⁶⁸Ga-FAPI-CB-00 was diluted with normal saline, and each mouse was injected with 3.7 MBq via the tail vein. PET scanning imaging was carried out in 1 hour, and reconstruction was carried out with a PET image processing software. The obtained image is as shown in Fig. 10. It can be seen that the ⁶⁸Ga-FAPI-CB-00 probe has significantly higher uptake in the tumor site than ⁶⁸Ga-FAPI-04 (since the drug is primarily metabolized by the kidneys, it has a high uptake in the bladder for a short period of time and is excreted via urine), but the background also increases to some extent.

### Example 7b: PET/CT imaging of ⁶⁸Ga-FAPI-CB-01 in HT-1080-FAP tumor-bearing mouse model

A ⁶⁸Ge-⁶⁸Ga generator was eluted with 5 mL of 0.6 M high-purity hydrochloric acid to obtain a Ga-68 hydrochloric acid solution. 1 mL of the Ga-68 solution after elution was taken, and 100 µL of 3 M sodium hydroxide and 130 µL of 3M sodium acetate were added to adjust the acidity to a final pH of 4.0. 12 µg of FAPI-CB-01 compound was added, and the reaction mixture was heated to 90°C and maintained for 10 min. The reaction solution was passed through a C₁₈ cartridge to remove free ions, and the C₁₈ column was then eluted with an ethanol solution. Labeled ⁶⁸Ga-FAPI-CB-01 was obtained. The labeled ⁶⁸Ga-FAPI-CB-01 was diluted with normal saline, and each mouse was injected with 3.7 MBq via the tail vein. PET scanning imaging was carried out in 1 hour, and reconstruction was carried out with a PET image processing software. The obtained representative image is as shown in Fig. 11. It can be seen that the ⁶⁸Ga-FAPI-CB-01 probe has significantly higher uptake in the tumor site than ⁶⁸Ga-FAPI-04 (since the drug is primarily metabolized by the kidneys, it has a high uptake in the bladder for a short period of time and is excreted via urine), but the background increases to a relatively limited extent.

### Example 7c: PET/CT imaging of ⁶⁸Ga-FAPI-CB-02 in HT-1080-FAP tumor-bearing mouse model

⁶⁸Ga-FAPI-04, ⁶⁸Ga-hydro-FAPI-CB-02 (the structure is as shown in Fig. 12a), and ⁶⁸Ga-FAPI-CB-02 were labeled by the method described in Example 7b. The labeled drug was diluted with normal saline, and each mouse was injected with about 3.7 MBq of three molecules successively via the tail vein at intervals of 12 hours. PET scanning imaging was carried out in 1 hour, and reconstruction was carried out with a PET image processing software. The obtained image is as shown in Fig. 12b. It can be seen that the uptake of the ⁶⁸Ga-FAPI-CB-02 probe in the tumor site is significantly higher, whereas the control ⁶⁸Ga-FAPI-04 and ⁶⁸Ga-hydro-FAPI-CB-02 hydrolyzed by a fluorosulfate both have a lower tumor uptake (the quantitative results are as shown in Fig. 12c), thus indicating the beneficial effects brought about by introducing additional covalent warheads.

### Example 7d: PET/CT imaging of ⁸⁶Y-FAPI-CB-02 in HT-1080-FAP tumor-bearing mouse model and pharmacokinetic data

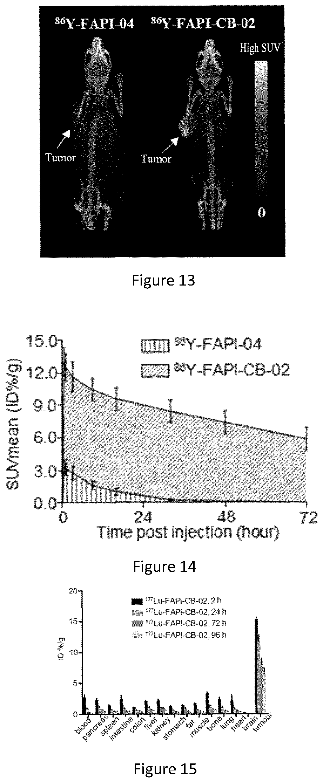

The target [⁸⁶Sr]SrCO₃ bombarded by an accelerator was eluted with 5 mL of 0.1 M high-purity hydrochloric acid to obtain a Y-86 hydrochloric acid solution. 1 mL of the Y-86 solution after elution was taken, and 90 µL of 3 M sodium acetate was added to adjust the acidity to a final pH of 4.0. 12 µg of FAPI-CB-02 precursor was added, and the reaction mixture was heated to 90°C and maintained for 10 min. The reaction solution was passed through a C18 cartridge to remove free ions, and the C18 column was then eluted with an ethanol solution. Labeled ⁸⁶Y-FAPI-CB-02 was obtained. The labeled ⁸⁶Y-FAPI-CB-02 was diluted with normal saline, and each mouse was injected with 29.6 MBq via the tail vein. PET scanning imaging was carried out at multiple time points over a long period of time, and reconstruction was carried out with a PET image processing software. The obtained contrast image representing individual mice at 9 hours is as shown in Fig. 13. It can be seen that the ⁸⁶Y-FAPI-CB-02 probe still has significantly higher uptake and retention in the tumor site at a later time point as compared with ⁸⁶Y-FAPI-04.

The tumor uptake value can be semi-quantitatively determined from the Standard Uptake Value (SUV) obtained after the analysis of a reconstructed image resulting from PET-CT scanning with the built-in software of the equipment. SUV refers to the ratio of the radioactivity of the imaging agent taken up by a local tissue to the average injection activity of the whole body: SUV = Radioactive concentration in lesion (kBq/ml) / (Injection dose (MBq) / Body weight (kg)). According to the SUV data obtained in this experiment, a pharmacokinetic curve in the tumor, namely the integral area under the tumor uptake-time curve (area under curve, AUC), was plotted. Compared with ⁸⁶Y-FAPI-04, ⁸⁶Y-FAPI-CB-02 has a significant increase of no less than 6 times in AUC (n = 4, Fig. 14) and is expected to greatly improve the therapeutic effect of the corresponding therapeutic radionuclide-labeled drug.

### Example 7e: Biological distribution of ¹⁷⁷Lu-FAPI-CB-02 HT-1080-FAP in tumor-bearing mouse model

Referring to the labeling method in the above examples, labeled ¹⁷⁷Lu-FAPI-CB-02 can be easily obtained. Nu/Nu mice (18-20 g) with HT1080-FAP xenograft tumor were taken and randomly divided into 4 groups, with 7-8 mice in each group. The mice were anesthetized in an isoflurane atmosphere (2-3%) and injected via the tail vein with 200 µL of a ⁶⁸Ga-FAPI-CB-02 preparation with an activity of 0.74 MBq. The mice were anesthetized in an isoflurane atmosphere (2-3%) and then sacrificed by neck breaking at different time points (60 min, 120 min, and 240 min) after injection. The organs of interest were dissected and taken out. The radioactivity and weight of each organ were measured by a gamma counter and a balance, and the percentage of radioactivity taken up by the organ per unit mass was calculated (denoted as %ID/g). The results are as shown in Fig. 15. The uptake of ¹⁷⁷Lu-FAPI-CB-02 in liver and kidney is relatively high (similar to that of ¹⁷⁷Lu-FAPI-04). The uptake value of the radioactive substance tends to gradually decrease with time, and the tumor uptake is relatively high, so the tumor-normal organ uptake ratio gradually increases after a long period of time.

### Example 8 Tumor uptake value

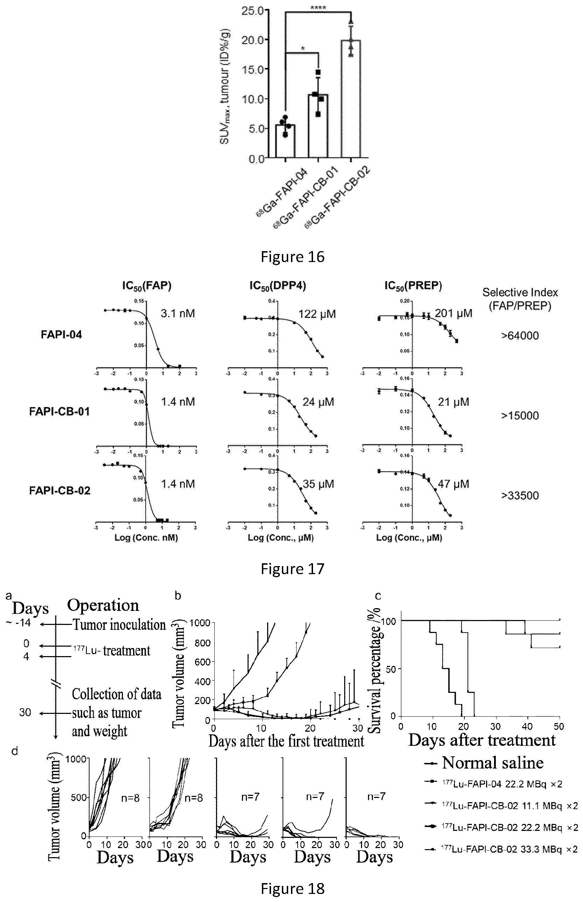

HT-1080-FAP tumor-bearing mice in the same group (4 mice in each group) were separately injected via the tail vein with 18.5 MBq of ⁶⁸Ga-FAPI-04 at time 0. Subsequently, PET-CT scanning was carried out on day 1. After ⁶⁸Ga essentially completely decayed over 24 hours, 18.5 MBq of ⁶⁸Ga-FAPI-CB-02 was injected via the tail vein. PET-CT scanning was carried out again to obtain image data. The tumor uptake value can be semi-quantitatively determined from the Standard Uptake Value (SUV) obtained after the analysis of a reconstructed image resulting from PET-CT scanning with the built-in software of the equipment. SUV refers to the ratio of the radioactivity of the imaging agent taken up by a local tissue to the average injection activity of the whole body: SUV = Radioactive concentration in lesion (kBq/ml) / (Injection dose (MBq) / Body weight (kg)). As shown in Fig. 16, compared with FAPI-04 in the most relevant prior art, in the HT-1080-FAP mouse model, the maximum standard tumor uptake values (SUVₘₐₓ) of FAPI-CB-01 and FAPI-CB-02 molecules of the present disclosure are significantly improved by about 2 times and 4 times, respectively (n = 4, p < 0.005), and they also have obvious advantage in PET-CT imaging. Therefore, the tumor uptake values of the nuclide-labeled FAPI-CB-01 and FAPI-CB-02 of the present disclosure are significantly improved, which is of great significance for improving the clinical tumor detection rate.

### Example 9 Binding selectivity of precursor molecule to homologous proteins of target

Taking FAP target as an example, homologous proteins thereof include, but are not limited to, DPP-4 and PREP. The structural similarity thereof may lead to a decreased binding selectivity of the modified precursor molecules for the homologous proteins, resulting in an increased uptake in non-target organs.

The binding selectivity for homologous proteins was determined by a competitive inhibition experiment on the substrate of the enzymes, and the binding/inhibition selectivity thereof is indirectly evaluated by the median inhibitory concentration (IC₅₀) values obtained.

By reference to the method described in Extended Structure-Activity Relationship and Pharmacokinetic Investigation of (4-Quinolinoyl)glycyl-2-cyanopyrrolidine Inhibitors of Fibroblast Activation Protein (FAP). Journal of Medicinal Chemistry 57, 3053-3074 (2014), published by Jansen et al., the initial rate of p-nitroaniline (pNA) released from the substrate (absorption at 405 nm) was measured by using a microplate reader (PerkinElmer, VictorTM X5) at 37°C in a final volume of 200 µL to determine the kinetic properties of the enzyme activity. One unit of enzyme activity is defined as the amount of the enzyme that catalyzes the substrate to release 1 µmol pNA per minute under detection conditions. All measurements were made in duplicate to ensure reproducibility. The IC₅₀ value is defined as the inhibitor concentration that leads to a 50% decrease in activity under detection conditions, and is calculated by GraphPad according to the inhibitor concentration and the absorption of pNA.

The determination method for IC₅₀ on FAP is as follows: the substrate Z-Gly-Pro-p-nitroanilide (50 µM, Cat# G-58468, HEOWNS) was used to measure FAP activity at pH 7.4. The FAP determination buffer is composed of 50 mM Tris pH = 7.4 and contains 1 M NaCl and 1.0 mg/mL bovine serum albumin. Before adding FAP protein (0.05 µg per well), the buffer, substrate, and inhibitor were mixed. The mixture was then incubated at 37°C for 6 hours, and the release of pNA was then measured. The concentration of the inhibitor varies from 0.01 nM to 200 µM. The IC₅₀ value was determined according to the above method.

Determination of IC50 on DPP-4: The substrate Z-Gly-Pro-p-nitroanilide (100 µM, Cat# G-58468, HEOWNS) was used at pH 8.3 for determining the DPP-4 activity. The DPP-4 determination buffer is composed of 50 mM Tris pH = 8.3 and contains 1 mM EDTA. Before adding DPP-4 protein (0.05 µg per well), the buffer, substrate, and inhibitor were mixed. The mixture was then incubated at 37°C for 20 min, and the release of pNA was measured. The concentration of the inhibitor varies from 0.01 nM to 200 µM. The IC50 value was determined according to the above method.

Determination of IC50 on PREP: The substrate Gly-Pro-p-nitroanilide (0.1 mM) was used at pH 7.5 for measuring the PREP activity. The PREP determination buffer comprises 0.1M K₃PO₄ pH = 8.3 and contains 1 mM EDTA and 10 mM DTT. The buffer, substrate, and inhibitor were mixed, and PREP protein (0.05 µg per well) was then added. The mixture was then incubated at 37°C for 20 min, and the release of pNA was measured. The concentration of the inhibitor ranges from 0.01 nM to 200 µM. The IC₅₀ value was determined according to the above method.

The IC₅₀ results are as shown in Fig. 17. It can be seen that FAPI-CB-01 and FAPI-CB-02 after covalent modification of FAPI-04 still maintain the original high selectivity of FAPI-04 to FAP protein.

### Example 10 ¹⁷⁷Lu radionuclide therapy experiment in tumor mouse model

When the average tumor size reached about 100 mm³, HT-1080-FAP tumor mice in the same batch were randomly divided into five groups, with n = 7 or 8 in each group. Two rounds of treatment were then carried out on day 0 and day 4. The treatment group was injected with a corresponding dose of ¹⁷⁷Lu-labeled FAPI-CB-02 or control FAPI-04 (radioactivity yield > 99%, radioactivity purity > 99%, and specific activity ≈ 11.2 MBq/nmol). The tumor size and weight of the mice were monitored every 2 or 3 days.

As shown in Fig. 18, 18a is a schematic diagram of the treatment scheme, 18b is an averaged tumor growth curve, 18c is a Kaplan-Meier curve (survival curve) 50 days after treatment, and 18d is a detailed tumor growth curve of each mouse in each treatment group. It can be seen that compared with the normal saline control group or FAPI-04 control group, using the same or a higher dose (¹⁷⁷Lu activity) of FAPI-CB-02 results in a significantly better tumor inhibition effect, and using a lower dose may also result in a better therapeutic effect.

### Example 11 ²²⁵Ac radionuclide therapy experiment in tumor mouse model

When the average tumor size reached about 100 mm³, HT-1080-FAP tumor mice in the same batch were randomly divided into five groups, with n = 7 or 8 in each group. Two rounds of treatment were then carried out on day 0 and day 4. The treatment group was injected with a corresponding dose of ²²⁵Ac-labeled FAPI-CB-02 or control FAPI-04 (radioactivity purity > 99%, and specific activity ≈ 10 kBq/nmol). The tumor size and weight of the mice were monitored every 2 or 3 days.

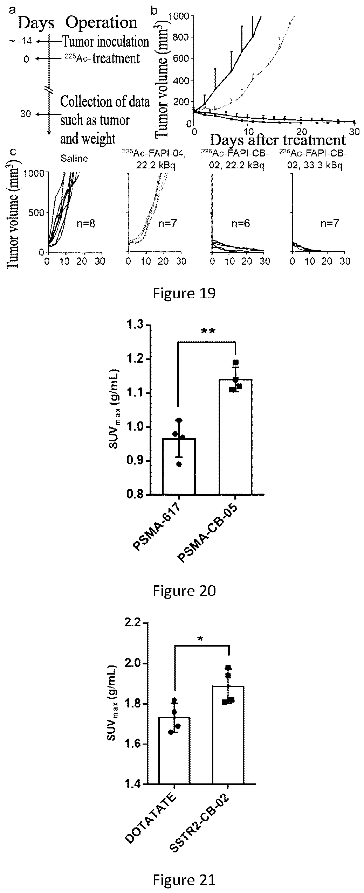

As shown in Fig. 19, 19a is a schematic diagram of the treatment scheme, 19b is an averaged tumor growth curve, and 19c is a detailed tumor growth curve of each mouse in each treatment group. It can be seen that compared with the normal saline control group or FAPI-04 control group, using the same or a higher dose (²²⁵Ac activity) of FAPI-CB-02 results in a significantly better tumor inhibition effect, and using a lower dose may also result in a better therapeutic effect.

### Example 12 Experiment in tumor mouse model with high PSMA expression - PET/CT imaging

All PET/CT scans were carried out on Mediso nanoScan^{®} PET 122S small animal PET/CT.

A certain number (n = 4) of LNCaP tumor mice (for the construction method, see the method in Preclinical Evaluation of a Tailor-Made DOTA-Conjugated PSMA Inhibitor with Optimized Linker Moiety for Imaging and Endoradiotherapy of Prostate Cancer, published by Martina Benesova et al.) were taken and intravenously injected at specified time points with 7.4-18.5 MBq of ⁶⁸Ga-PSMA-617 (as a control, the labeled precursor is readily commercially available, and the structure is specifically disclosed) and ⁶⁸Ga-PSMA-CB-05 molecules (Note: the injection of the control and investigation molecules was separated apart by at least 12 hours until the previous ⁶⁸Ga molecule essentially completely decayed and was essentially completely metabolized). Imaging was carried at the time point of 2 hours, and the SUVₘₐₓ value in tumor was obtained by a software. The histogram of the obtained results is as shown in Fig. 20. The drug tumor uptake of ⁶⁸Ga-PSMA-CB-05 is significantly higher than that of ⁶⁸Ga-PSMA-617 (p < 0.05).

### Example 13 Experiment in tumor mouse model with high SSTR2 expression - PET/CT imaging

All PET/CT scans were carried out on Mediso nanoScan^{®} PET 122S small animal PET/CT.

A certain number (n = 4) of AR42J tumor mice (for the construction method, see the method in Combination radionuclide therapy using ¹⁷⁷Lu-and ⁹⁰Y-labeled somatostatin analogs, published by Marion de Jong et al.) were taken and intravenously injected at specified time points with 7.4-18.5 MBq of ⁶⁸Ga-DOTATATE (as a control, the labeled precursor is readily commercially available, and the structure is specifically disclosed) and ⁶⁸Ga-SSTR2-CB-02 molecules (Note: the injection of the control and investigation molecules was separated apart by at least 12 hours until the previous ⁶⁸Ga molecule essentially completely decayed and was essentially completely metabolized). Imaging was carried at the time point of 2 hours, and the SUVₘₐₓ value in tumor was obtained by a software. The histogram of the obtained results is as shown in Fig. 21. The drug tumor uptake of ⁶⁸Ga-SSTR2-CB-02 is significantly higher than that of ⁶⁸Ga-DOTATATE (p < 0.05).

### Example 14 Experiment in tumor mouse model with high FOLR1 expression - PET/CT imaging

All PET/CT scans were carried out on Mediso nanoScan^{®} PET 122S small animal PET/CT.

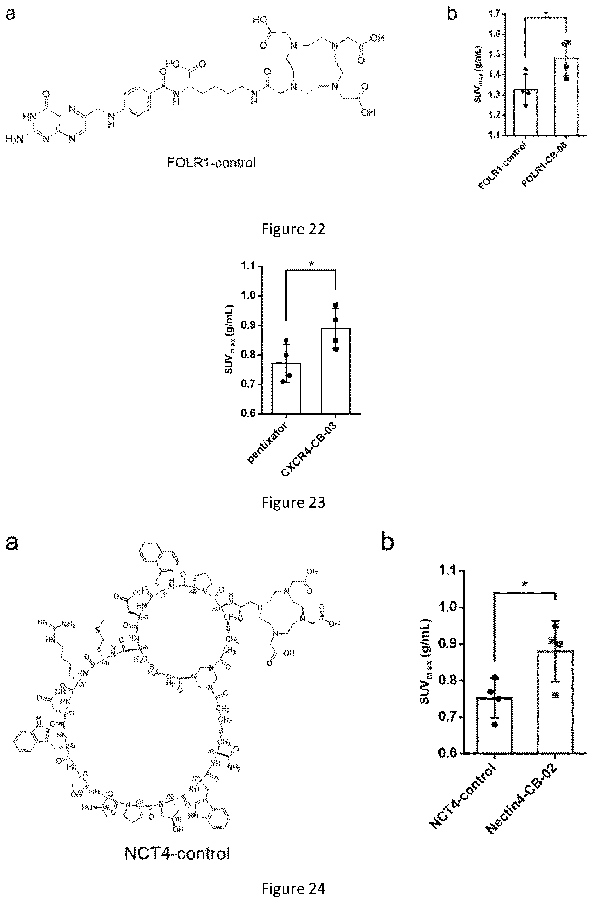

A certain number (n = 4) of KB tumor mice (for the construction method, see the method in Synthesis and biological evaluation of ⁶⁸Ga-labeled Pteroyl-Lys conjugates for folate receptor-targeted tumor imaging, published by Xuran Zhang et al.) were taken and intravenously injected at specified time points with 7.4-18.5 MBq of ⁶⁸Ga-FOLR1-control (as a control, with a structure as shown in Fig. 21a, obtained by reference to the synthesis method in Synthesis and biological evaluation of ⁶⁸Ga-labeled Pteroyl-Lys conjugates for folate receptor-targeted tumor imaging, published by Xuran Zhang et al.) and ⁶⁸Ga-FOLR1-CB-06 molecules (Note: the injection of the control and investigation molecules was separated apart by at least 12 hours until the previous ⁶⁸Ga molecule essentially completely decayed and was essentially completely metabolized). Imaging was carried at the time point of 2 hours, and the SUVₘₐₓ value in tumor was obtained by a software. The histogram of the obtained results is as shown in Fig. 22. The drug tumor uptake of ⁶⁸Ga-FOLR1-CB-06 is significantly higher than that of ⁶⁸Ga-FOLR1 (p < 0.05).

### Example 15 Experiment in tumor mouse model with high CXCR4 expression - PET/CT imaging

All PET/CT scans were carried out on Mediso nanoScan^{®} PET 122S small animal PET/CT.

A certain number (n = 4) of Daudi tumor mice (for the construction method, see the method in PET Imaging of CXCR4 Receptors in Cancer by a New Optimized Ligand, published by Oliver Demmer et al.) were taken and intravenously injected at specified time points with 7.4-18.5 MBq of ⁶⁸Ga-pentixafor (as a control, the labeled precursor is readily commercially available, and the structure is specifically disclosed) and ⁶⁸Ga-CXCR4-CB-03 molecules (Note: the injection of the control and investigation molecules was separated apart by at least 12 hours until the previous ⁶⁸Ga molecule essentially completely decayed and was essentially completely metabolized). Imaging was carried at the time point of 2 hours, and the SUVₘₐₓ value in tumor was obtained by a software. The histogram of the obtained results is as shown in Fig. 23. The drug tumor uptake of ⁶⁸Ga-CXCR4-CB-03 is significantly higher than that of ⁶⁸Ga-pentixafor (p < 0.05).

### Example 16 Experiment in tumor mouse model with high Nectin-4 expression - PET/CT imaging

All PET/CT scans were carried out on Mediso nanoScan^{®} PET 122S small animal PET/CT.

A certain number (n = 4) of SW-780 tumor mice (for the construction method, see the methods in Discovery of BT8009:A Nectin-4 Targeting Bicycle Toxin Conjugate for the Treatment of Cancer, published by Gemma E. Mudd et al., and First-in-human study of the radioligand ⁶⁸Ga-N188 targeting nectin-4 for PET/CT imaging of advanced urothelial carcinoma, published by Xiaojiang Duan et al.) were taken and intravenously injected at specified time points with 7.4-18.5 MBq of ⁶⁸Ga-NCT4-control (as a control, with a structure as shown in Fig. 24a, easily obtained by reference to the synthesis method in First-in-human study of the radioligand ⁶⁸Ga-N188 targeting nectin-4 for PET/CT imaging of advanced urothelial carcinoma, published by Xiaojiang Duan et al. ) or ⁶⁸Ga-Nectin4-CB-02 molecules (Note: the injection of the control and investigation molecules was separated apart by at least 12 hours until the previous ⁶⁸Ga molecule essentially completely decayed and was essentially completely metabolized). Imaging was carried at the time point of 2 hours, and the SUVₘₐₓ value in tumor was obtained by a software. The histogram of the obtained results is as shown in Fig. 24b. The drug tumor uptake of ⁶⁸Ga-Nectin4-CB-02 is significantly higher than that of ⁶⁸Ga-NCT4-control (p < 0.05).

Unless the context clearly indicates otherwise, in the description and the appended claims, the singular forms "a", "an", and "the" include plural forms. Unless otherwise defined, all technical and scientific terms used herein have the same meanings as commonly understood by those of ordinary skill in the art. In addition to the specific order disclosed, the methods described herein may be carried out in any logically possible order.

Representative examples are intended to help illustrate the present disclosure and are not meant to be and should also not be interpreted as limiting the scope of the present disclosure. In fact, in addition to those shown and described herein, many modifications made to the present disclosure and many other examples thereof will become obvious to those skilled in the art, including examples and references to scientific and patent documents cited herein. The examples comprise important additional information, examples and guidance that can be used in practice by the present disclosure in its various examples and equivalents.

## Claims

1. A trifunctional compound or a pharmaceutically acceptable salt, stereoisomer, or solvate thereof, wherein the trifunctional compound has a structure of general formula (I), (II), (III), (IV), or (V), 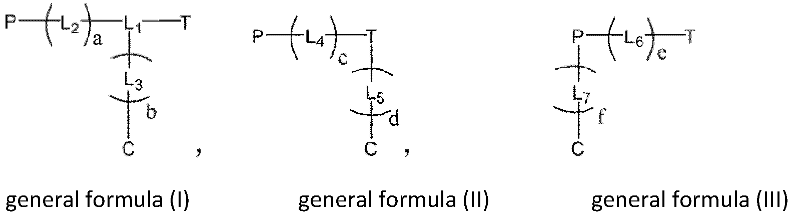 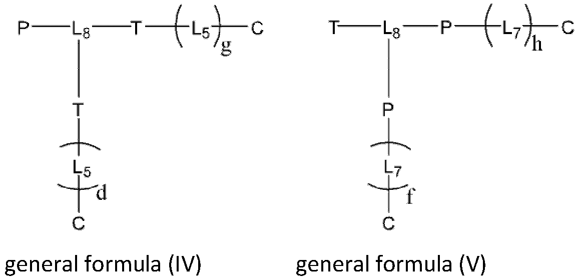
wherein P is a payload comprising at least one radionuclide-containing group, at least one chelating group capable of chelating a radionuclide, or at least one optical dye group;
T is a targeting moiety comprising at least one targeting group capable of targeting a protein or tissue, wherein the targeting group is a small molecule, a polypeptide, or a nucleic acid aptamer;
C is a covalent warhead capable of forming a reversible or irreversible covalent linkage with the protein or tissue targeted by T;
L₁, L₂, L₃, L₄, L₅, L₆, L₇, and L₈ are each independently a linker unit; and
a, b, c, d, e, f, g, and h are independently an integer between 0 and 6.

2. The trifunctional compound or the pharmaceutically acceptable salt, stereoisomer or solvate thereof according to claim 1, wherein C is selected from the group consisting of: 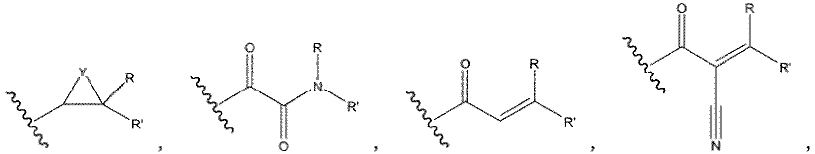 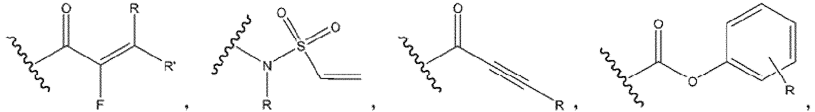 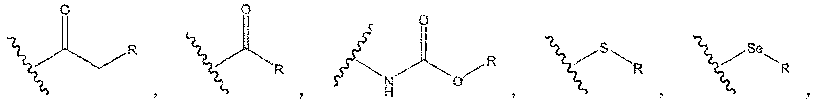 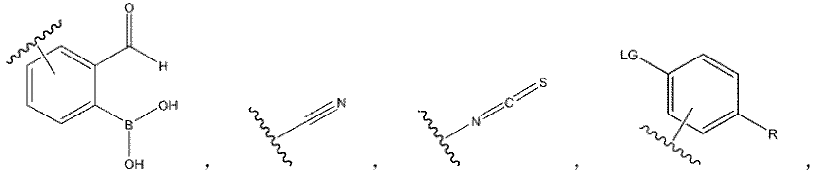 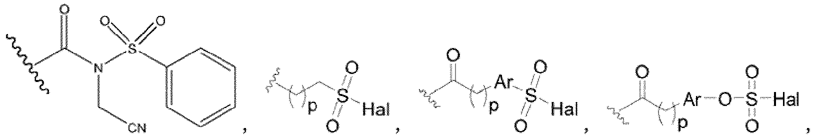 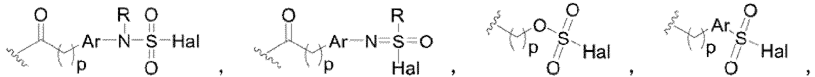 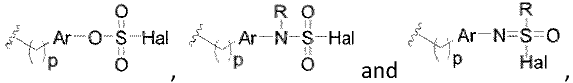 wherein:
Y is selected from the group consisting of O, S, and NR¹;
LG is a leaving group replaceable by a protein residue and is preferably halogen or OTs,
with Ts being p-toluenesulfonyl;
R and R' are independently of each other selected from the group consisting of hydrogen, halogen, nitro, cyano, an optionally substituted alkyl, an optionally substituted cycloalkyl, an optionally substituted heterocycloalkyl, an optionally substituted aryl, an optionally substituted heteroaryl, and an optionally substituted amino group;
R¹ is selected from the group consisting of hydrogen, an optionally substituted alkyl, an optionally substituted cycloalkyl, an optionally substituted heterocycloalkyl, an optionally substituted aryl, and an optionally substituted heteroaryl;
Ar is an optionally substituted aryl or an optionally substituted heteroaryl;
Hal is halogen, preferably F or Cl; and
p is an integer between 0 and 12.

3. The trifunctional compound or the pharmaceutically acceptable salt, stereoisomer or solvate thereof according to claim 1, wherein C is selected from the group consisting of: 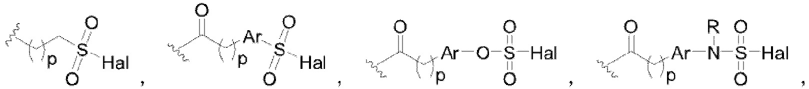 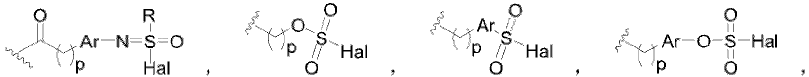 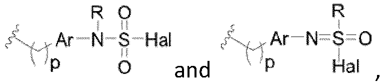 wherein:
Ar is an optionally substituted C₆-C₁₆ aryl, preferably an optionally substituted phenyl, or
an optionally substituted C₅-C₁₂ heteroaryl;
Hal is F;
p is an integer between 0 and 6; and
R is selected from the group consisting of hydrogen, an optionally substituted alkyl, an optionally substituted cycloalkyl, an optionally substituted heterocycloalkyl, an optionally substituted aryl, and an optionally substituted heteroaryl.

4. The trifunctional compound or the pharmaceutically acceptable salt, stereoisomer or solvate thereof according to claim 1, wherein C is selected from the group consisting of: 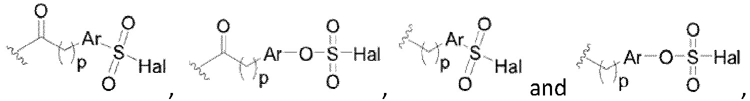 wherein:
Ar is phenyl;
Hal is F; and
p is an integer between 0 and 4.

5. The trifunctional compound or the pharmaceutically acceptable salt, stereoisomer or solvate thereof according to any one of claims 1 to 4, wherein the targeting group targets a target selected from:
fibroblast activation protein-α (FAP-α), prostate-specific membrane antigen (PSMA), poliovirus receptor-associated protein 4 (Nectin-4), programmed death receptor ligand 1 (PD-L1), programmed death receptor 1 (PD-1), human epidermal growth factor receptor 2 (HER2), integrin, gastrin-releasing peptide receptor (GRPR), somatostatin receptor (SSTR) such as SSTR2, folate receptor (FR) such as folate receptor 1 (FOLR1), estrogen receptor (ER), cytotoxic T lymphocyte-associated protein 4 (CTLA-4), chemokine receptor 4 (CXCR4), poly(ADP-ribose) polymerase (PARP), epidermal growth factor receptor (EGFR), fibroblast growth factor receptor (FGFR), disialoganglioside (GD2), vascular endothelial growth factor (VEGF), metastasis-associated lung adenocarcinoma transcript 1 (MALAT-1), insulin growth factor 1 (IGF-1), mesothelin (MSLN), tumor endothelial marker (TEM-1), interleukin-12 (IL-12), leukocyte differentiation antigen CD 13 (CD13), leukocyte differentiation antigen CD 19 (CD19), leukocyte differentiation antigen CD 20 (CD20), leukocyte differentiation antigen CD 22 (CD22), leukocyte differentiation antigen CD 33 (CD33), leukocyte differentiation antigen CD 37 (CD37), leukocyte differentiation antigen CD 38 (CD38), leukocyte differentiation antigen CD 44 (CD44), death receptor 5 (DR5), peroxidase 1 (Prdx I), large neutral amino acid transporter 1 (LAT1), angiotensin converting enzyme 2 (ACE2), vesicular monoamine transporter 2 (VMAT2), dopamine transporter (DAT), and Sigma-1 receptor,
wherein the targeting group optionally comprises one or more warheads or structures capable of forming a reversible or irreversible covalent linkage with the targeted protein or tissue.

6. The trifunctional compound or the pharmaceutically acceptable salt, stereoisomer or solvate thereof according to any one of claims 1 to 5, wherein the targeting group targets fibroblast activation protein-α (FAP-α), prostate-specific membrane antigen (PSMA), integrin, chemokine receptor 4 (CXCR4), folate receptor (FR) such as folate receptor 1 (FOLR1), poliovirus receptor-associated protein 4 (Nectin-4), or somatostatin receptor (SSTR) such as SSTR2, and is selected from the following structures:
Group I: structures targeting fibroblast activation protein-a and having general formula (VI), preferably general formula (VII), 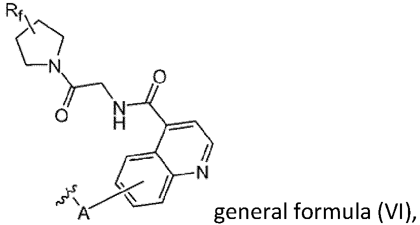
wherein A is selected from O, S, and NR_{A}, with R_{A} being selected from H and a C₁-C₆ alkyl, and
a plurality of R_{f} are present on the pyrrolidine ring, in which each R_{f} is independently selected from H, F, Cl, -CN, and a C₁-C₆ alkyl, and optionally two R_{f} groups on adjacent carbons may be connected to each other to form a cycloalkyl, preferably a C₃-C₇ cycloalkyl; 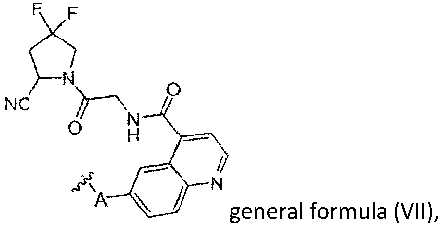
wherein A is selected from O, S, and NR_{A}, where R_{A} is selected from H, methyl, ethyl, n-propyl, and isopropyl;
Group II: peptidomimetic groups having general formula (VIII), 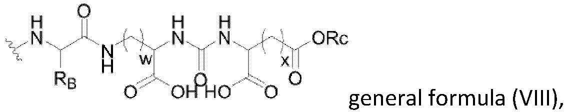
wherein R_{B} is -CH₂R_{B'} in which R_{B'} is an aryl, preferably an optionally substituted phenyl or naphthyl,
R_{c} is H or an alkyl,
w and x are independently 1, 2, 3, or 4, and
the peptidomimetic group preferably has the following structure 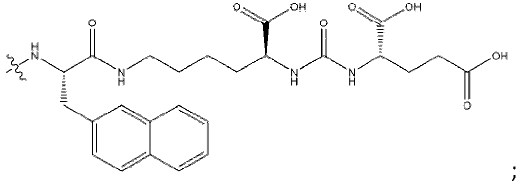
Group III: cyclic peptide groups having general formula (X), 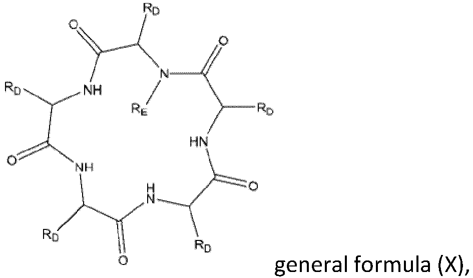
wherein each R_{D} is independently selected from H, -CH₂R_{D'}, -CH₂COOH, -CH₂CH₂CH₂NH₂, - CH₂CH₂CH₂CH₂NH₂, and 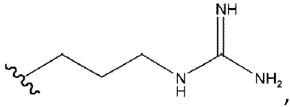 **with** R_{D}, **being** an aryl, preferably an optionally substituted phenyl or naphthyl, wherein the cyclic peptide is connected to the rest of the trifunctional compound via R_{D'} and/or the terminal -NH₂ of R_{D}, or the -CH₂R_{D}, or R_{D'} may form a part of the covalent warhead C;
R_{E} is H or methyl;
the cyclic peptide group preferably has the following formula 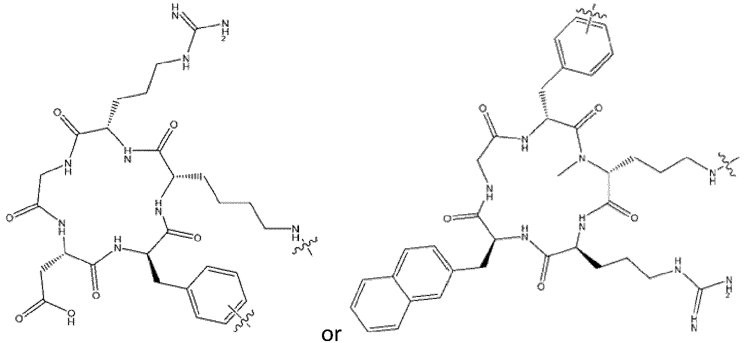
wherein the two wavy lines in each formula represent optional linkage sites;
Group IV: folic groups having general formula (XI), 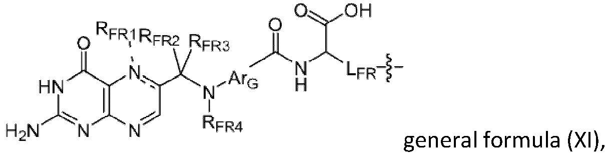
wherein the dashed line represents a bond that is present or absent, and only one of the bonds represented by the two dashed lines exists,
R_{FR1}, when present, is H or a C₁-C₄ alkyl,
R_{FR2}, R_{FR3}, and R_{FR4} are each independently selected from H or a C₁-C₄ alkyl,
Ar_{G} is an aryl or heteroaryl, preferably an optionally substituted phenyl or pyridyl,
L_{FR} is -CH₂-R_{FR5}-, -(CH₂)₂-R_{FR5}-, -(CH₂)₃-R_{FR5}-, -(CH₂)₃-R_{FR5}-, or -(CH₂)₄-R_{FR5}-, in which R_{FR5} is selected from carbonyl (-CO-) or NR_{FR6}, and R_{FR6} is H or a C₁-C₄ alkyl, and
the folic group preferably has the following structure 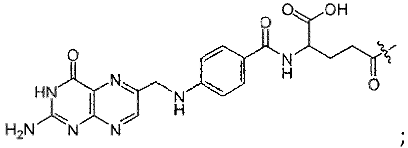
Group V: disulfide bond-containing cyclic groups having general formula (XII), 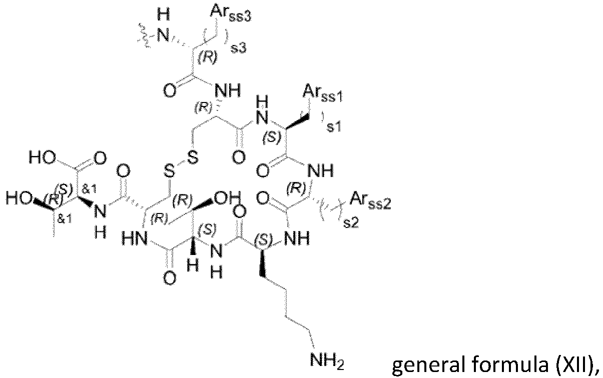
wherein Arₛₛ₁, Arₛₛ₂, and Arₛₛ₃ are each independently selected from an optionally substituted aryl or heteroaryl, preferably an optionally substituted C₆-C₁₀ aryl or C₅-C₁₀ heteroaryl, preferably, Arₛₛ₁ and Arₛₛ₃ are a C₆-C₁₀ aryl optionally substituted with -OH, and
Arₛₛ₂ is a C₅-C₁₀ heteroaryl,
s1, s2, and s3 are each independently selected from 1, 2, 3, or 4,
wherein the disulfide bond-containing cyclic group is connected to the rest of the trifunctional compound via a site shown by the wavy line, or -(CH₂)ₛ₃Arₛₛ₃ or Arₛₛ₃ may form a part of the covalent warhead C,
the disulfide bond-containing cyclic group preferably has the following structure 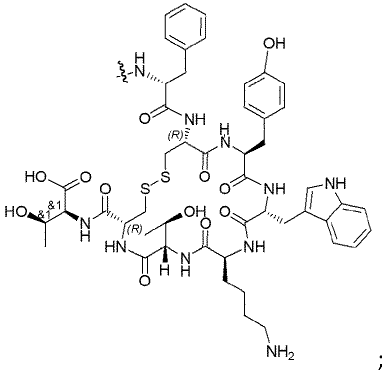 and
Group VI: bicyclic peptide groups, preferably bicyclic peptide groups targeting poliovirus receptor-associated protein 4 (Nectin-4),
more preferably, the bicyclic peptide group has the following structure

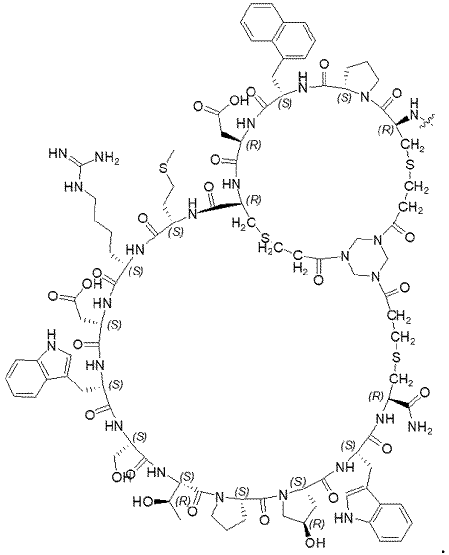

7. The trifunctional compound or the pharmaceutically acceptable salt, stereoisomer or solvate thereof according to any one of claims 1 to 6, wherein the payload comprises at least one group selected from the following groups:
Group I: groups with at least one radionuclide, selected from: 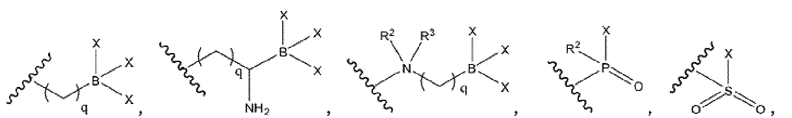 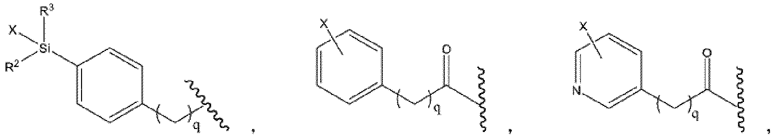 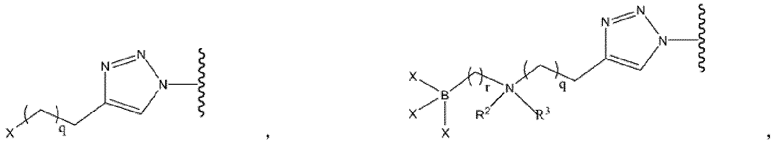 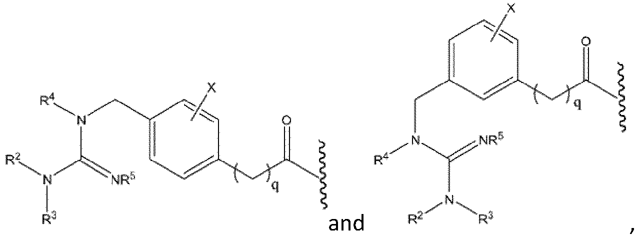
wherein R², R³, R⁴, and R⁵ are independently of each other selected from the group consisting of hydrogen, an optionally substituted alkyl, an optionally substituted cycloalkyl, an optionally substituted heterocycloalkyl, an optionally substituted aryl, and an optionally substituted heteroaryl,
X is selected from ¹⁸F, ¹²³I, ¹²⁴I, ¹²⁵I, ¹³¹I, and ²¹¹At, or non-radioactive isotopes thereof,
q and r are independently an integer between 0 and 4; and
the N on the triazole ring at the linkage site shown herein may not exist on the payload, but on a linker;
Group II: chelating groups capable of chelating a radionuclide, selected from: 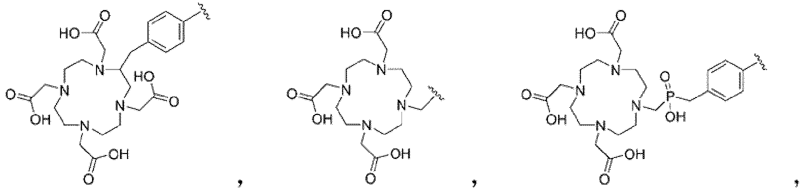 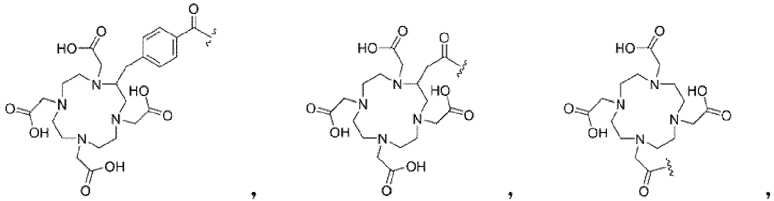 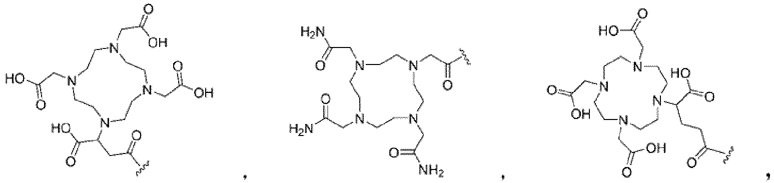 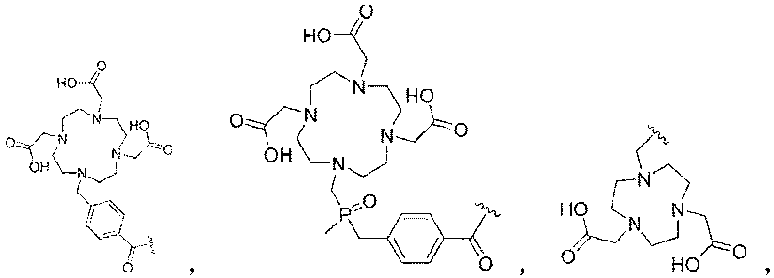 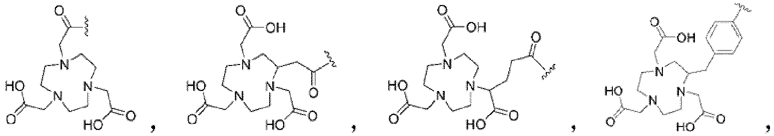 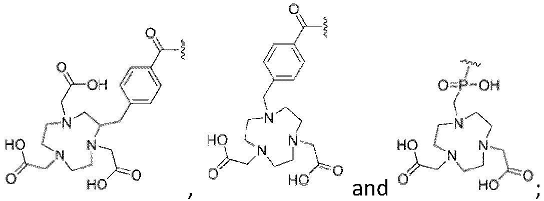
Group III: optical dye groups selected from:

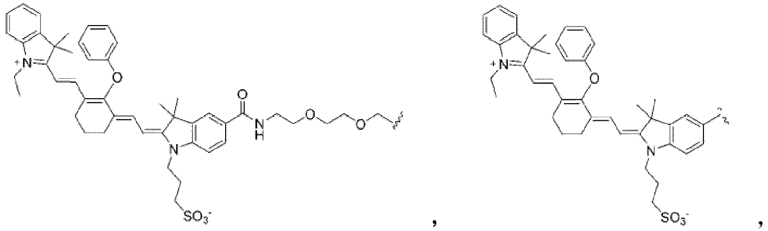

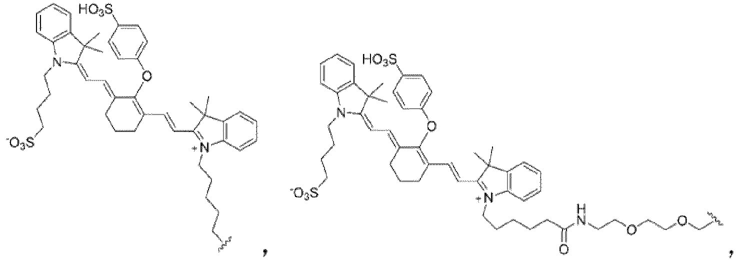

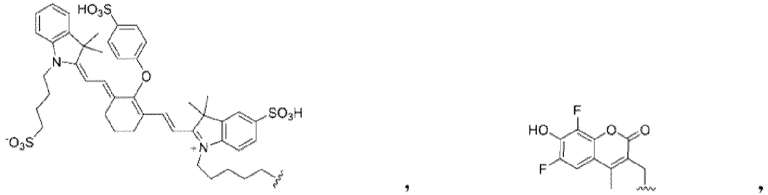

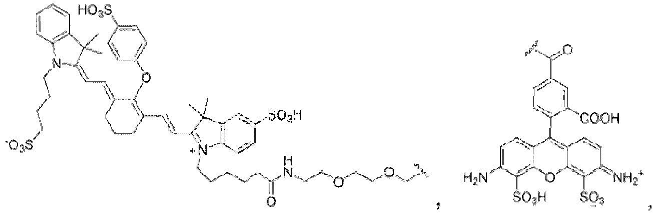

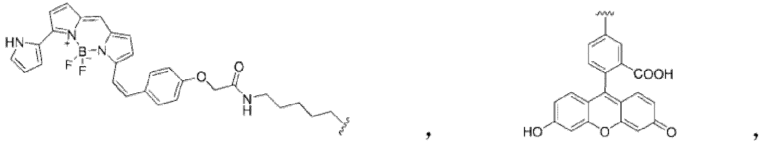

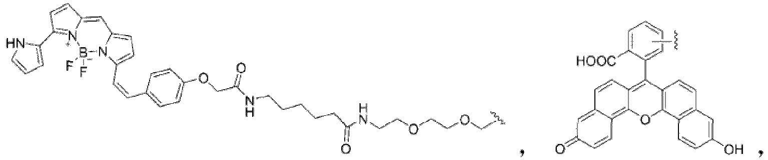

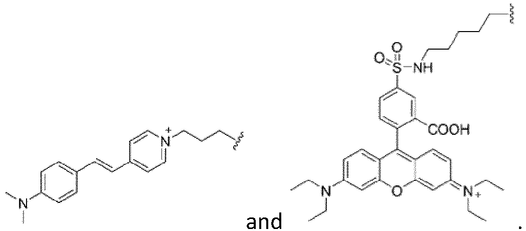

8. The trifunctional compound or the pharmaceutically acceptable salt, stereoisomer or solvate thereof according to any one of claims 1 to 7, wherein
L₁ and L₈ are independently selected from: 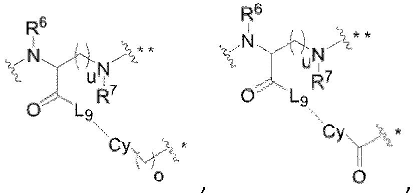 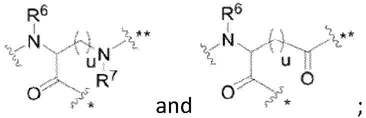
L₂, L₃, L₄, L₅, L₆, and L₇ are each independently selected from:
a single bond, 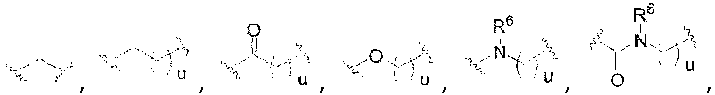 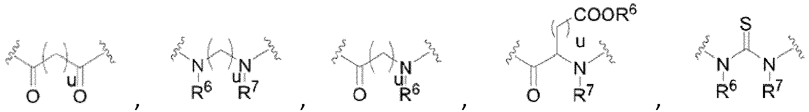 and 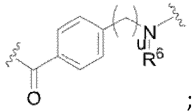
L₉ is a single bond, -CH₂-, -NHCH₂-, or 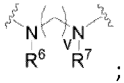
Cy is selected from 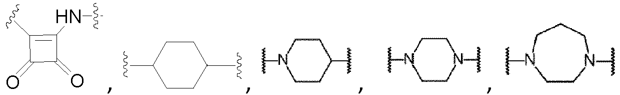 and 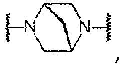
a to h are independently 0, 1, 2, or 3,
u, v, and o are independently 1, 2, 3, 4, or 5,
R⁶ and R⁷ are independently of each other selected from the group consisting of hydrogen, an optionally substituted alkyl, an optionally substituted cycloalkyl, an optionally substituted heterocycloalkyl, an optionally substituted aryl, and an optionally substituted heteroaryl, and
* and ** show the sites linked to two moieties of the trifunctional compound optionally via L₂, L₃, L₄, L₅, L₆, or L₇,
provided that in the linkers formed from L₁, L₂, L₃, L₄, L₅, L₆, L₇, and L₈, heteroatoms are not directly covalently bonded to each other, where the heteroatoms are selected from N, O, and S.

9. The trifunctional compound or the pharmaceutically acceptable salt, stereoisomer or solvate thereof according to any one of claims 1 to 8, wherein the trifunctional compound has a structure of general formula (I), wherein at least one L₁ is 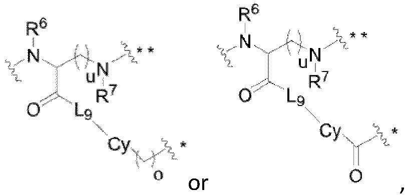
L₁ preferably has the following structure: 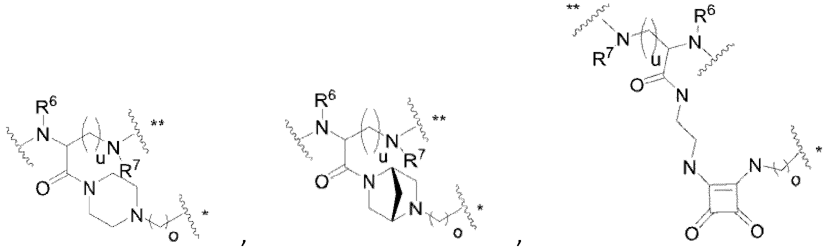 or 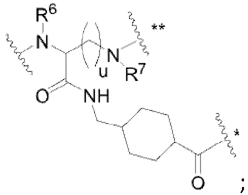
wherein R⁶, R⁷, L₉, Cy, a, b, o, and u are as defined in claim 8.

10. The trifunctional compound or the pharmaceutically acceptable salt, stereoisomer or solvate thereof according to claim 1, wherein the trifunctional compound has a structure of general formula (I): 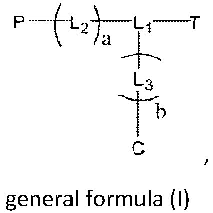 wherein:
L₁ is a trivalent linker unit,
L₂ and L₃ are each independently a divalent linker unit;
a and b are independently an integer between 0 and 6;
C is a covalent warhead selected from the group consisting of: 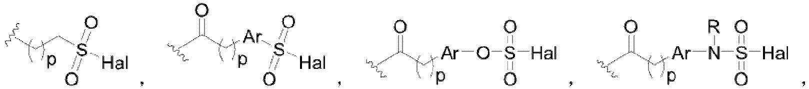 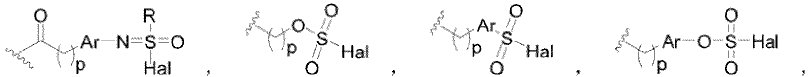 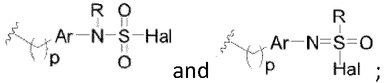 wherein:
Ar is an optionally substituted C₆-C₁₀ aryl, preferably an optionally substituted phenyl, or an optionally substituted;
Hal is F or Cl;
p is an integer between 0 and 6;
R is selected from the group consisting of hydrogen, an optionally substituted alkyl, an optionally substituted cycloalkyl, an optionally substituted heterocycloalkyl, an optionally substituted aryl, and an optionally substituted heteroaryl;
T is a targeting group targeting fibrocyte activation protein-α (FAP-α); and
P is a chelating group capable of chelating a radionuclide.

11. The trifunctional compound or the pharmaceutically acceptable salt, stereoisomer or solvate thereof according to claim 10, wherein the trifunctional compound has a structure of general formula (I),
wherein:
L₁ is a trivalent linker selected from: 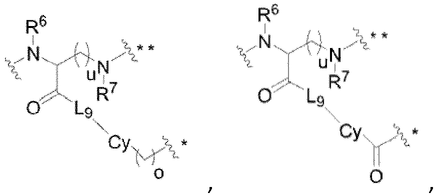 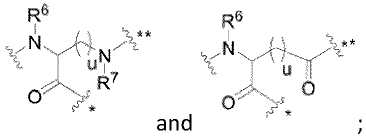
L₂ and L₃ are each independently selected from:
a single bond, 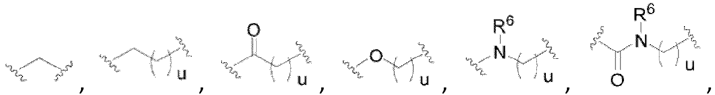 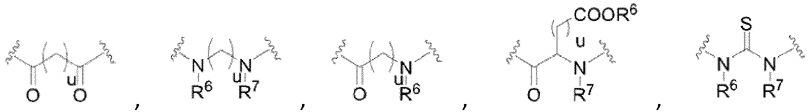 and 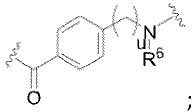
L₉ is a single bond, -CH₂-, -NHCH₂-, or 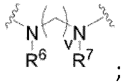
Cy is selected from 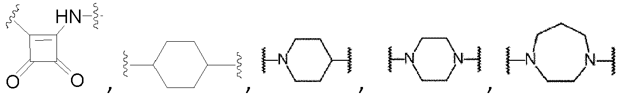 and 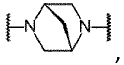
a, b, and p are independently 0, 1, 2, or 3,
u is independently 1, 2, 3, 4, or 5,
R⁶ and R⁷ are independently of each other selected from the group consisting of hydrogen, an optionally substituted alkyl, an optionally substituted cycloalkyl, an optionally substituted heterocycloalkyl, an optionally substituted aryl, and an optionally substituted heteroaryl, and
* is the site linking T,
** is the site linking C,
provided that in the linkers formed by L₁, L₂, and L₃, heteroatoms are not directly covalently bonded to each other, where the heteroatoms are selected from N, O, and S; C is selected from the group consisting of: 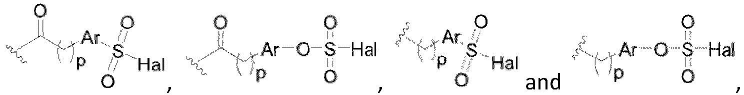 wherein:
Ar is phenyl;
Hal is F;
p is an integer between 0 and 4;
T is a targeting group targeting fibrocyte activation protein-α (FAP-α); and
P is a chelating group capable of chelating a radionuclide and is selected from:

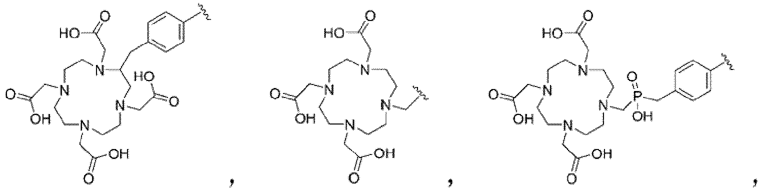

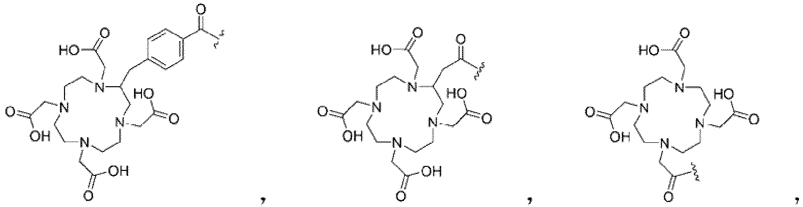

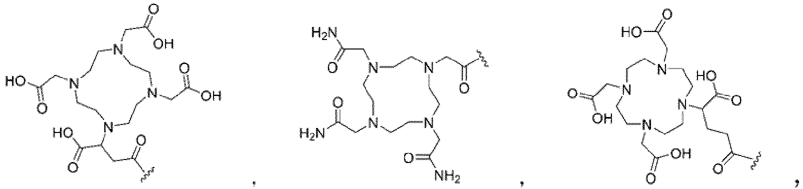

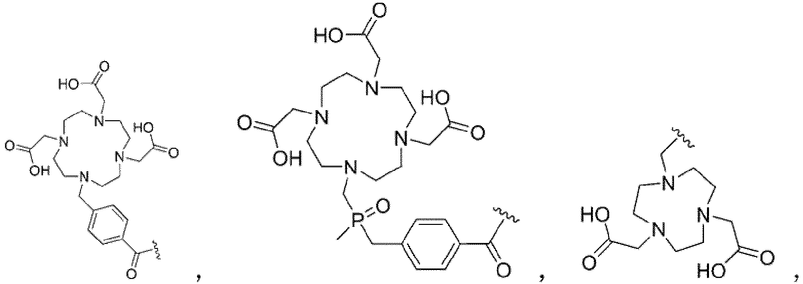

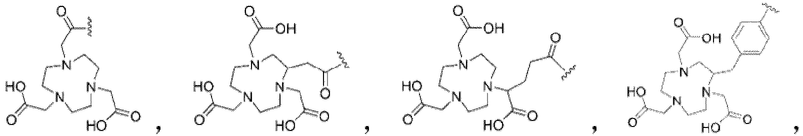

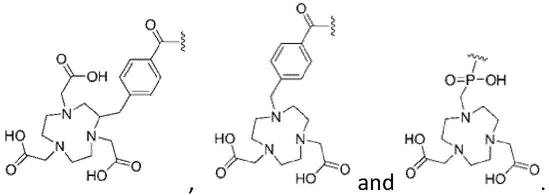

12. The trifunctional compound or the pharmaceutically acceptable salt, stereoisomer or solvate thereof according to claim 10 or 11, wherein the trifunctional compound has a structure of general formula (I),
wherein:
C and P are as defined in claim 11,
L₁ has the following structure: 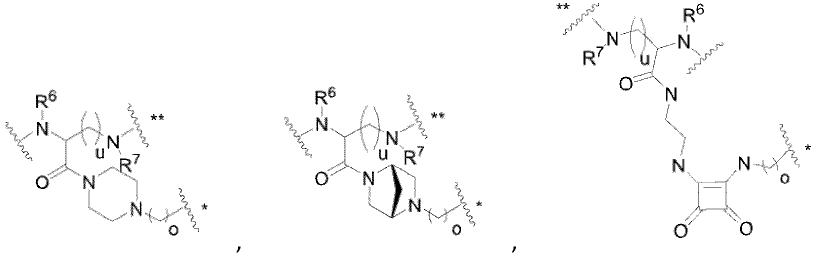 or 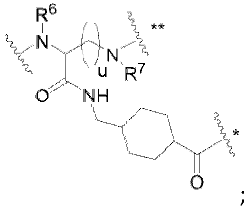
a and b are both 1,
L₂ and L₃ are independently selected from:
a single bond, 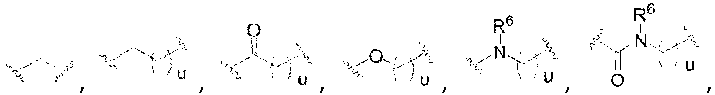 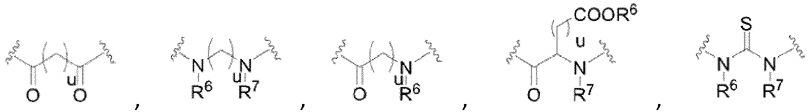 and 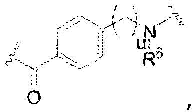
wherein u and o are independently 1, 2, 3, 4, or 5,
R⁶ and R⁷ are independently of each other selected from hydrogen and an optionally substituted C₁-C₄,
* is the site linking T,
** is the site linking C,
provided that in the linkers formed by L₁, L₂, and L₃, heteroatoms are not directly covalently bonded to each other, where the heteroatoms are selected from N, O, and S;
T has general formula (VI), preferably general formula (VII), 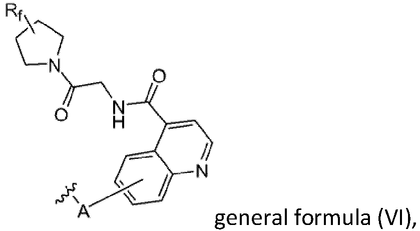
wherein A is selected from O, S, and NR_{A}, with R_{A} being selected from H and a C₁-C₆ alkyl, a plurality of R_{f} are present on the pyrrolidine ring, in which each R_{f} is independently selected from H, F, Cl, -CN, and a C₁-C₆ alkyl, and optionally two R_{f} groups on adjacent carbons may be connected to each other to form a cycloalkyl, preferably a C₃-C₇ cycloalkyl; 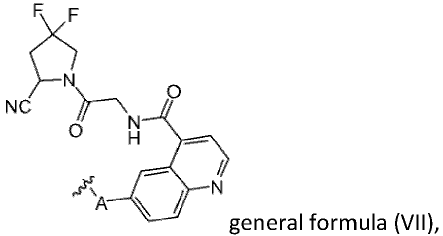
wherein A is selected from O, S, and NR_{A} in which R_{A} is selected from H, methyl, ethyl, n-propyl, and isopropyl.

13. The trifunctional compound or the pharmaceutically acceptable salt, stereoisomer or solvate thereof according to claim 1, wherein the trifunctional compound has a structure of general formula (I),
wherein:
L₁ is a trivalent linker group selected from: 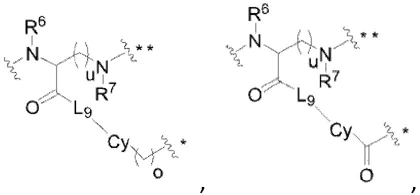 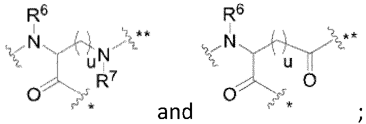
L₂ and L₃ are each independently selected from:
a single bond, 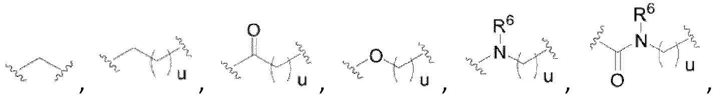 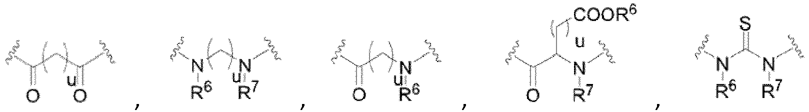 and 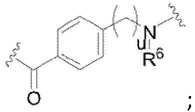
L₉ is a single bond, -CH₂-, -NHCH₂-, or 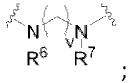
Cy is selected from 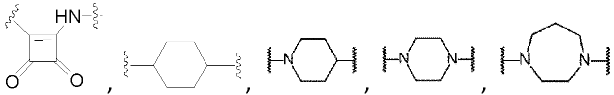 and 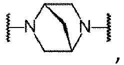
a and b are independently 0, 1, 2, or 3,
u is independently 1, 2, 3, 4, or 5,
R⁶ and R⁷ are independently of each other selected from the group consisting of hydrogen, an optionally substituted alkyl, an optionally substituted cycloalkyl, an optionally substituted heterocycloalkyl, an optionally substituted aryl, and an optionally substituted heteroaryl, and
* is the site linking T,
** is the site linking C,
provided that in the linkers formed by L₁, L₂, and L₃, heteroatoms are not directly covalently bonded to each other, where the heteroatoms are selected from N, O, and S;
C is selected from the group consisting of the following groups: 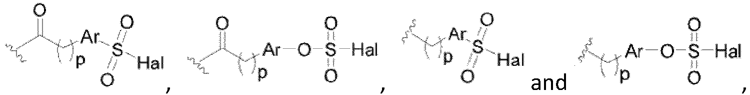 wherein:
Ar is phenyl;
Hal is F;
p is an integer between 0 and 4;
T is a targeting group targeting prostate-specific membrane antigen (PSMA);
P is a chelating group capable of chelating a radionuclide and is selected from:

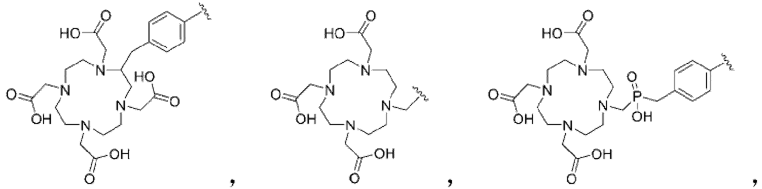

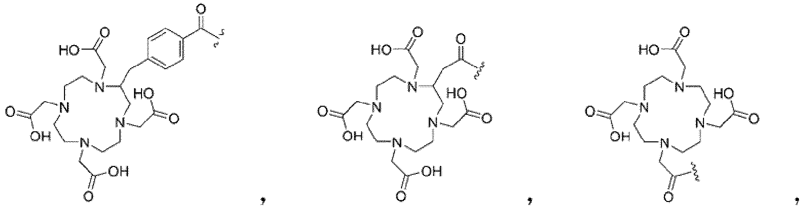

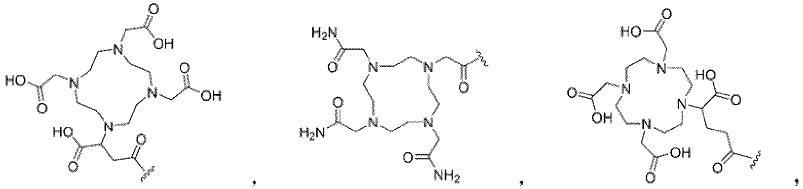

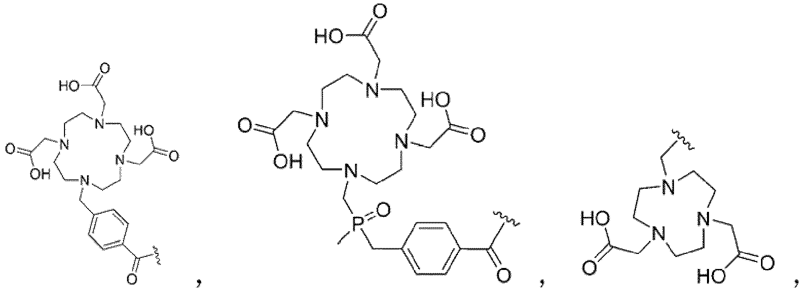

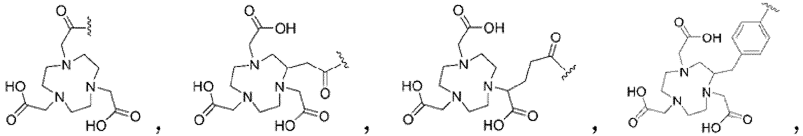

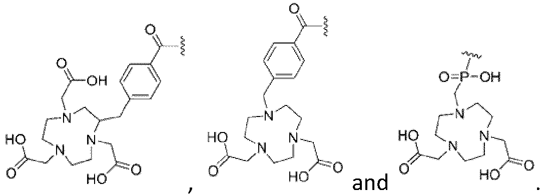

14. The trifunctional compound or the pharmaceutically acceptable salt, stereoisomer or solvate thereof according to claim 13, wherein the trifunctional compound has a structure of general formula (I),
wherein:
C and P are as defined in claim 13,
L₁ has the following structure: 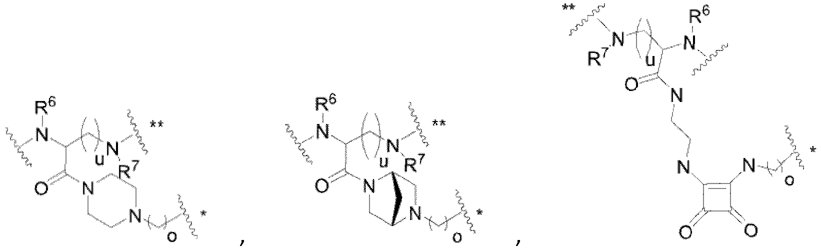 or 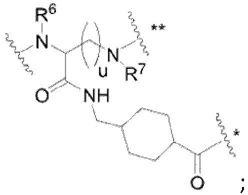
a and b are both 1,
L₂ and L₃ are independently selected from:
a single bond, 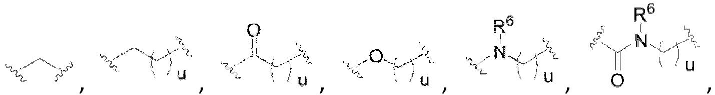 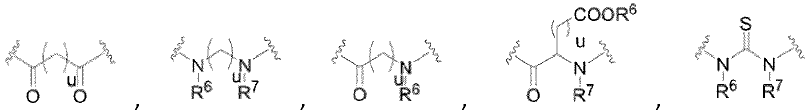 and 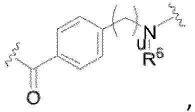
wherein u and o are independently 1, 2, 3, 4, or 5,
R⁶ and R⁷ are independently of each other selected from hydrogen and an optionally substituted C₁-C₄,
* is the site linking T,
** is the site linking C,
provided that in the linkers formed by L₁, L₂, and L₃, heteroatoms are not directly covalently bonded to each other, where the heteroatoms are selected from N, O, and S;
T has general formula (VIII), 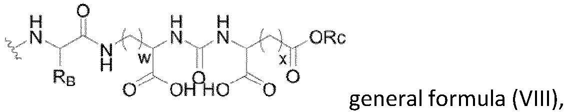
wherein R_{B} is -CH₂R_{B'} in which R_{B'} is an aryl, preferably an optionally substituted phenyl or naphthyl,
R_{c} is H or an alkyl,
w and x are independently 1, 2, 3, or 4, and
T preferably has the following structure

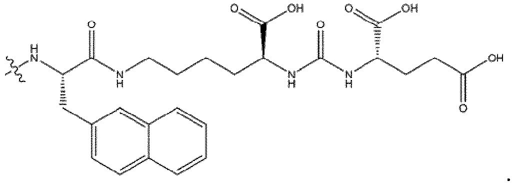

15. The trifunctional compound or the pharmaceutically acceptable salt, stereoisomer or solvate thereof according to claim 1, wherein the trifunctional compound has a structure of general formula (I),
wherein:
L₁ is a trivalent linker group selected from: 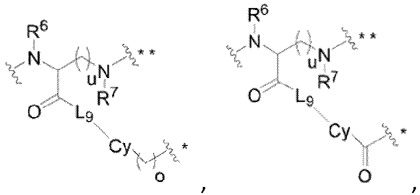 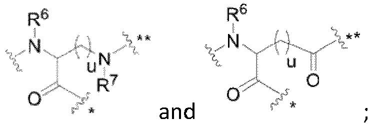
L₂ and L₃ are each independently selected from:
a single bond, 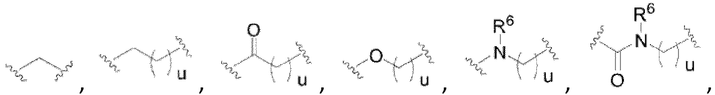 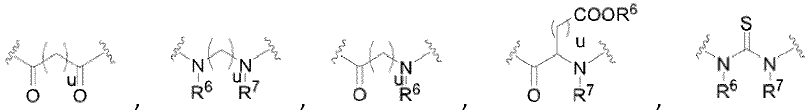 and 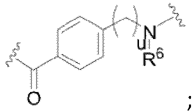
L₉ is a single bond, -CH₂-, -NHCH₂-, or 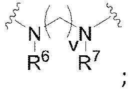
Cy is selected from 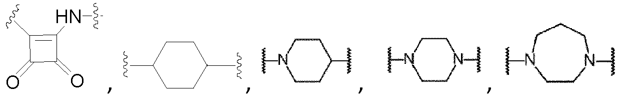 and 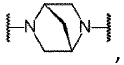
a and b are independently 0, 1, 2, or 3,
u is independently 1, 2, 3, 4, or 5,
R⁶ and R⁷ are independently of each other selected from the group consisting of hydrogen, an optionally substituted alkyl, an optionally substituted cycloalkyl, an optionally substituted heterocycloalkyl, an optionally substituted aryl, and an optionally substituted heteroaryl, and
* is the site linking C,
** is the site linking P,
provided that in the linkers formed by L₁, L₂, and L₃, heteroatoms are not directly covalently bonded to each other, where the heteroatoms are selected from N, O, and S;
C is selected from the group consisting of: 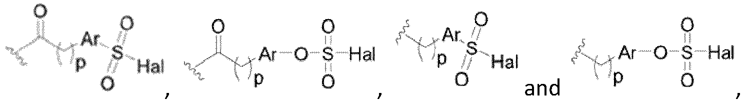 wherein:
Ar is phenyl;
Hal is F;
p is an integer between 0 and 4;
T is a targeting group targeting folate receptor (FR) such as folate receptor 1 (FOLR1);
P is a chelating group capable of chelating a radionuclide and is selected from:

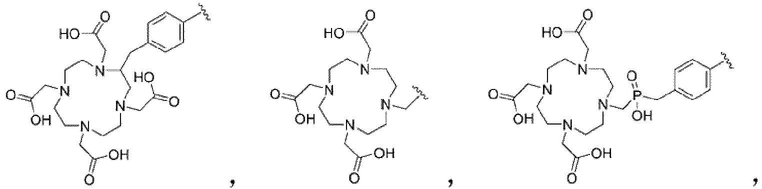

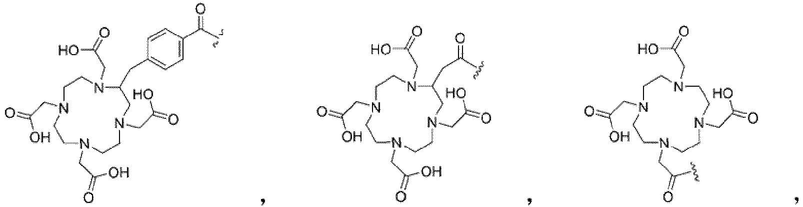

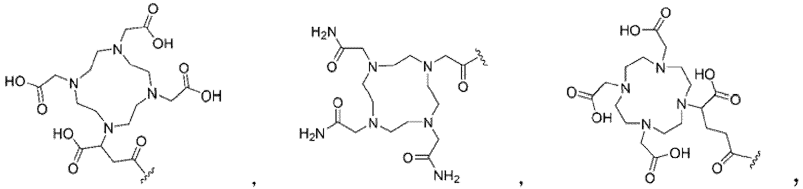

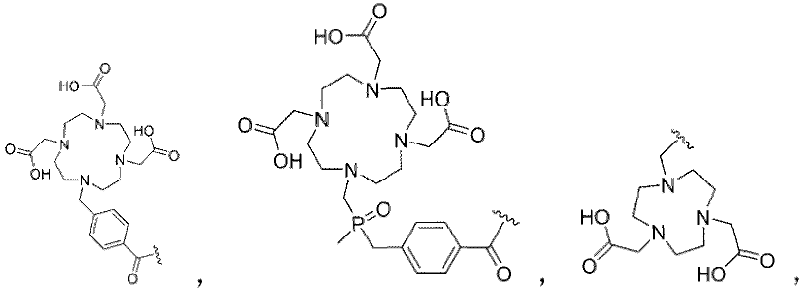

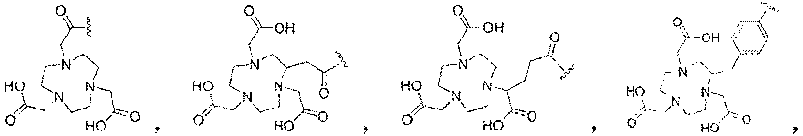

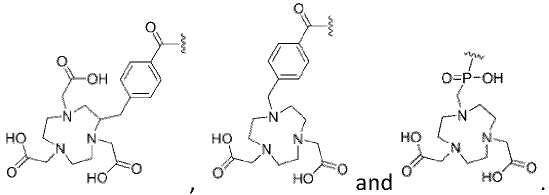

16. The trifunctional compound or the pharmaceutically acceptable salt, stereoisomer or solvate thereof according to claim 15, wherein the trifunctional compound has a structure of general formula (I),
wherein:
C and P are as defined in claim 15,
L₁ has the following structure: 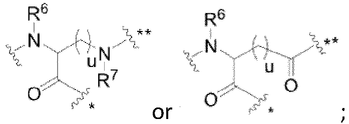
a and b are both 1,
L₂ and L₃ are independently selected from:
a single bond, 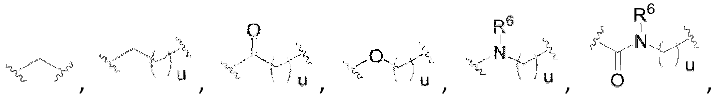 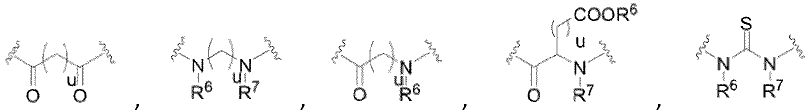 and 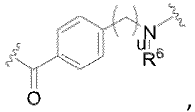
wherein u is independently 1, 2, 3, 4, or 5,
R⁶ and R⁷ are independently of each other selected from hydrogen and an optionally substituted C₁-C₄,
* is the site linking C,
** is the site linking P,
provided that in the linkers formed by L₁, L₂, and L₃, heteroatoms are not directly covalently bonded to each other, where the heteroatoms are selected from N, O, and S;
T has general formula (XI), 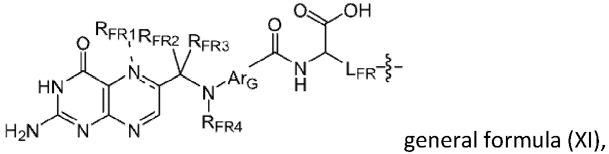
wherein the dashed line represents a bond that is present or absent, and only one of the bonds shown by the two dashed lines exists,
R_{FR1}, when present, is H or a C₁-C₄ alkyl,
R_{FR2}, R_{FR3}, and R_{FR4} are each independently selected from H or a C₁-C₄ alkyl,
Ar_{G} is an aryl or heteroaryl, preferably an optionally substituted phenyl or pyridyl,
L_{FR} is -CH₂-R_{FR5}-, -(CH₂)₂-R_{FR5}-, -(CH₂)₃-R_{FR5}-, -(CH₂)₃-R_{FR5}-, or -(CH₂)₄-R_{FR5}-, in which R_{FR5} is selected from carbonyl (-CO-) or NR_{FR6}, and R_{FR6} is H or a C₁-C₄ alkyl, and
T preferably has the following structure

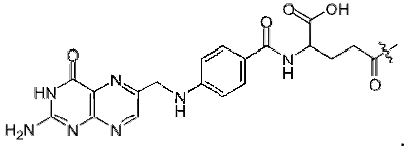

17. The trifunctional compound or the pharmaceutically acceptable salt, stereoisomer or solvate thereof according to claim 1, wherein the trifunctional compound has a structure of general formula (I),
wherein:
L₁ is a trivalent linker selected from: 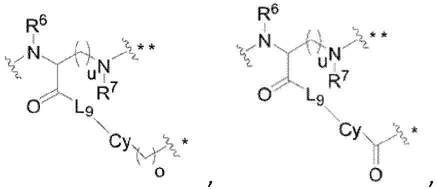 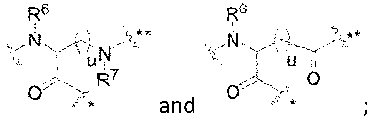
L₂ and L₃ are each independently selected from:
a single bond, 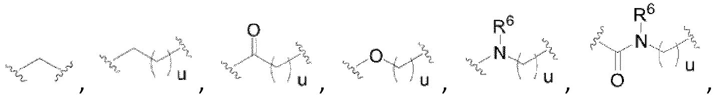 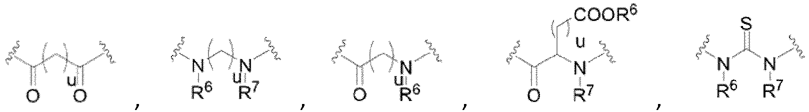 and 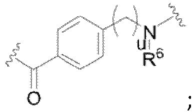
L₉ is a single bond, -CH₂-, -NHCH₂-, or 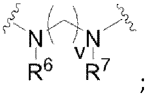
Cy is selected from 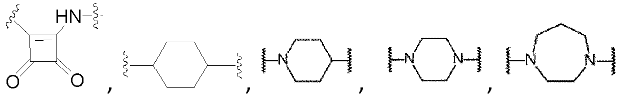 and 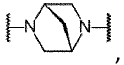
a and b are independently 0, 1, 2, or 3,
u is independently 1, 2, 3, 4, or 5,
R⁶ and R⁷ are independently of each other selected from the group consisting of hydrogen, an optionally substituted alkyl, an optionally substituted cycloalkyl, an optionally substituted heterocycloalkyl, an optionally substituted aryl, and an optionally substituted heteroaryl, and
* is the site linking T,
** is the site linking C,
provided that in the linkers formed by L₁, L₂, and L₃, heteroatoms are not directly covalently bonded to each other, where the heteroatoms are selected from N, O, and S;
C is selected from the group consisting of: 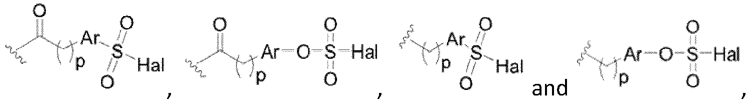 wherein:
Ar is phenyl;
Hal is F;
p is an integer between 0 and 4;
T is a targeting group targeting poliovirus receptor-associated protein 4 (Nectin-4);
P is a chelating group capable of chelating a radionuclide and is selected from:

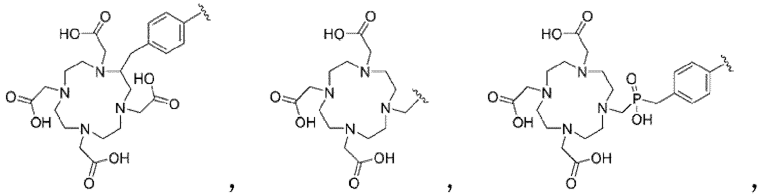

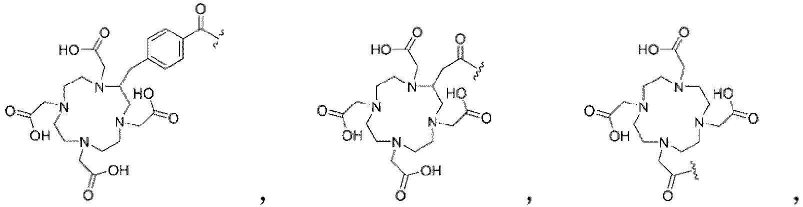

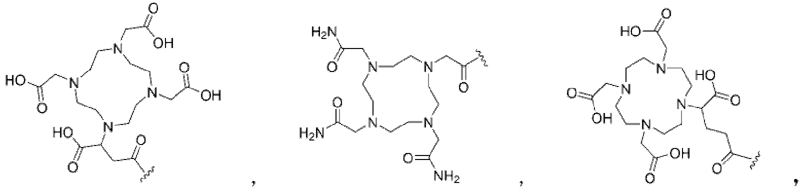

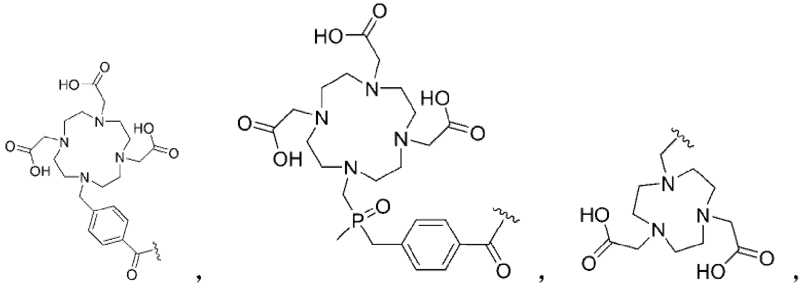

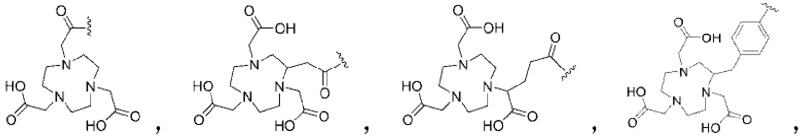

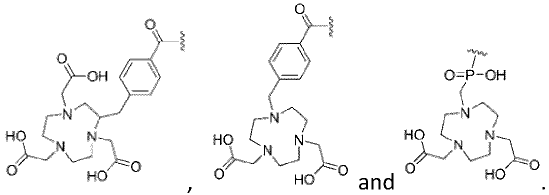

18. The trifunctional compound or the pharmaceutically acceptable salt, stereoisomer or solvate thereof according to claim 17, wherein the trifunctional compound has a structure of general formula (I),
wherein:
C and P are as defined in claim 17,
L₁ has the following structure: 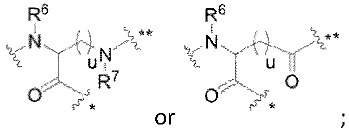
a and b are both 1,
L₂ and L₃ are independently selected from:
a single bond, 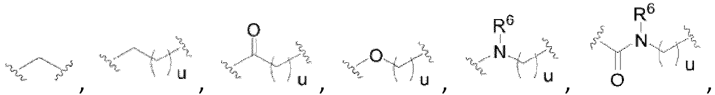 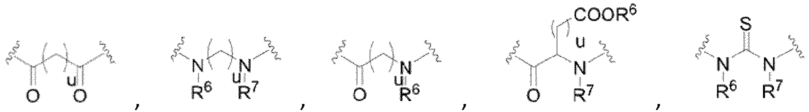 and 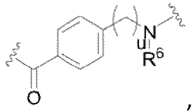
wherein u is 1, 2, 3, 4, or 5,
R⁶ and R⁷ are independently of each other selected from hydrogen and an optionally substituted C₁-C₄,
* is the site linking T,
** is the site linking C,
provided that in the linkers formed by L₁, L₂, and L₃, heteroatoms are not directly covalently bonded to each other, where the heteroatoms are selected from N, O, and S;
T is a bicyclic peptide group, preferably a bicyclic peptide group targeting poliovirus receptor-associated protein 4 (Nectin-4),
more preferably, the bicyclic peptide group has the following structure

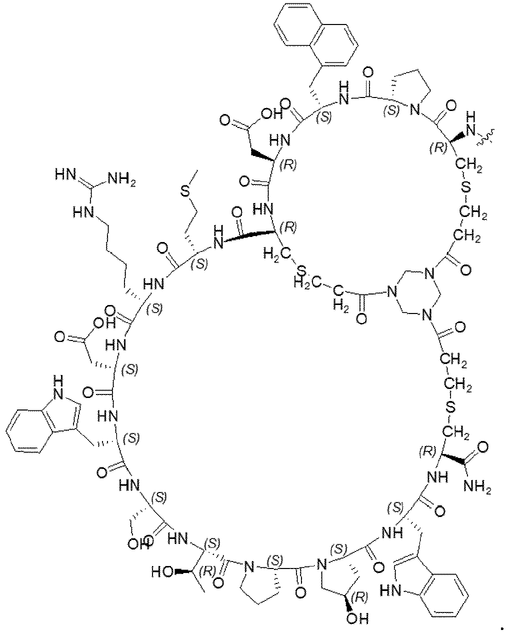

19. The trifunctional compound or the pharmaceutically acceptable salt, stereoisomer or solvate thereof according to claim 1, wherein the trifunctional compound has a structure of general formula (II): 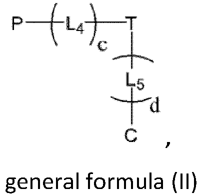 wherein:
L₄ and L₅ are each independently a divalent linker unit;
c and d are independently an integer of 0 to 6;
C is a covalent warhead selected from the group consisting of: 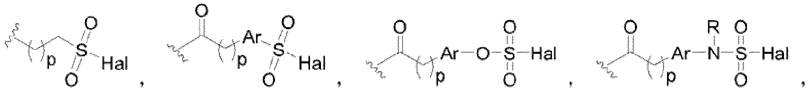 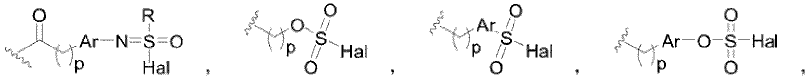 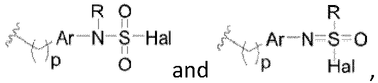 wherein:
Ar is an optionally substituted C₆-C₁₀ aryl, preferably an optionally substituted phenyl, or an optionally substituted C₅-C₁₂ heteroaryl;
Hal is F or Cl;
p is an integer between 0 and 6; and
R is selected from the group consisting of hydrogen, an optionally substituted alkyl, an optionally substituted cycloalkyl, an optionally substituted heterocycloalkyl, an optionally substituted aryl, and an optionally substituted heteroaryl;
T is a targeting group targeting chemokine receptor 4 (CXCR4); and
P is a chelating group capable of chelating a radionuclide.

20. The trifunctional compound or the pharmaceutically acceptable salt, stereoisomer or solvate thereof according to claim 19, wherein the trifunctional compound has a structure of general formula (II):
wherein:
L₄ and L₅ are each independently selected from:
a single bond, 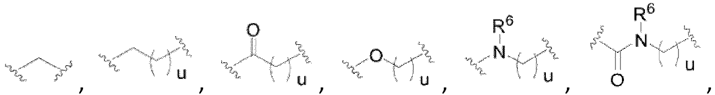 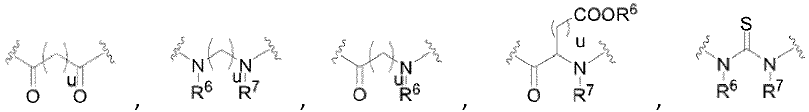 and 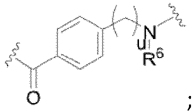
c and d are independently 0, 1, 2, or 3,
u is independently 1, 2, 3, 4, or 5,
R⁶ and R⁷ are independently of each other selected from the group consisting of hydrogen, an optionally substituted alkyl, an optionally substituted cycloalkyl, an optionally substituted heterocycloalkyl, an optionally substituted aryl, and an optionally substituted heteroaryl, and
provided that in the linkers formed by L₄ and L₅, heteroatoms are not directly covalently bonded to each other, where the heteroatoms are selected from N, O, and S;
C is selected from the group consisting of: 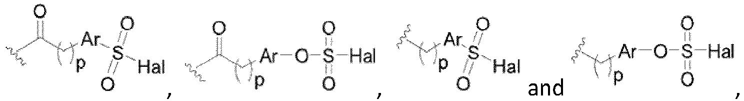 wherein:
Ar is phenyl;
Hal is F;
p is an integer between 0 and 3;
T is a targeting group targeting chemokine receptor 4 (CXCR4); and
P is a chelating group capable of chelating a radionuclide and is selected from:

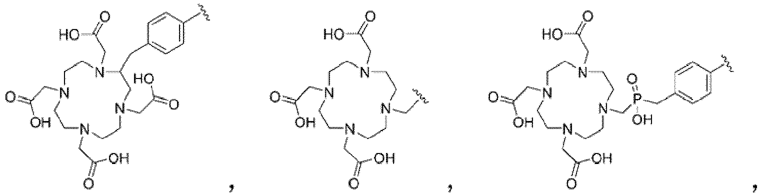

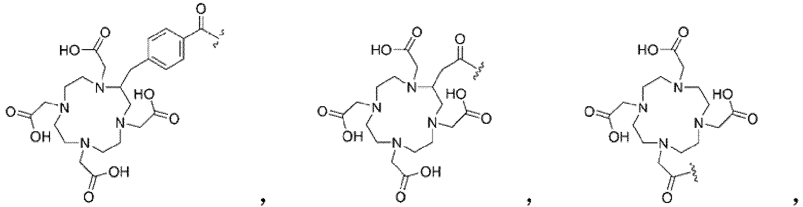

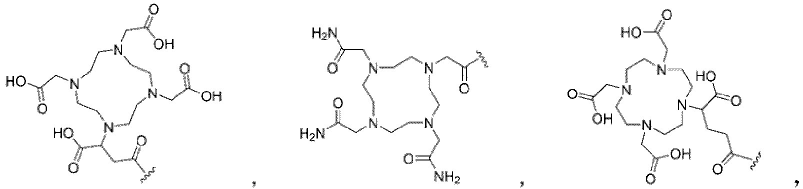

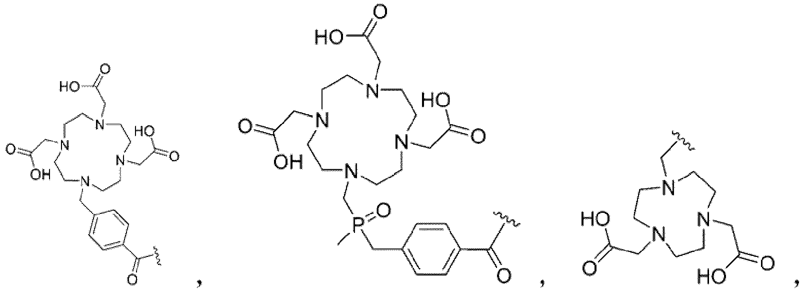

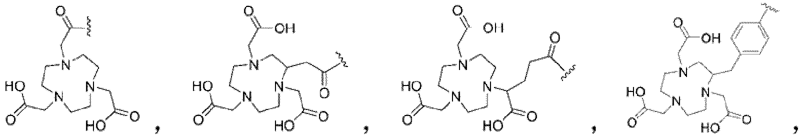

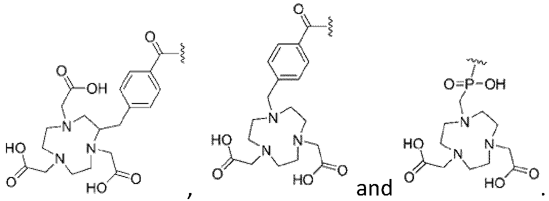

21. The trifunctional compound or the pharmaceutically acceptable salt, stereoisomer or solvate thereof according to claim 19 or 20, wherein the trifunctional compound has a structure of general formula (II):
wherein:
C and P are as defined in claim 20,
c and d are both 1,
L₄ and L₅ are independently selected from:
a single bond, 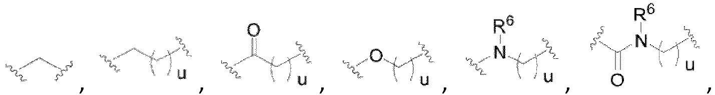 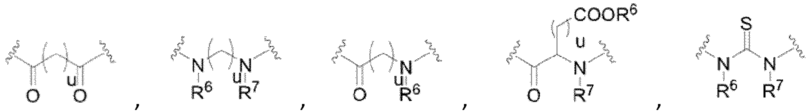 and 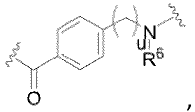
wherein u and o are independently 1, 2, 3, 4, or 5,
R⁶ and R⁷ are independently of each other selected from hydrogen and an optionally substituted C₁-C₄,
provided that in the linkers formed by L₁, L₂, and L₃, heteroatoms are not directly covalently bonded to each other, where the heteroatoms are selected from N, O, and S;
T has general formula (X), 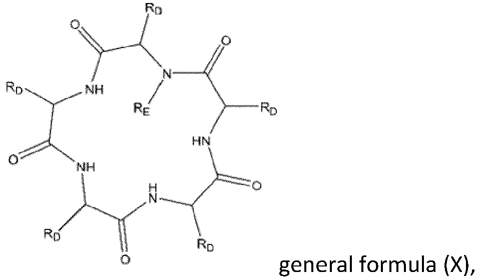
wherein each R_{D} is independently selected from H, -CH₂R_{D'}, -CH₂COOH, -CH₂CH₂CH₂NH₂, - CH₂CH₂CH₂CH₂NH₂, and 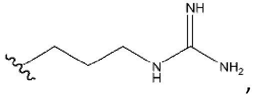 with R_{D'} being an aryl, preferably an optionally substituted phenyl or naphthyl, wherein the cyclic peptide is connected to the rest of the trifunctional compound via R_{D'} and/or the terminal -NH₂ of R_{D}, or the -CH₂R_{D'} or R_{D'} may form a part of the covalent warhead C;
R_{E} is H or methyl;
T preferably has the following formula 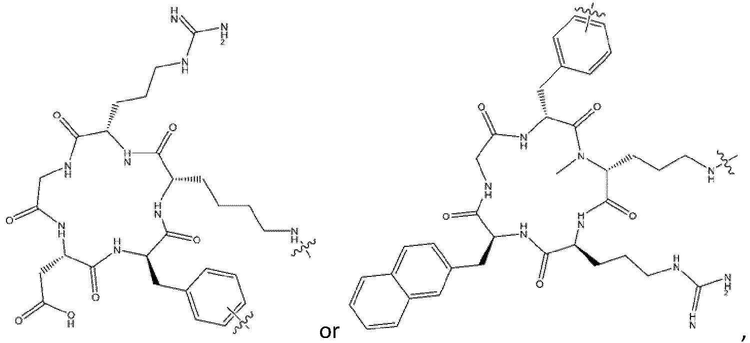
wherein the two wavy lines in each formula represent optional linkage sites.

22. The trifunctional compound or the pharmaceutically acceptable salt, stereoisomer or solvate thereof according to claim 1, wherein the trifunctional compound has a structure of general formula (II): 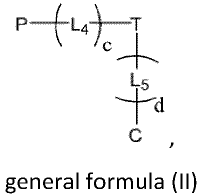 wherein:
L₄ and L₅ as shown are each independently a divalent linker unit;
c and d are independently an integer of 0 to 6;
C is a covalent warhead selected from the group consisting of: 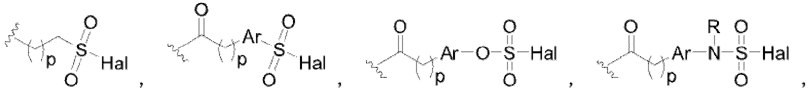 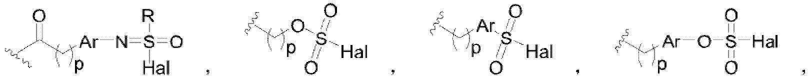 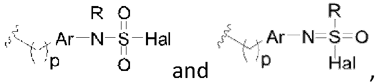 wherein:
Ar is an optionally substituted C₆-C₁₀ aryl, preferably an optionally substituted phenyl, or an optionally substituted C₅-C₁₂ heteroaryl;
Hal is F or Cl;
p is an integer between 0 and 6; and
R is selected from the group consisting of hydrogen, an optionally substituted alkyl, an optionally substituted cycloalkyl, an optionally substituted heterocycloalkyl, an optionally substituted aryl, and an optionally substituted heteroaryl;
T is a targeting group targeting somatostatin receptor (SSTR) such as SSTR2; and
P is a chelating group capable of chelating a radionuclide.

23. The trifunctional compound or the pharmaceutically acceptable salt, stereoisomer or solvate thereof according to claim 22, wherein the trifunctional compound has a structure of general formula (II):
wherein:
L₄ and L₅ are each independently selected from:
a single bond, 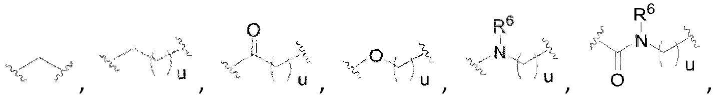 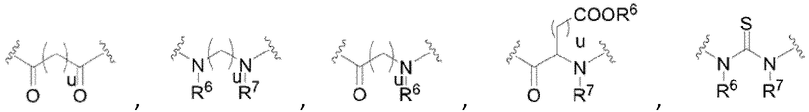 and 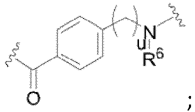
c and d are independently 0, 1, 2, or 3,
u is independently 1, 2, 3, 4, or 5,
R⁶ and R⁷ are independently of each other selected from the group consisting of hydrogen, an optionally substituted alkyl, an optionally substituted cycloalkyl, an optionally substituted heterocycloalkyl, an optionally substituted aryl, and an optionally substituted heteroaryl, and
provided that in the linkers formed by L₄ and L₅, heteroatoms are not directly covalently bonded to each other, where the heteroatoms are selected from N, O, and S;
C is selected from the group consisting of: 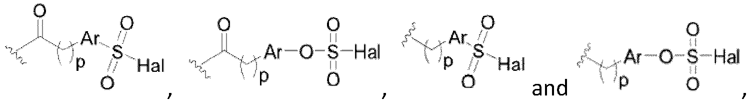 wherein:
Ar is phenyl;
Hal is F;
p is an integer between 0 and 3;
T is a targeting group targeting somatostatin receptor (SSTR) such as SSTR2; and
P is a chelating group capable of chelating a radionuclide and is selected from:

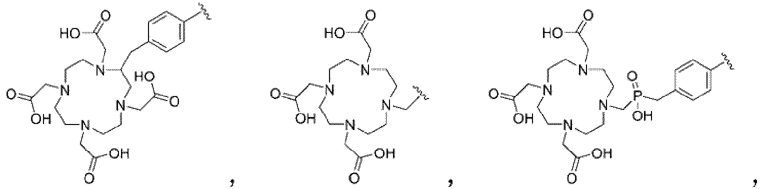

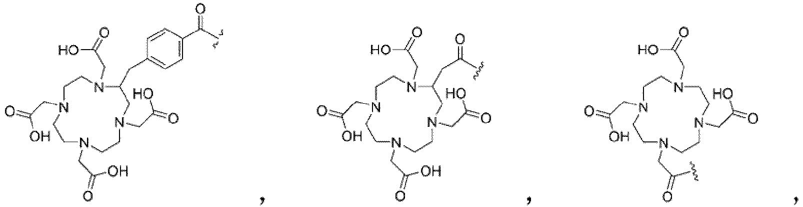

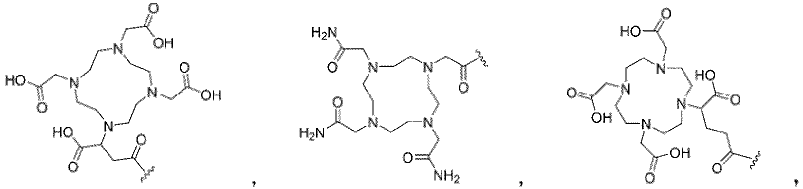

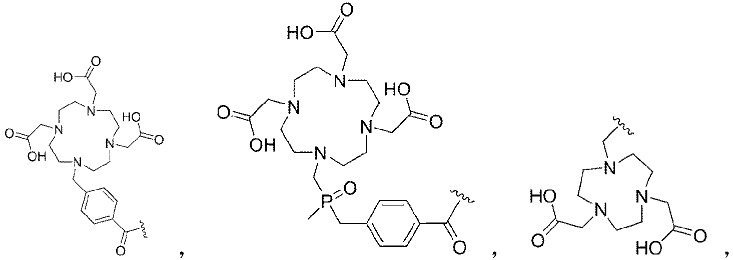

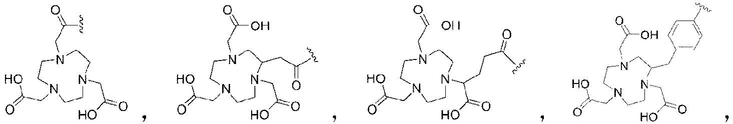

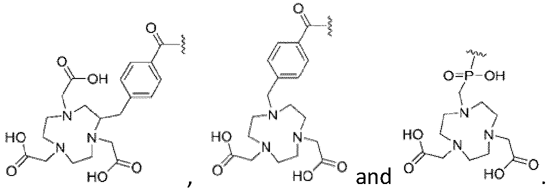

24. The trifunctional compound or the pharmaceutically acceptable salt, stereoisomer or solvate thereof according to claim 22 or 23, wherein the trifunctional compound has a structure of general formula (II):
wherein:
C and P are as defined in claim 23,
c and d are both 1,
L₄ and L₅ are independently selected from:
a single bond, 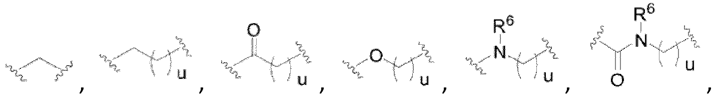 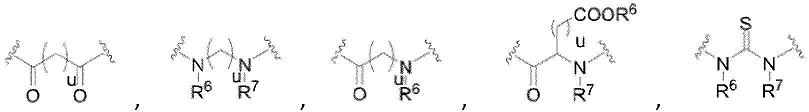 and 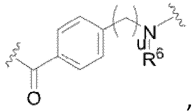
wherein u and o are independently 1, 2, 3, 4, or 5,
R⁶ and R⁷ are independently of each other selected from hydrogen and an optionally substituted C₁-C₄,
provided that in the linkers formed by L₁, L₂, and L₃, heteroatoms are not directly covalently bonded to each other, where the heteroatoms are selected from O, N, and S;
T has general formula (XII), 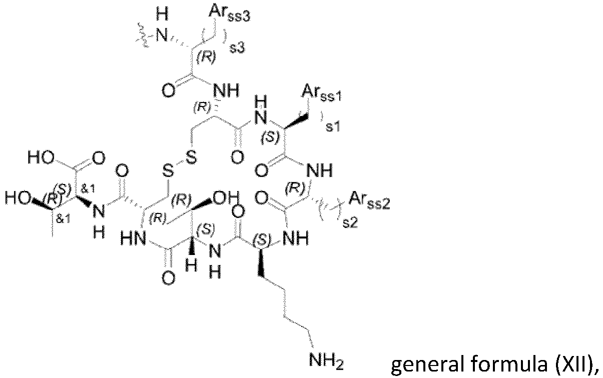
wherein Arₛₛ₁, Arₛₛ₂, and Arₛₛ₃ are each independently selected from an optionally substituted aryl or heteroaryl, preferably an optionally substituted C₆-C₁₀ aryl or C₅-C₁₀ heteroaryl, preferably, Arₛₛ₁ and Arₛₛ₃ are a C₆-C₁₀ aryl optionally substituted with -OH, and
Arₛₛ₂ is a C₆-C₁₀ heteroaryl,
s1, s2, and s3 are each independently selected from 1, 2, 3, or 4,
wherein T is connected to the rest of the trifunctional compound via the site shown by the wavy line, or -(CH₂)ₛ₃Arₛₛ₃ or Arₛₛ₃ may form a part of the covalent warhead C,
T preferably has the following structure

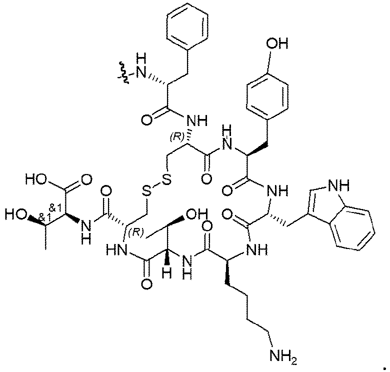

25. The trifunctional compound or the pharmaceutically acceptable salt, stereoisomer or solvate thereof according to any one of claims 1 to 24, which has one of the following structures:

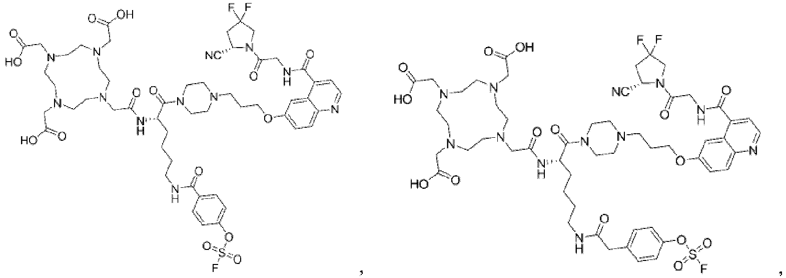

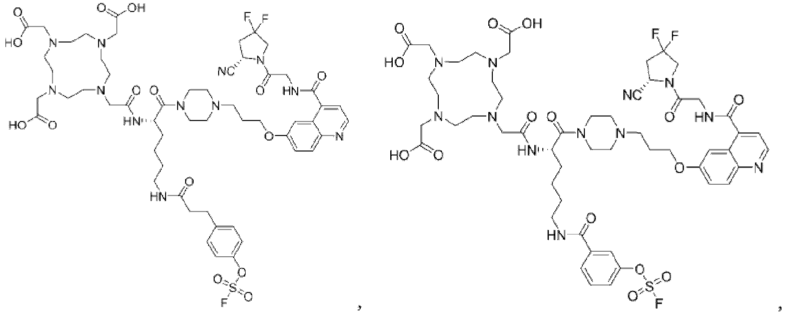

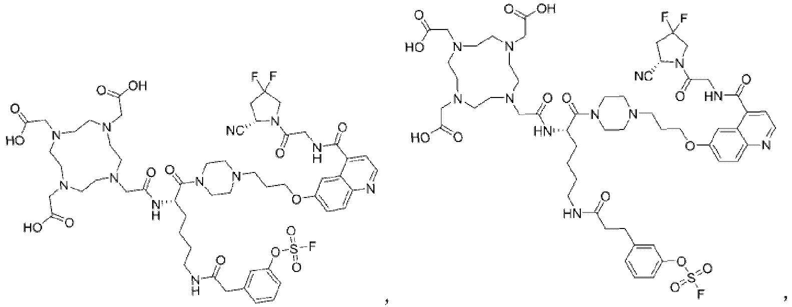

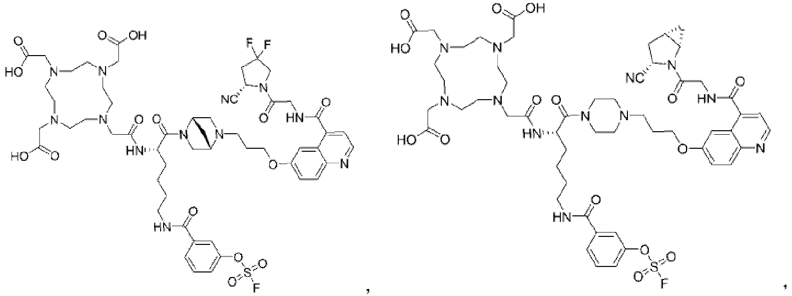

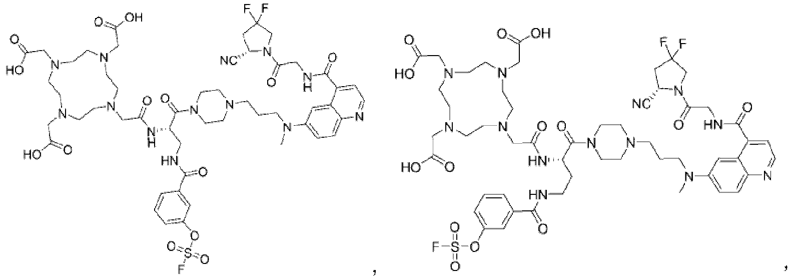

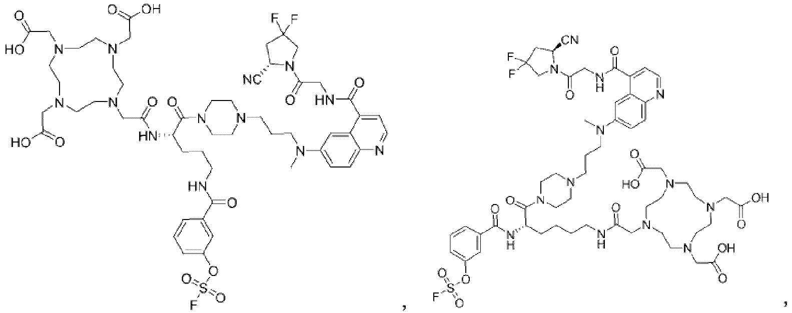

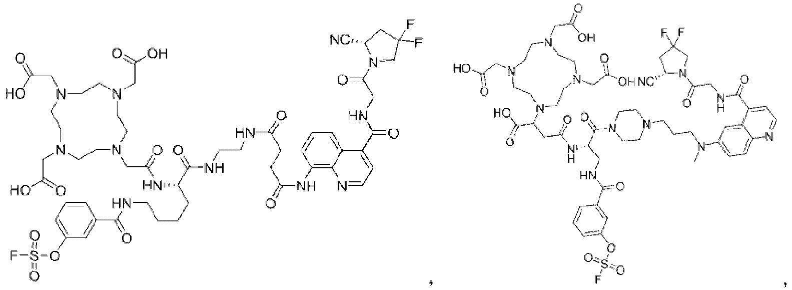

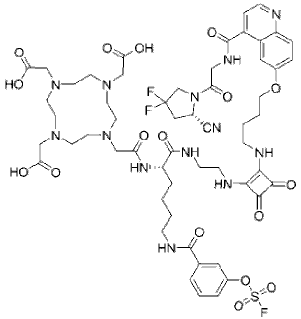

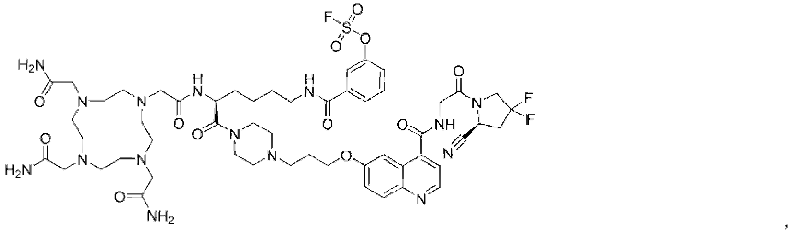

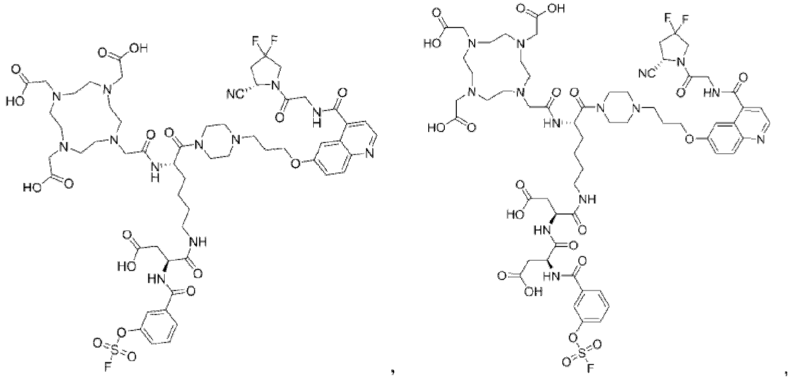

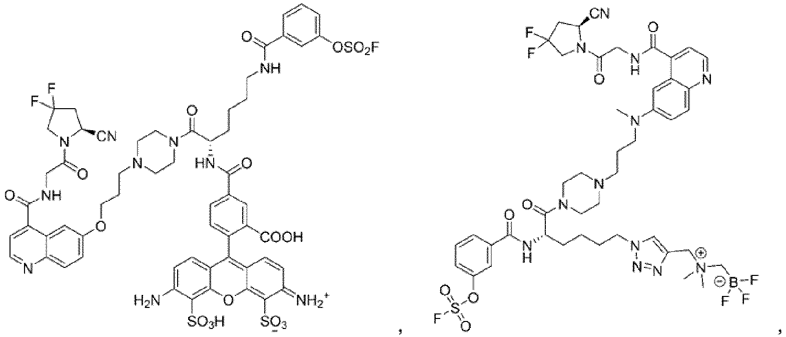

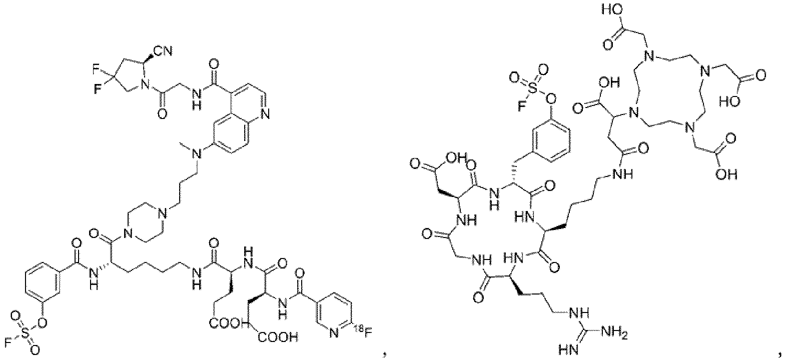

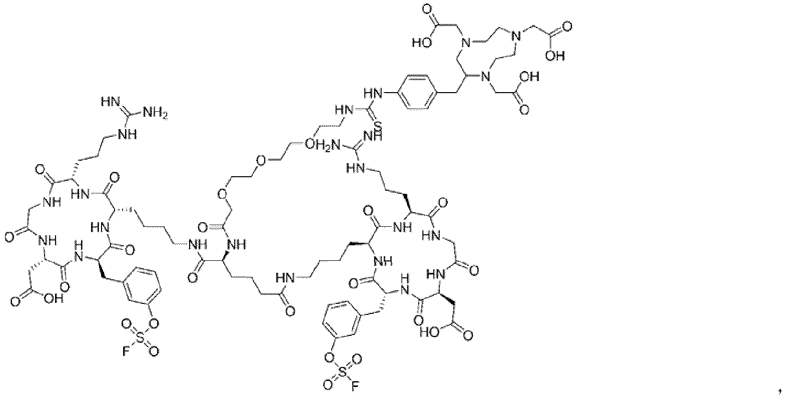

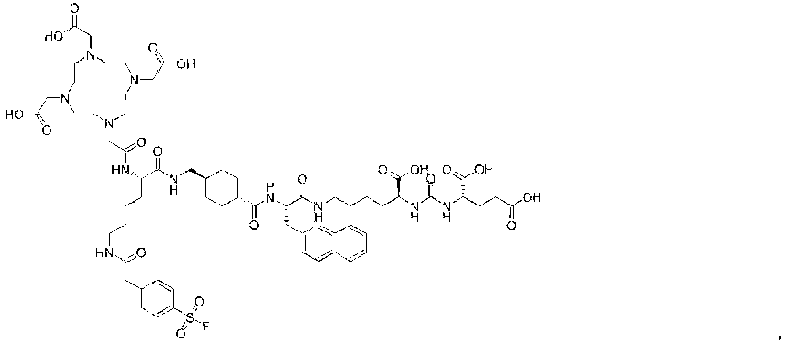

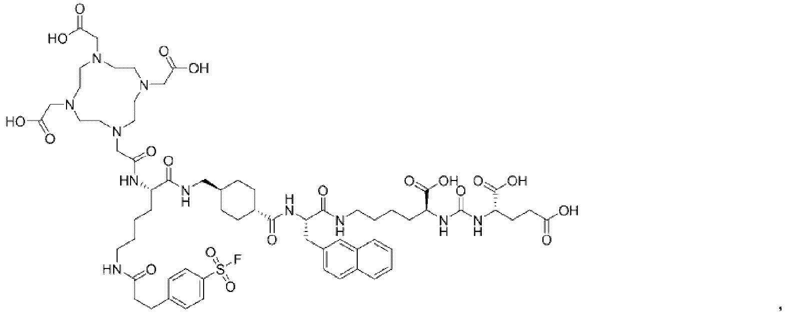

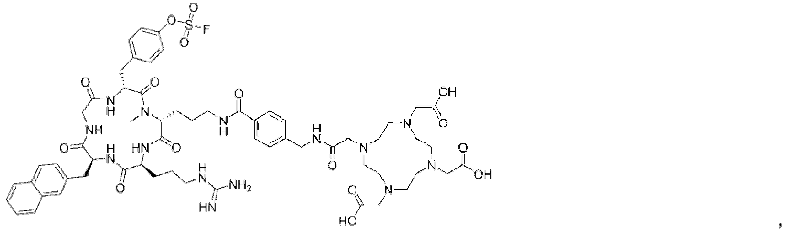

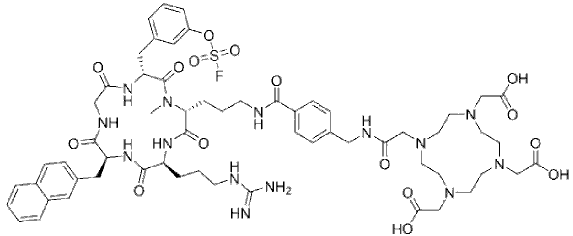

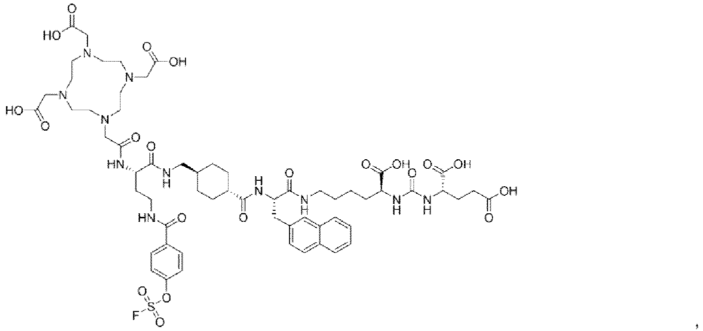

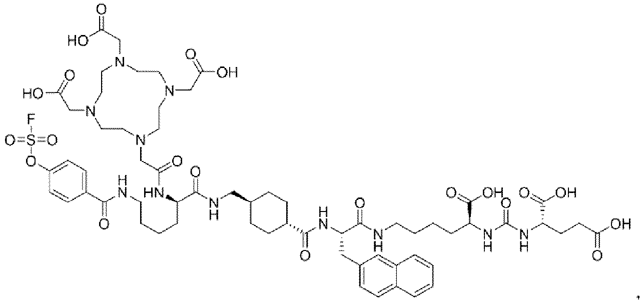

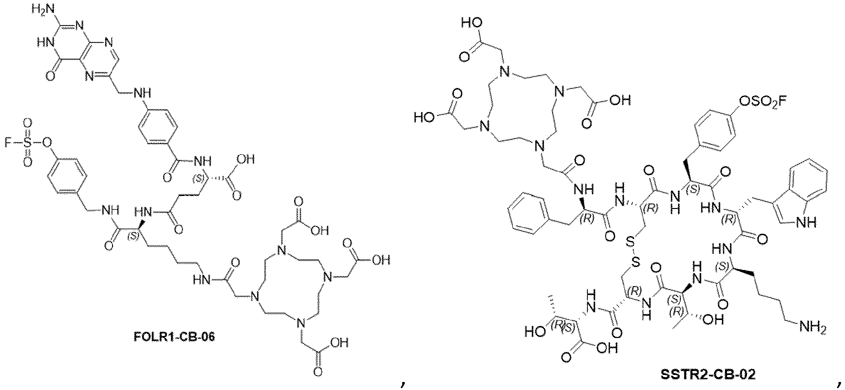

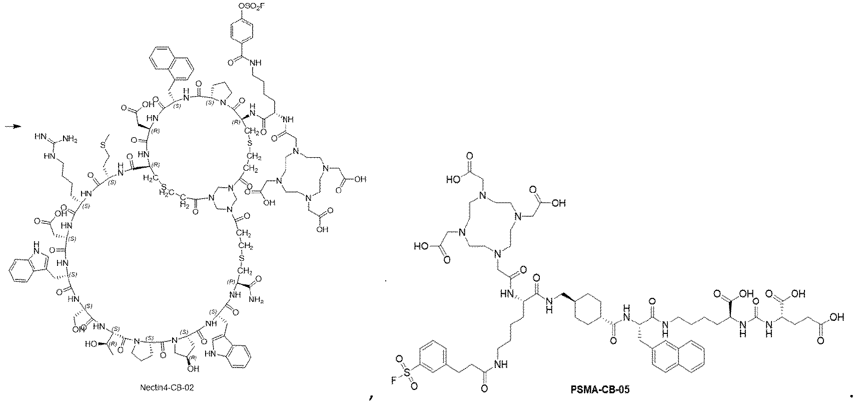

26. The trifunctional compound or the pharmaceutically acceptable salt, stereoisomer or solvate thereof according to any one of claims 7 to 25, wherein the payload is a chelating group capable of chelating a radionuclide and has a chelated radionuclide.

27. The trifunctional compound or the pharmaceutically acceptable salt, stereoisomer or solvate thereof according to any one of claims 7 to 26, wherein the radionuclide carried or chelated by the trifunctional compound is a positron nuclide, a β emitter, an α emitter, an isotope that emits Auger electrons, an isotope that emits X-rays, an isotope that emits fluorescence, or a stable metal/nonmetal element coordinated with a radionuclide, preferably ¹¹C, ¹³N, ¹⁵O, ¹⁸F and coordination metals thereof, ⁴⁷Sc, ⁵¹Cr, ⁶⁷Ga, ⁶⁸Ga, ⁸⁶Y, ⁹⁰Y, ⁶⁴Cu, ⁶⁷Cu, ⁷²As, ⁷²Se, ⁸⁹Zr, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁴²Pr, ¹⁵¹Eu, ¹⁵³Eu, ¹⁶⁹Eu, ¹⁵⁹Gd, ¹⁶¹Tb, ¹⁷⁷Lu, ¹⁹⁸Au, ¹⁹⁹Ag, ²⁰¹Tl, ²¹¹At, ²⁰³Pb, ²¹²Pb, ²¹²Bi, ²¹³Bi, ²²⁵Ac, ^{99m}Tc, ¹¹¹In, ¹⁴⁹Pm, ¹⁵³Sm, ¹⁶⁵Dy, ¹⁶⁹Er, ¹⁸⁶Re, ¹⁸⁸Re, ¹⁹⁷Hg, ²²⁷Th, ⁶⁷Ga, ⁶⁸Ga, ⁸⁶Y, ⁹⁰Y, ⁵⁵Co, ¹³⁹La, ¹⁴⁰La, ¹⁴⁹Tb, ¹⁵²Tb, ¹⁵⁵Tb, ¹⁶⁶Ho, ¹⁷⁵Yb, ²²⁶Th, ²²³Ra, and ²³⁰U.

28. A pharmaceutical composition comprising the trifunctional compound or the pharmaceutically acceptable salt, stereoisomer or solvate thereof according to any one of claims 1 to 27, and a pharmaceutically acceptable carrier.

29. A kit comprising or consisting of the trifunctional compound or the pharmaceutically acceptable salt, stereoisomer or solvate thereof according to any one of claims 1 to 27 or the pharmaceutical composition according to claim 28, and instructions for diagnosing a disease.

30. A method for diagnosing or treating a disease, the method comprising administering to a subject a therapeutically effective amount of the trifunctional compound or the pharmaceutically acceptable salt, stereoisomer or solvate thereof according to any one of claims 1 to 27, wherein the disease is preferably a central nervous system disease, a metabolic disease, preferably a cardiovascular metabolic disease, or cancer.

31. The method according to claim 30, wherein the cancer is selected from prostate cancer, breast cancer, pancreatic cancer, liver cancer, lung cancer, gastric cancer, renal cancer, ovarian cancer, bladder cancer, esophageal cancer, head and neck cancer, thymic cancer, cervical cancer, endometrial cancer, neuroendocrine tumor, thyroid cancer, intestinal cancer, glioma, and bone metastasis cancer.
